# EUROPÄISCHE PATENTSCHRIFT

(11) **EP 3 016 959 B1**
(45) Veröffentlichungstag und Bekanntmachung des Hinweises auf die Patenterteilung: **27.12.2017**
(21) Anmeldenummer: 14729862.4
(22) Anmeldetag: 06.06.2014
(51) Int. Cl.: C07F 15/00, C09K 11/00, H01L 51/00, H05B 33/10, C07D 221/22, C07D 401/04, C07D 413/04, C07D 471/04, C07D 491/18, C07D 495/04

(54) **POLYCYCLISCHE VERBINDUNGEN**
POLYCYCLIC COMPOUNDS
COMPOSÉS POLYCYCLIQUES

(30) Priorität: 02.07.2013 WO PCT/EP2013/001926; 18.07.2013 EP 13003625
(43) Veröffentlichungstag der Anmeldung: 11.05.2016
(73) Patentinhaber: Merck Patent GmbH, 64293 Darmstadt (DE)
(72) Erfinder: STOESSEL, Philipp, 60487 Frankfurt am Main (DE); JOOSTEN, Dominik, 60487 Frankfurt am Main (DE); KOENEN, Nils, 64285 Darmstadt (DE)
(86) Internationale Anmeldenummer: PCT/EP2014/001556
(87) Internationale Veröffentlichungsnummer: WO 2015/000546

(56) Entgegenhaltungen:
- WO-A1-2006/131192
- WO-A2-2014/023377
- EMILYE BOSCO ET AL: "Rational Design of Small Molecule Inhibitors Targeting the Rac GTPase-p67Signaling Axis in Inflammation", CHEMISTRY AND BIOLOGY, CURRENT BIOLOGY, LONDON, GB, Bd. 19, Nr. 2, 22. Dezember 2011 (2011-12-22), Seiten 228-242, XP028461901, ISSN: 1074-5521, DOI: 10.1016/J.CHEMBIOL.2011.12.017 [gefunden am 2012-01-12]
- CHRIS D. SMITH, JULIA I. GAVRILYUK, ALAN J. LOUGH, ROBERT A. BATEY: "Lewis acid catalyzed three-component hetero-Diels-Alder (Povarov) reaction of N-arylimines with strained norbornene-derived dienophiles", J. ORG. CHEM., Bd. 75, Nr. 3, 29. Dezember 2009 (2009-12-29), Seiten 702-715, XP002728071, & Chris D Smith ET AL: "S1 Lewis Acid Catalyzed Three-Component Hetero Diels-Alder (Povarov) Reaction of N-Arylimines with Norbornene-Derived Dienophiles, Supporting Information", J. ORG. CHEM. 2010, 75, 702-715, 29. Dezember 2009 (2009-12-29), XP055132245, Gefunden im Internet: URL:http://pubs.acs.org/doi/suppl/10.1021/ jo9021106/suppl_file/jo9021106_si_004.pdf [gefunden am 2014-07-29]

## Beschreibung

Die vorliegende Erfindung betrifft Verbindungen, welche sich für den Einsatz in elektronischen Vorrichtungen eignen. Weiterhin betrifft die vorliegende Erfindung Verfahren zu deren Herstellung und elektronische Vorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische Feldeffekt-Transistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:
(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

In E. Bosco et al, Chemistry and Biology, Current Biology, London, Bd. 19, Nr. 2, 2011, 228-242 wird eine NO₂-substituierte Verbindung mit der Bezeichnung Analog 17 offenbart, die in Zusammenhang mit der Entwicklung von Medikamenten untersucht wurde, die auf eine Inhibierung der ROS-Produktion von NOX Enzymen Einfluß nehmen sollten.

In C. Smith et al, J. Org. Chem. 2010, 75, 702-715 wird die Verbindung 5 beschrieben, die mit Br substituiert ist, und die als Nebenprodukt bei der Herstellung medizinisch- und biologisch-relevanter Verbindungen auftritt.

WO 2006/131192 A1 betrifft strukturell unterschiedliche polyzyklische aromatische überbrückte Verbindungen, die in organischen Elektrolumineszenzvorrichtungen verwendet werden können.

Zu diesen Eigenschaften gehört insbesondere die Lebensdauer der elektronischen Vorrichtungen. Ein weiteres Problem stellt insbesondere die Energieeffizienz dar, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein.

Aufgabe der vorliegenden Erfindung ist daher die Bereitstellung neuer Verbindungen, welche zu elektronischen Vorrichtungen mit verbesserten Eigenschaften führen. Insbesondere ist die Aufgabe Lochinjektionsmaterialien, Lochtransportmaterialien, Lochblockiermaterialien, Elektroneninjektionsmaterialien, Elektronenblockierrilaterialien und/oder Emittermaterialien bereitzustellen, welche verbesserte Eigenschaften in Bezug auf Effizienz, Betriebsspannung, Lebensdauer, Farbkoordinaten und/oder Farbreinheit, d. h. Breite der Emissionsbande, zeigen. Weiterhin sollten sich die Verbindungen möglichst einfach verarbeiten lassen, insbesondere eine gute Löslichkeit und Filmbildung zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verbindungen mit allen Merkmalen des Patentanspruchs 1 gelöst werden. Zweckmäßige Abwandlungen der erfindungsgemäßen Verbindungen werden in den auf Anspruch 1 rückbezogenen abhängigen Ansprüchen unter Schutz gestellt.

Gegenstand der Erfindung ist somit eine Verbindung, umfassend mindestens eine Struktur der Formeln (I) und/oder (II) 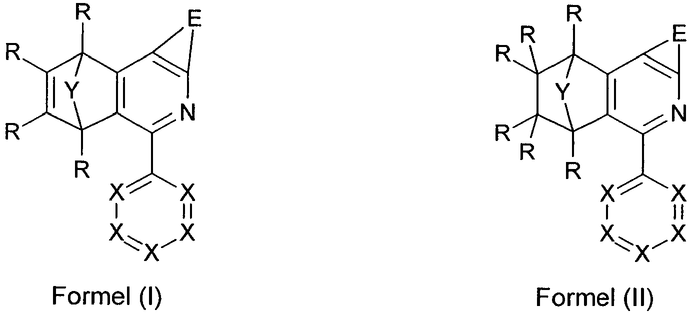 wobei für die verwendeten Symbole gilt:
- X: ist bei jedem Auftreten gleich oder verschieden CR oder N;
- E: ist eine bivalente Brücke, wobei die Gruppe E zusammen mit den hiermit verbundenen C-Atomen einen fünf- oder sechsgliedrigen Ring bildet;
- Y: ist eine bivalente Brücke, ausgewählt aus O, S, C(R)₂, C(R)=C(R), N(R), B(R), Si(R)₂, C=O, C=NR, C=C(R)₂, S=O, SO₂, C(R)₂-C(R)₂, P(R) und P(=O)R;
- R: ist bei jedem Auftreten gleich oder verschieden H, D, F, Cl, I, N(R¹)₂, CN, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyl- oder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
- R¹: ist bei jedem Auftreten gleich oder verschieden H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere benachbarte Reste R¹ miteinander oder R¹ mit R ein mono- oder polycyclisches, aliphatisches, aromatisches oder heteroaromatisches Ringsystem bilden; R² ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches Ringsystem bilden.

Dabei bedeutet "benachbarte Kohlenstoffatome" bzw. benachbarte "CH₂-Gruppen", dass die Kohlenstoffatome direkt aneinander gebunden sind.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

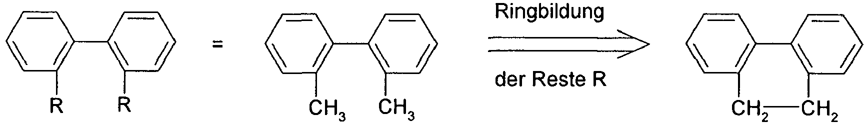

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

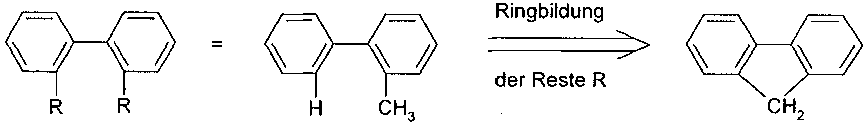

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5-60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind Verbindungen umfassend Strukturen der Formeln (I) und/oder (II), dadurch gekennzeichnet, dass E ausgewählt ist aus Gruppen der Formel X=X-X=X, X-W-X, W-X=X, X=X-W, worin W ausgewählt ist aus O, S, C(R)₂, N(R), B(R), Si(R)₂, C=O, S=O, SO₂, P(R) und P(=O)R, wobei X und R bei jedem Auftreten gleich oder verschieden sind und die zuvor genannte Bedeutung haben. Vorzugsweise ist W ausgewählt aus O, S und N(R). Besonders bevorzugt bildet E zusammen mit den hiermit verbundenen C-Atomen einen aromatischen oder heteroaromatischen Ring mit fünf- oder sechs Gliedern.

Bevorzugt sind weiterhin Verbindungen, umfassend mindestens eine Struktur der Formeln (la) und/oder (IIa) 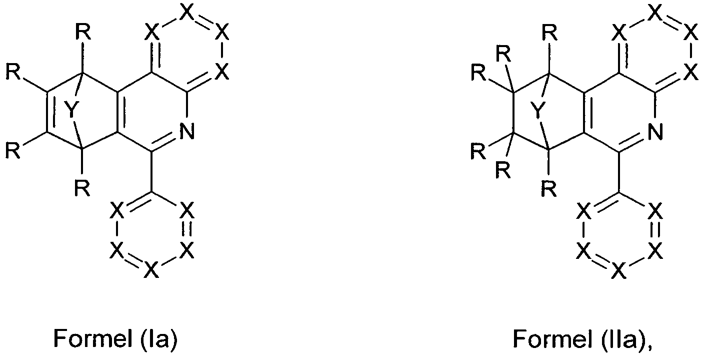 wobei die verwendeten Symbole die zuvor genannten Bedeutungen haben.

Besonders bevorzugt sind Verbindungen, umfassend mindestens eine Struktur der Formeln (Ia1), (Ia2), (IIa1) und/oder (IIa2) 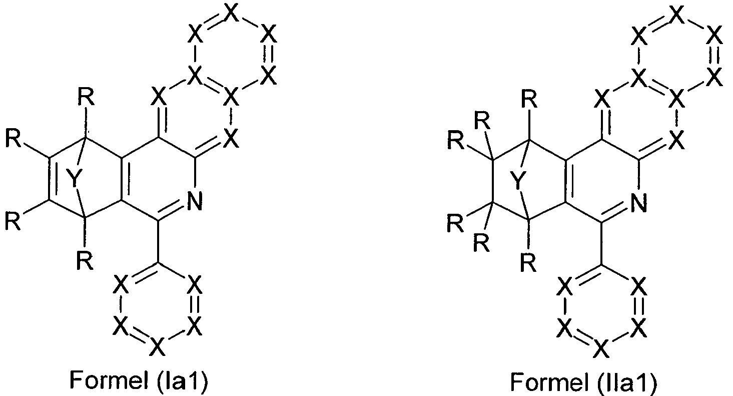 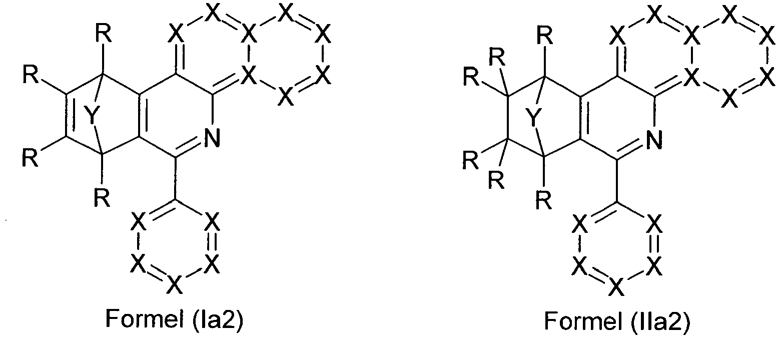 wobei die verwendeten Symbole die zuvor genannten Bedeutungen haben.

Ferner sind Verbindung mit mindestens einer Struktur der Formeln (Ia3) und/oder (Ia4) 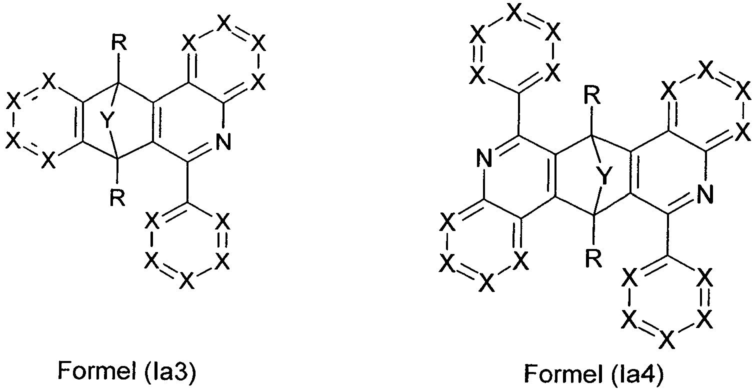 bevorzugt, wobei die verwendeten Symbole die zuvor genannten Bedeutungen haben.

Gemäß einer weiteren Ausgestaltung der vorliegenden Erfindung sind Verbindungen, umfassend mindestens eine Struktur der Formeln (Ib) und/oder (IIb) 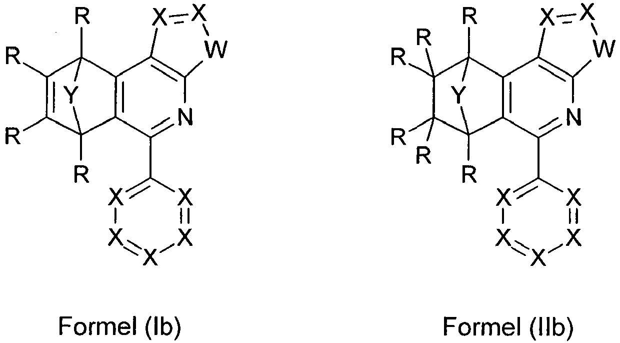 bevorzugt, wobei die verwendeten Symbole die zuvor genannten Bedeutungen haben.

Gemäß noch einer weiteren Ausgestaltung der vorliegenden Erfindung sind Verbindungen, umfassend mindestens eine Struktur der Formeln (Ib-1) und/oder IIb-1 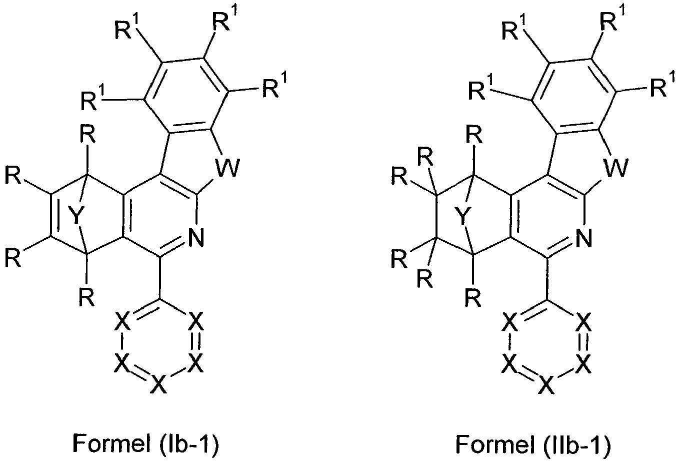 ganz bevorzugt, wobei die verwendeten Symbole die zuvor genannten Bedeutungen haben.

Ferner sind Verbindungen, umfassend Strukturen der Formel (Ic) 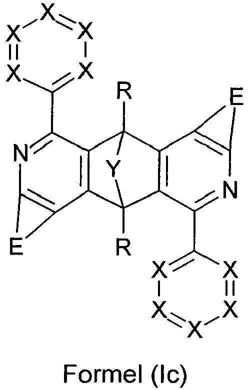 bevorzugt, wobei die verwendeten Symbole die die zuvor genannten Bedeutungen haben und E bei jedem Auftreten gleich oder verschieden sein kann.

Ferner kann vorgesehen sein, dass die Verbindungen mindestens zwei bicyclische Gruppen aufweisen, die durch ein Strukturelement umfassend eine Gruppe Y gebildet werden, wobei Y bei jedem Auftreten gleich oder verschieden sein kann und die zuvor genannte Bedeutung aufweist.

Vorzugsweise kann die Verbindung Strukturen der Formel CyE-(CyF)ₙ aufweisen, wobei für die Symbole und Indizes gilt:
n ist 2 oder 3
CyE ist ein Strukturelement ausgewählt aus den Formeln

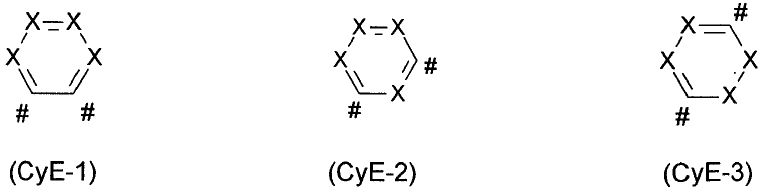

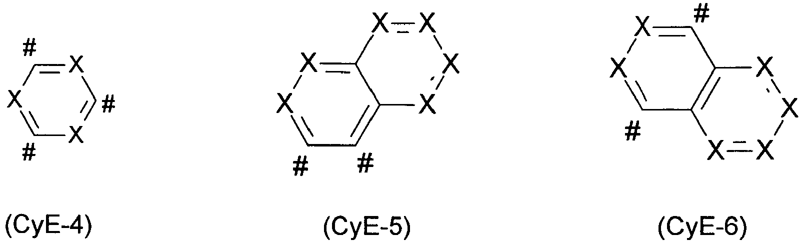

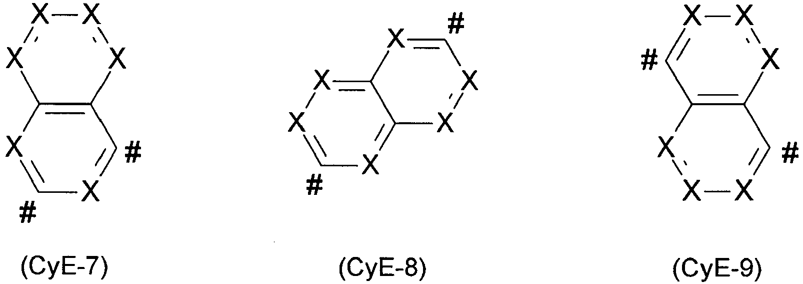

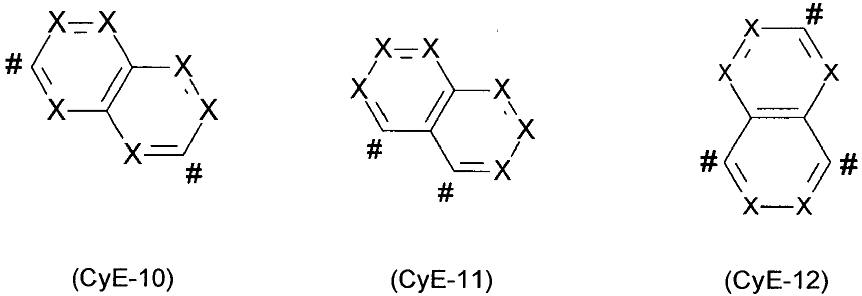

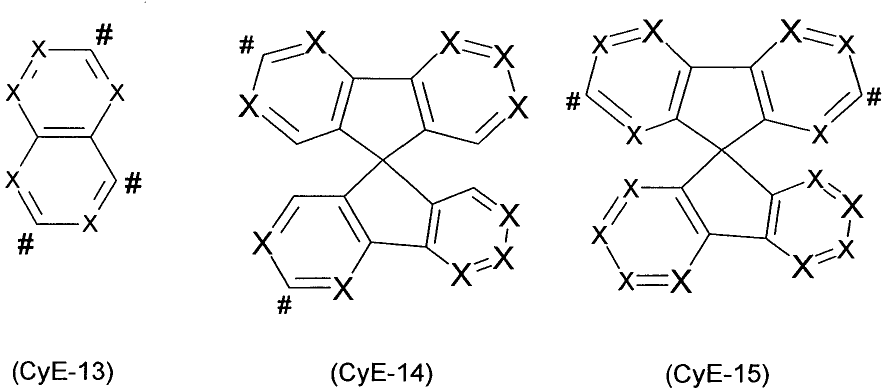

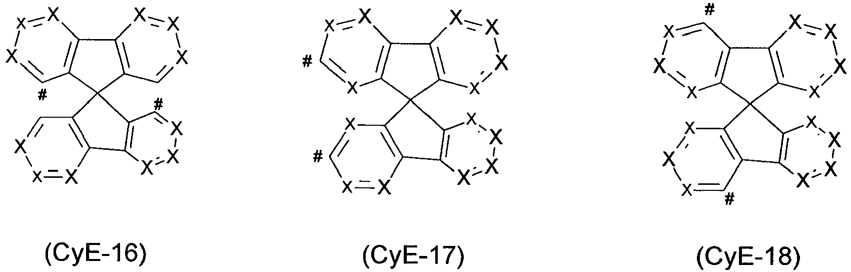

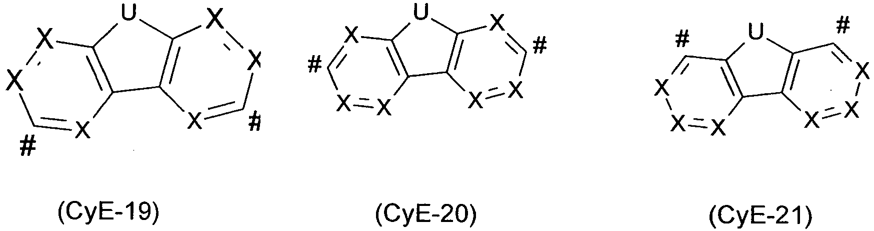

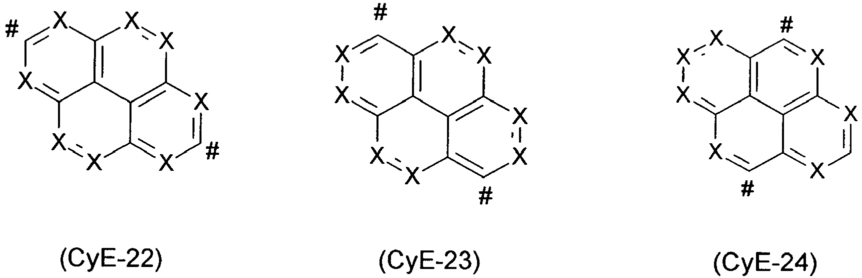

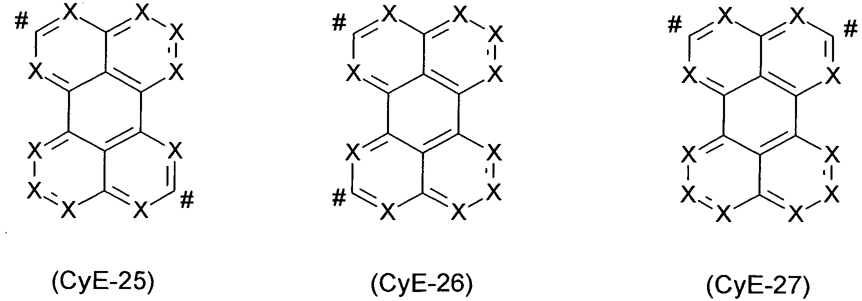

und CyF ist mindestens ein Strukturelement ausgewählt aus den Formeln 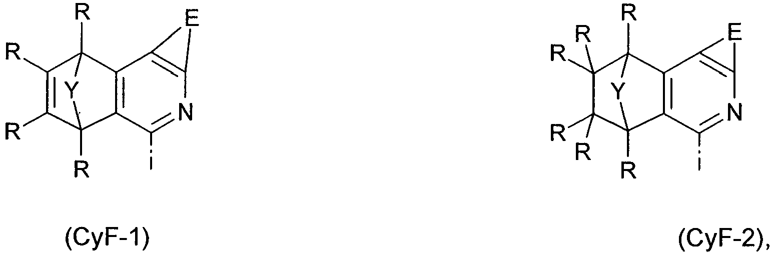 wobei die verwendeten Symbole E, R, Y und X die zuvor genannten Bedeutungen haben, U ausgewählt ist aus O, S, C(R)₂, N(R), B(R), Si(R)₂, C=O, S=O, SO₂, P(R) und P(=O)R, wobei U bevorzugt O, N(R) und C(R)₂, ganz bevorzugt O und C(R)₂, und besonders bevorzugt C(R)₂ bedeutet, die gestrichelte Linie in den Formeln CyF-1 und CyF-2 die Bindung an die Gruppe CyE kennzeichnen und Gruppe CyF jeweils an der durch # gekennzeichneten Position an CyE bindet.

In einer weiteren bevorzugten Ausführungsform der vorliegenden Erfindung ist U gleich O.

Besonders bevorzugt kann vorgesehen sein, dass CyF mindestens ein Strukturelement ausgewählt aus den Formeln ist 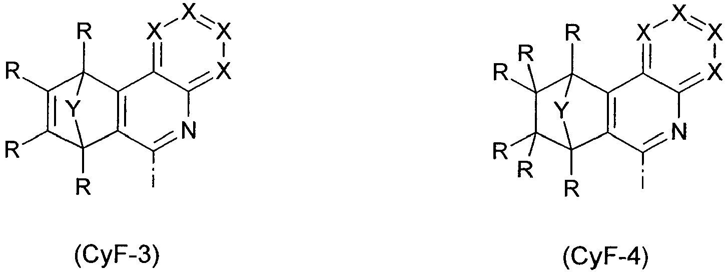 wobei die verwendeten Symbole R, Y und X die in Anspruch 1 genannten Bedeutungen haben, die gestrichelte Linie in den Formeln CyF-3 und CyF-4 die Bindung an CyE kennzeichnen und CyF jeweils an der durch # gekennzeichneten Position an CyE gebunden ist.

Bevorzugt stehen maximal drei Symbole X in CyE und/oder CyF für N, besonders bevorzugt stehen maximal zwei Symbole X in CyC für N, ganz besonders bevorzugt steht maximal ein Symbol X in CyC für N. Insbesondere bevorzugt stehen alle Symbole X für CR.

Bevorzugte Ausführungsformen der Gruppe CyE sind die Strukturen der folgenden Formeln (CyE-28) bis (CyE-52), wobei U ausgewählt ist aus O, S, C(R)₂, N(R), B(R), Si(R)₂, C=O, S=O, SO₂, P(R) und P(=O)R, wobei U vorzugsweise C(R)₂ bedeutet, und die Gruppe CyE jeweils an der durch # gekennzeichneten Position an CyF bindet,

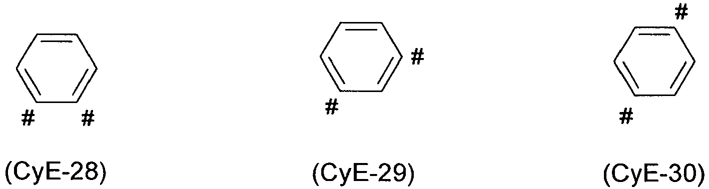

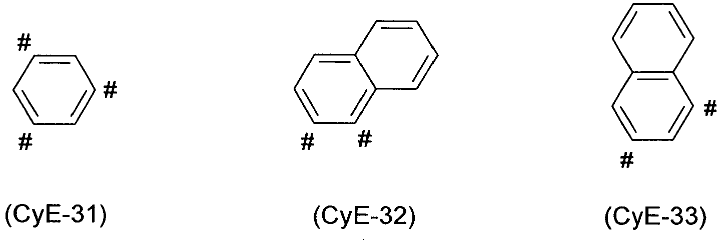

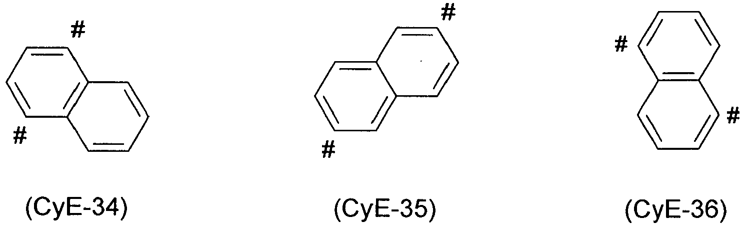

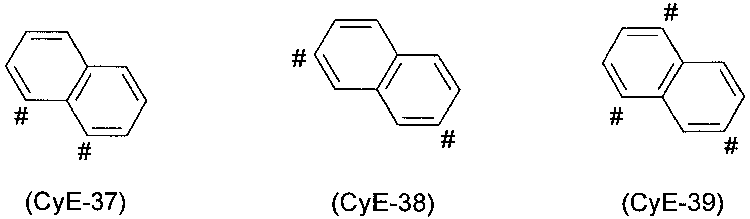

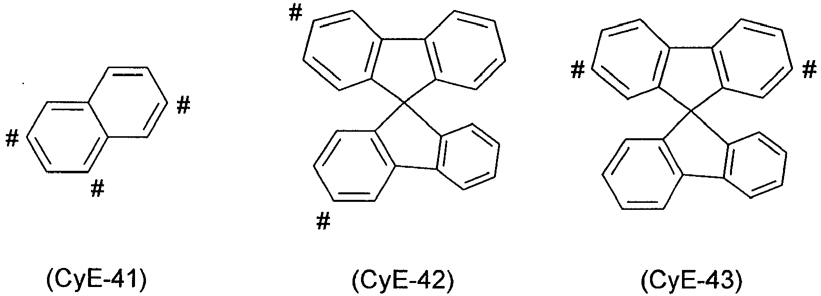

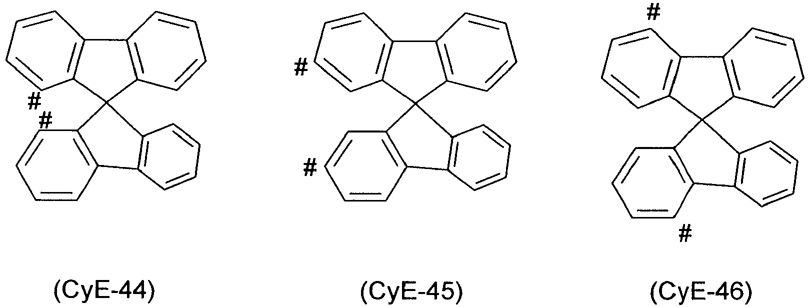

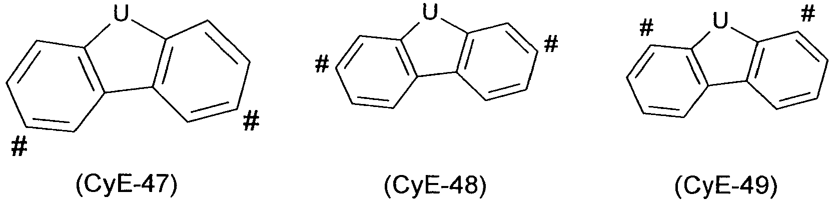

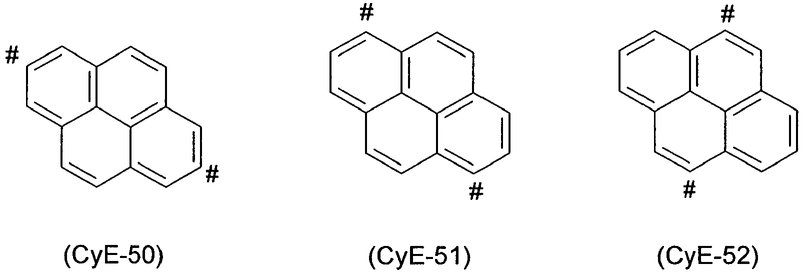

Ferner kann vorgesehen sein, dass die Verbindungen Strukturen der Formel CyG(CyH)ₙ aufweisen, wobei CyG und CyH zusammen jeweils einen Ring aufspannen und für die Symbole und Indizes gilt: n ist 2 oder 3

CyG ist ein Strukturelement ausgewählt aus den Formeln 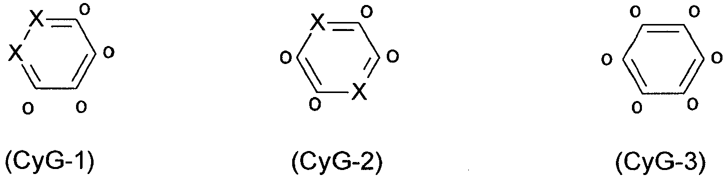 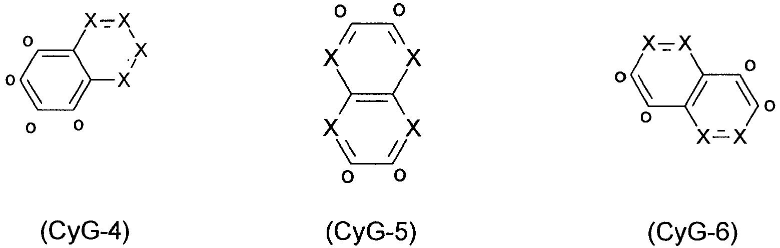 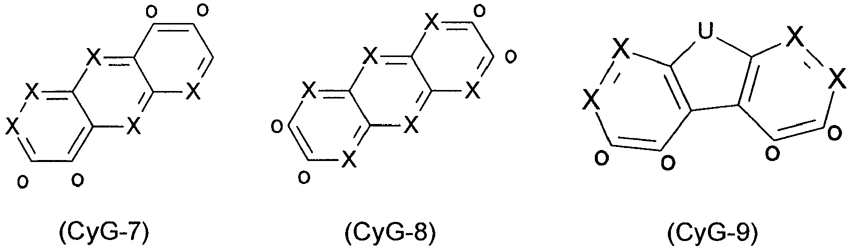 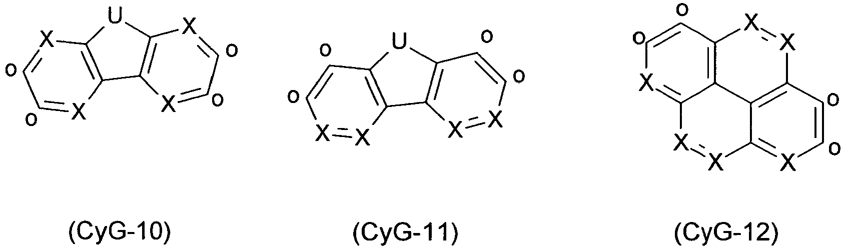 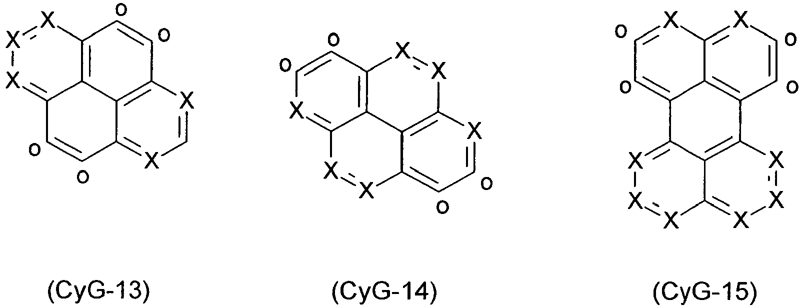 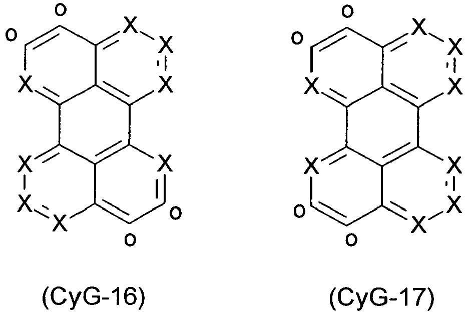 und CyH ist mindestens ein Strukturelement ausgewählt aus den folgenden Formeln 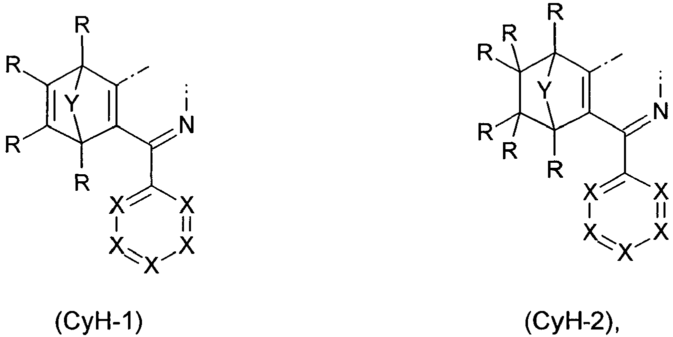 wobei die verwendeten Symbole R, Y und X die zuvor genannten Bedeutungen haben, U ausgewählt ist aus O, S, C(R)₂, N(R), B(R), Si(R)₂, C=O, S=O, SO₂, P(R) und P(=O)R, wobei U vorzugsweise C(R)₂ bedeutet, die gestrichelte Linie in den Formeln CyH-1 und CyH-2 die Bindung an CyG kennzeichnen und CyH jeweils an den durch o gekennzeichneten Positionen an CyG unter Bildung eines Ringes binden.

Bevorzugt stehen maximal drei Symbole X in CyG und/oder CyH für N, besonders bevorzugt stehen maximal zwei Symbole X in CyC für N, ganz besonders bevorzugt steht maximal ein Symbol X in CyC für N. Insbesondere bevorzugt stehen alle Symbole X für CR.

Besonders bevorzugte Gruppen CyG sind die Gruppen der folgenden Formeln (CyG-16) bis (CyG-28), 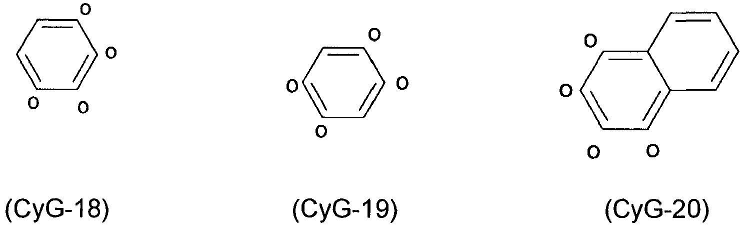 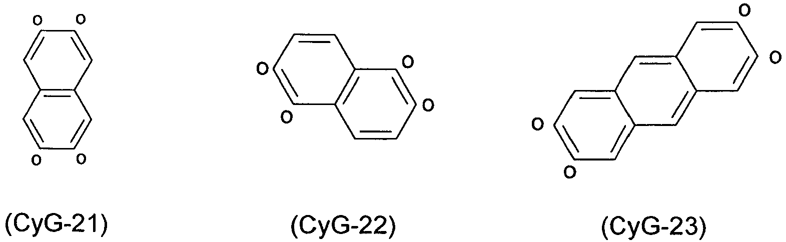 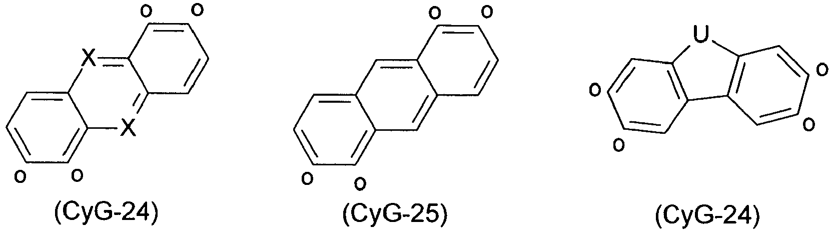 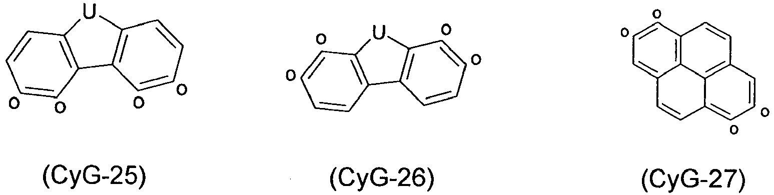 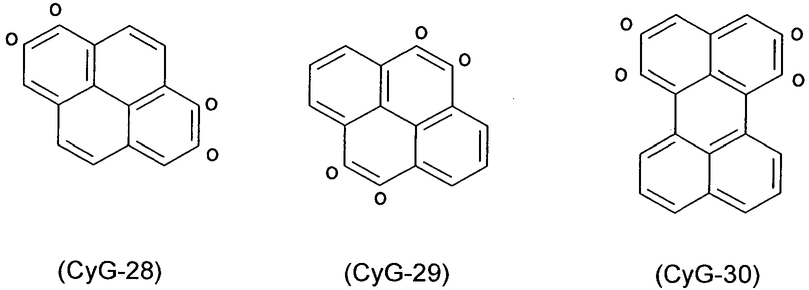 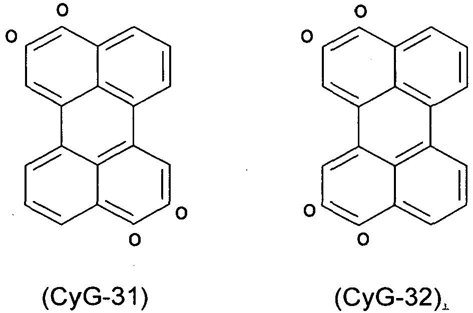 wobei das verwendete Symbol X die zuvor genannte Bedeutung hat, U ausgewählt ist aus O, S, C(R)₂, N(R), B(R), Si(R)₂, C=O, S=O, SO₂, P(R) und P(=O)R, wobei U vorzugsweise C(R)₂ bedeutet, und CyH jeweils an den durch o gekennzeichneten Positionen an CyG unter Bildung eines Ringes binden

Wenn in der Struktur der Formel (I) und/oder Formel (II) Reste R gebunden sind, so sind diese Reste R bei jedem Auftreten gleich oder verschieden bevorzugt ausgewählt aus der Gruppe bestehend aus H, D, F, I, N(R¹)₂, CN, Si(R¹)₃, B(OR¹)₂, C(=O)R¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, die jeweils mit einem oder mehreren Resten R¹ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste R¹ substituiert sein kann; dabei können zwei benachbarte Rest R oder R mit R¹ auch miteinander ein mono- oder polycyclisches, aliphatisches oder aromatisches Ringsystem bilden. Besonders bevorzugt sind diese Reste R bei jedem Auftreten gleich oder verschieden ausgewählt aus der Gruppe bestehend aus H, D, F, N(R¹)₂, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste R¹ substituiert sein kann; dabei können zwei benachbarte Reste R oder R mit R¹ auch miteinander ein mono- oder polycyclisches, aliphatisches oder aromatisches Ringsystem bilden.

Die erfindungsgemäßen Verbindungen, umfassend Strukturen der Formel (I) und/oder (II) können je nach Struktur auch chiral sein. Dies ist insbesondere dann der Fall, wenn sie Substituenten enthalten, beispielsweise Alkyl-, Alkoxy, Dialkylamino- oder Aralkylgruppen, welche ein oder mehrere Stereozentren aufweisen. Da es sich bei der Grundstruktur des Komplexes auch um eine chirale Struktur handeln kann, ist die Bildung von Diastereomeren und mehreren Enantiomerenpaaren möglich. Die erfindungsgemäßen Verbindungen umfassen dann sowohl die Mischungen der verschiedenen Diastereomere bzw. die entsprechenden Racemate wie auch die einzelnen isolierten Diastereomere bzw. Enantiomere.

Vorzugsweise kann die Verbindung in Form einer Enantiomerenmischung, besonders bevorzugt einer Diasteromerenmischung vorliegen. Hierdurch können die Eigenschaften von elektronischen Vorrichtungen, die unter Verwendung der erfindungsgemäßen Verbindungen erhältlich sind, unerwartet gesteigert werden. Zu diesen Eigenschaften gehören insbesondere die Lebensdauer der Vorrichtungen.

Weiterhin kann vorgesehen sein, dass dass in den Strukturen gemäß Formeln (I), (II), (la), (IIa), (Ia1), (IIa1), (Ia2), (IIa2), (Ia3), (Ia4), (Ib), (IIb) und (Ic) sowie den Strukturen gemäß Formel CyE-(CyF)ₙ und CyG(CyH)ₙ die Symbole X so gewählt werden, dass das Verhältnis von CR zu N vorgzusweise größer oder gleich 2, bevorzugt größer oder gleich 3 und besonders bevorzugt größer oder gleich 4 ist.

Besonders bevorzugte Verbindungen umfassen Strukuren gemäß den folgenden Formeln 1 bis 59:

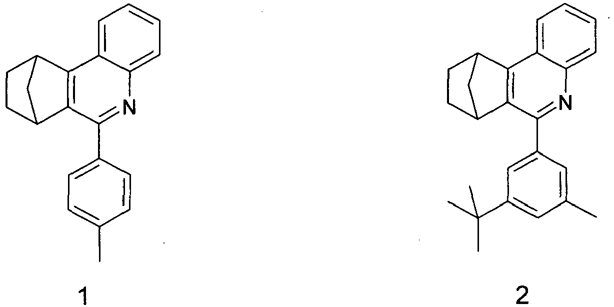

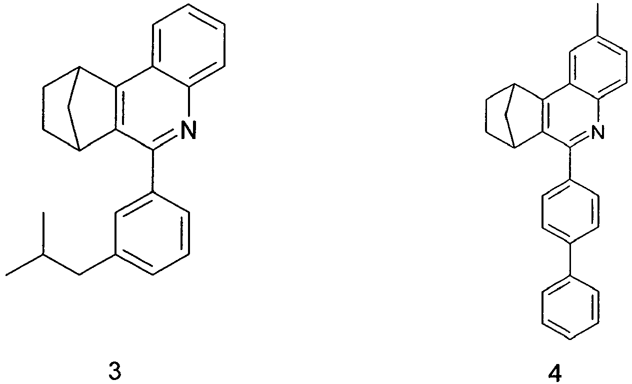

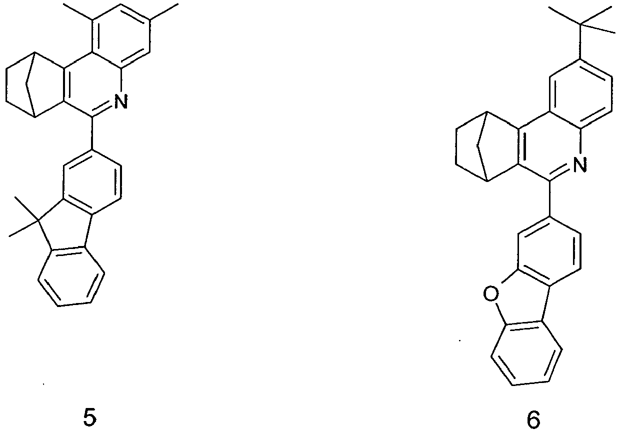

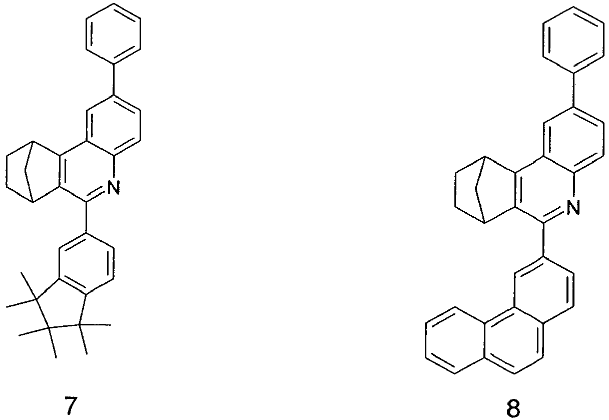

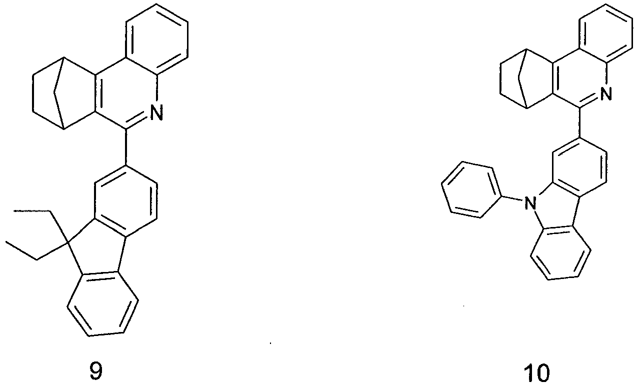

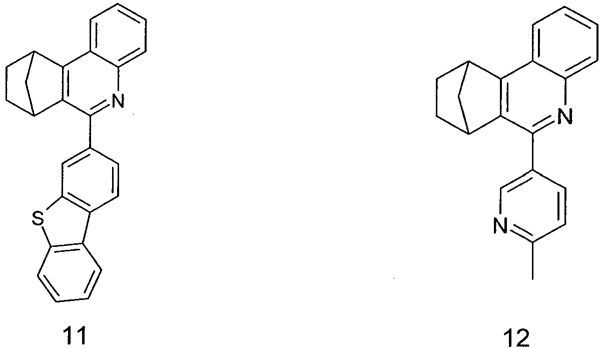

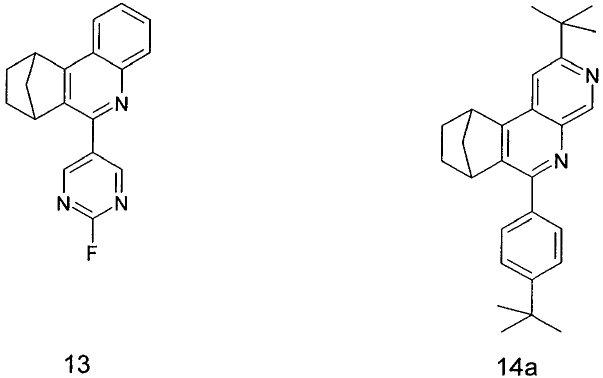

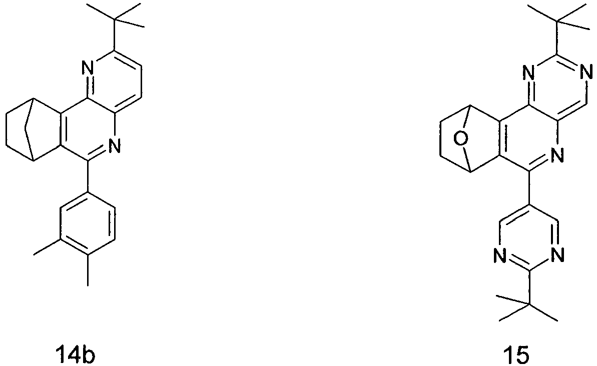

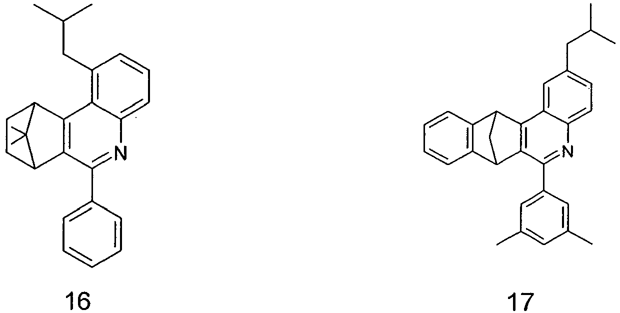

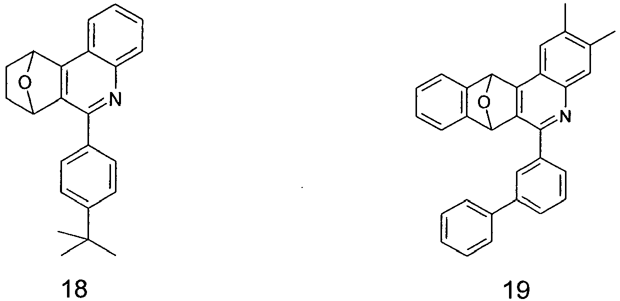

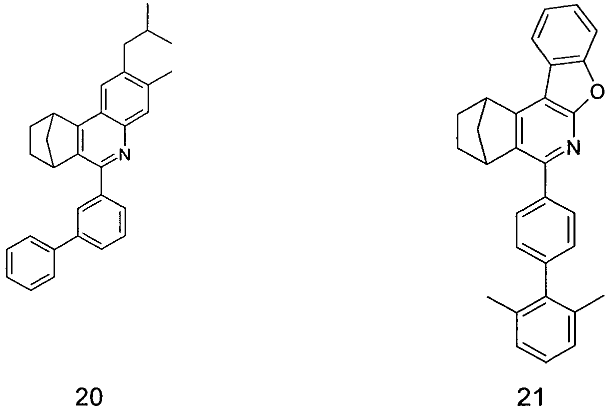

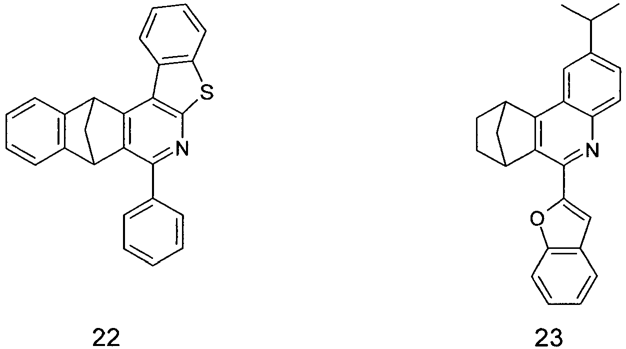

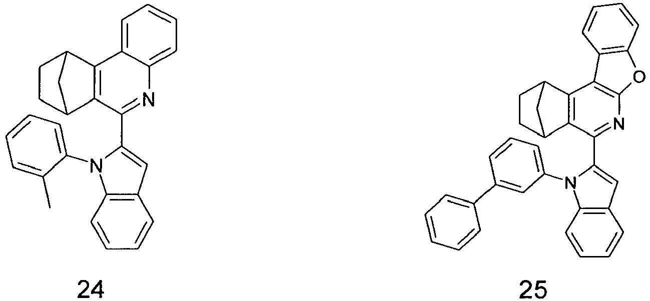

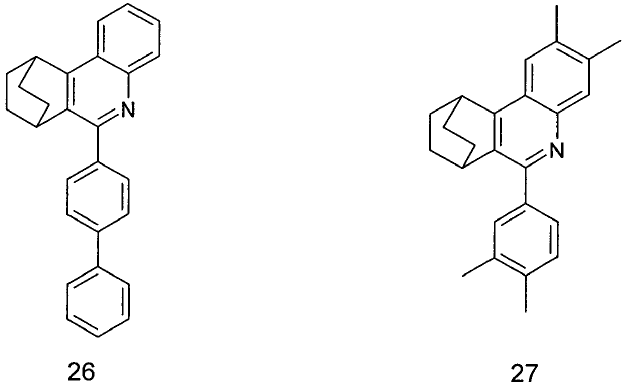

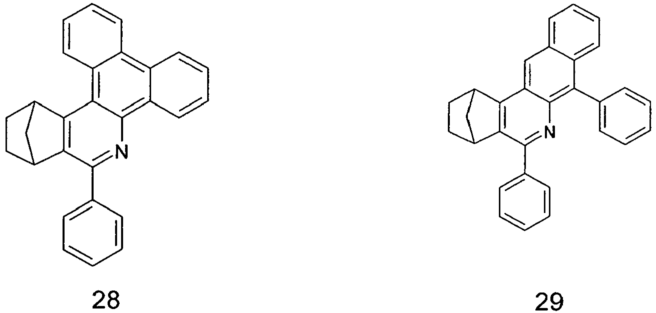

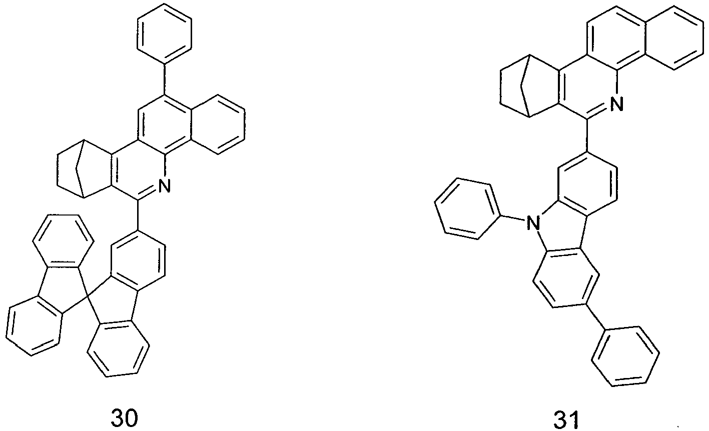

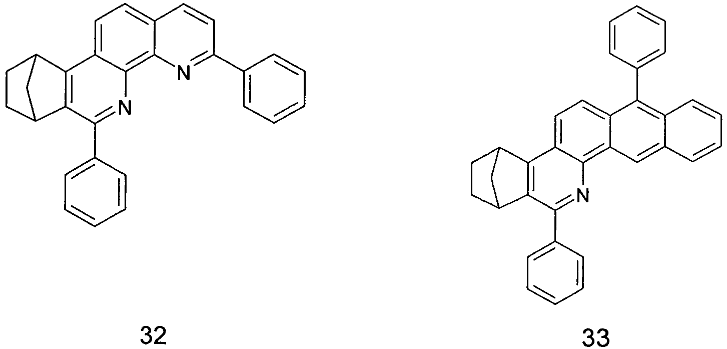

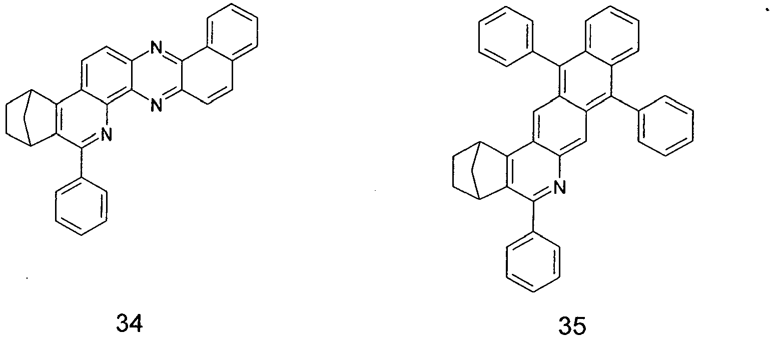

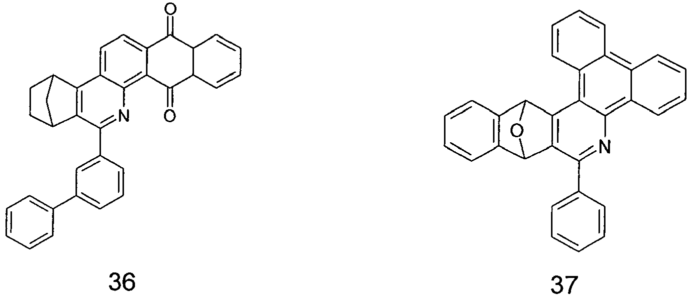

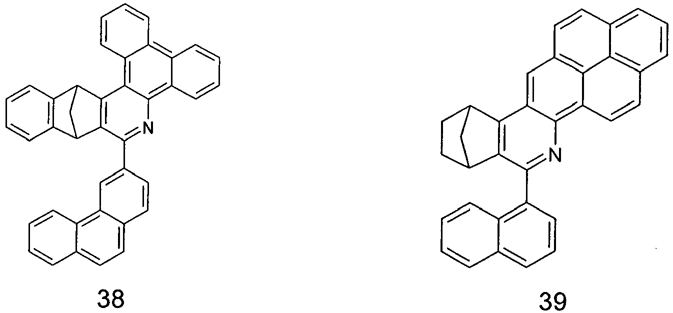

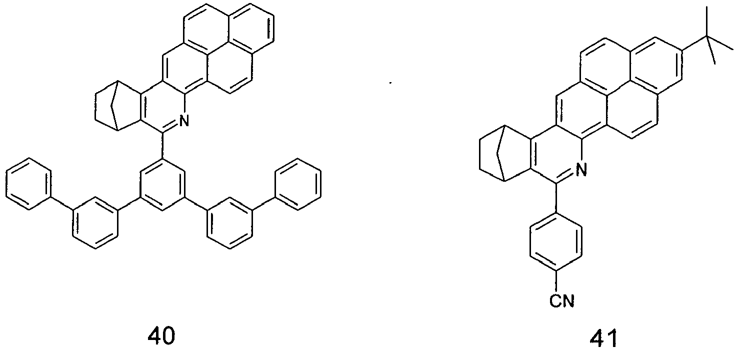

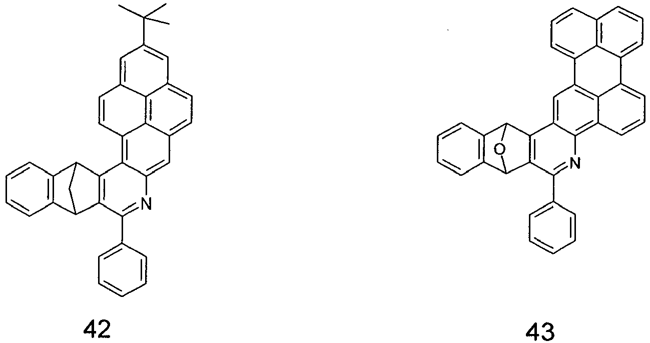

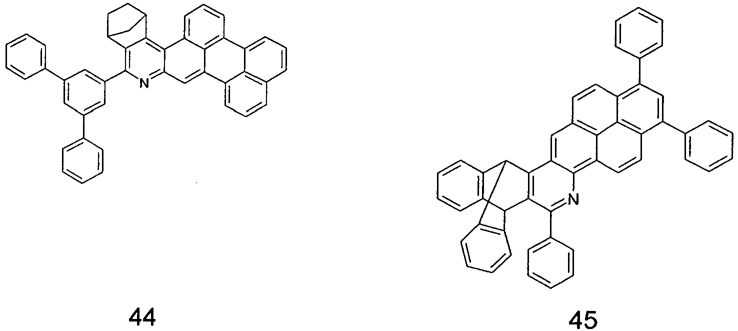

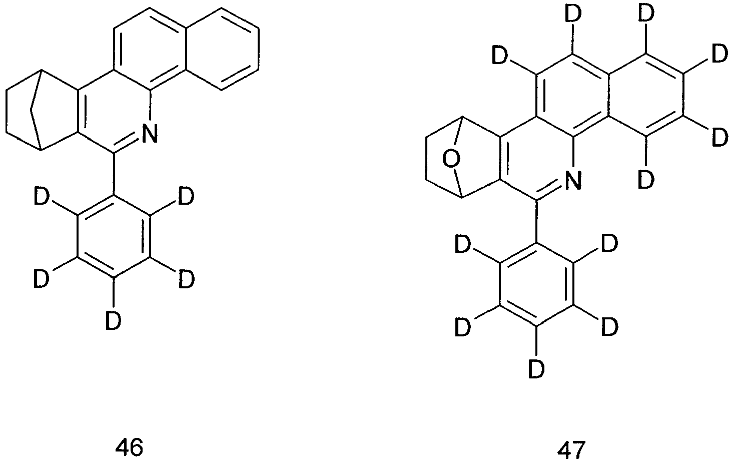

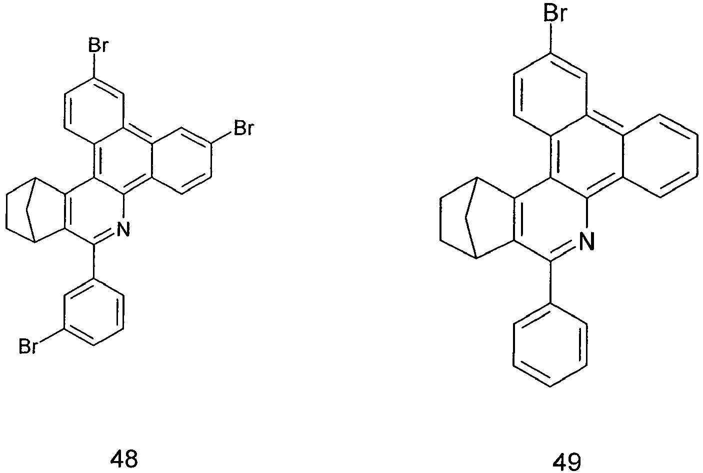

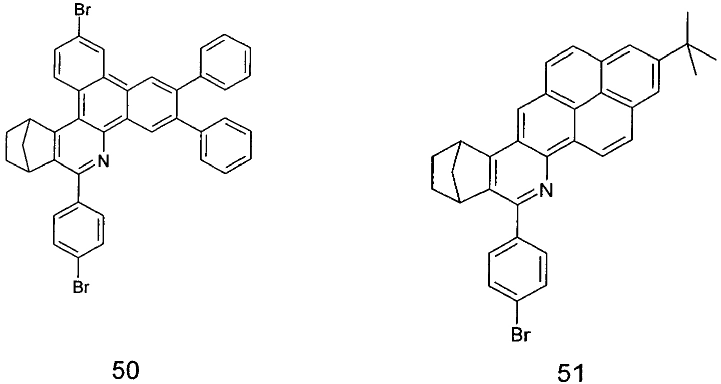

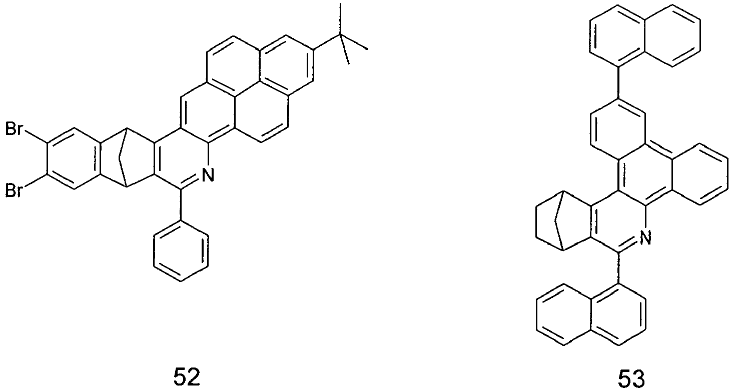

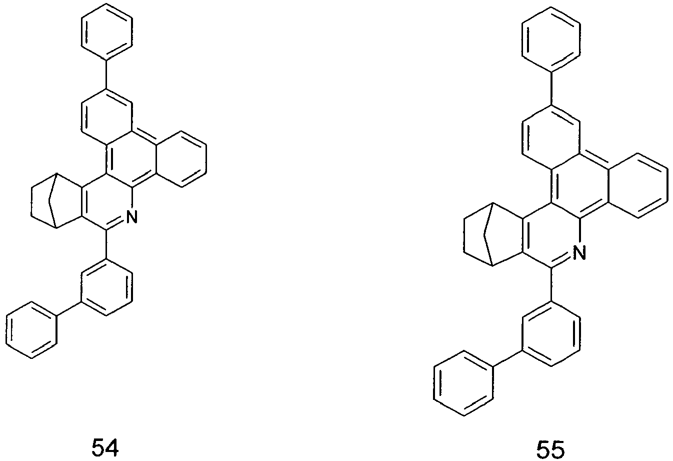

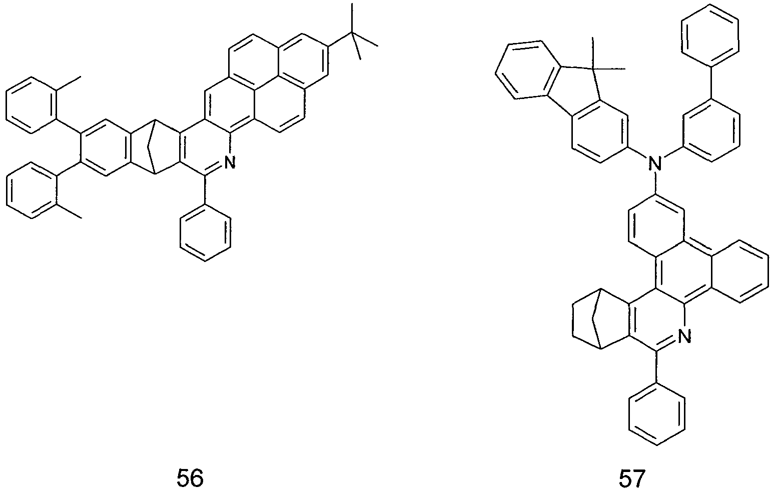

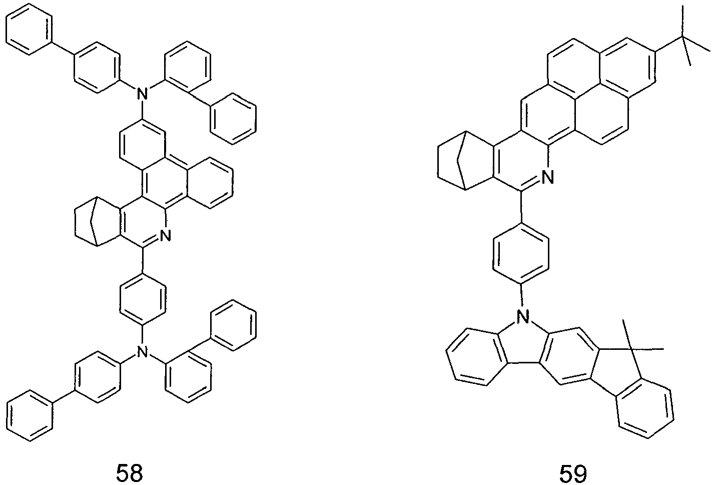

Weiterhin umfassen besonders bevorzugte Verbindungen Strukuren gemäß den folgenden Formeln 60 bis67:

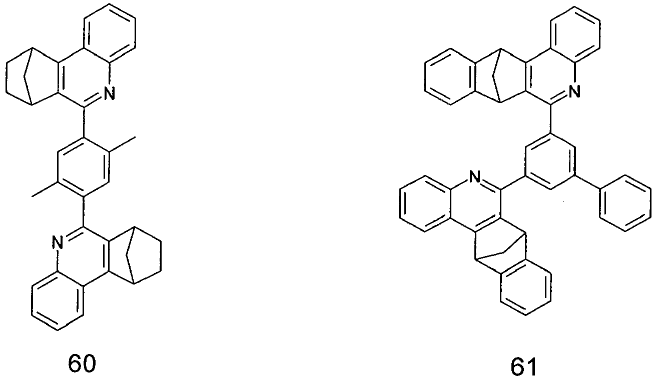

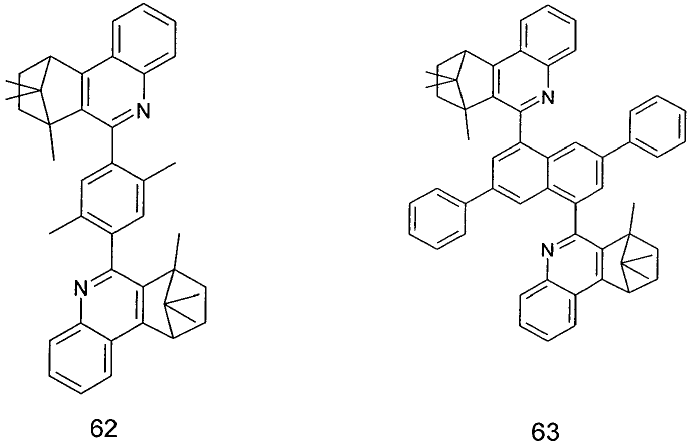

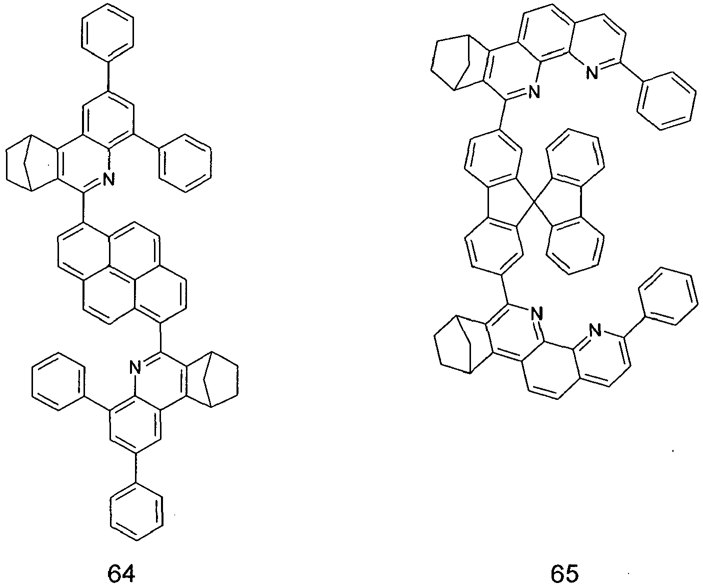

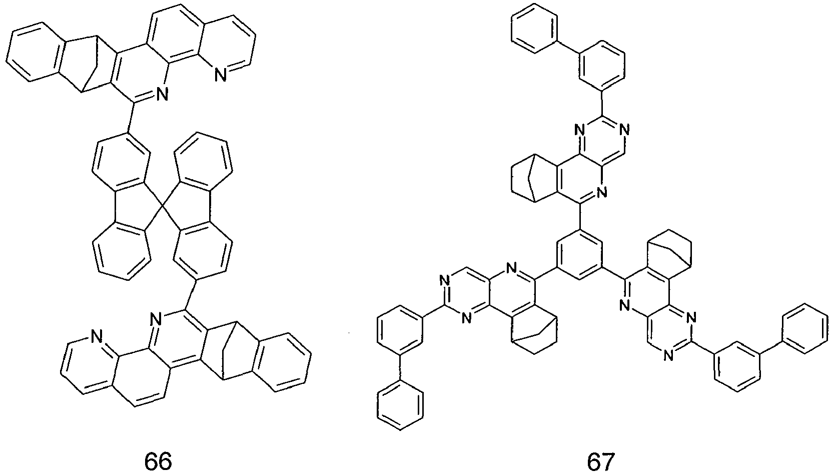

Die Verbindungen mit mindestens einer Struktur der Formeln 60, 61, 64 bis 67 können bevorzugt in Form eines Diastereomerengemisches vorliegen und/oder eingesetzt werden.

Ferner sind Verbindungen besonders bevorzugt, die Strukturen gemäß den folgenden Formeln 68 bis 77 umfassen:

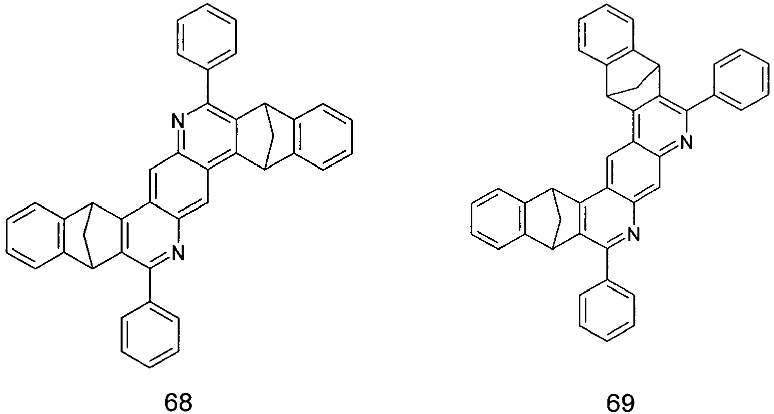

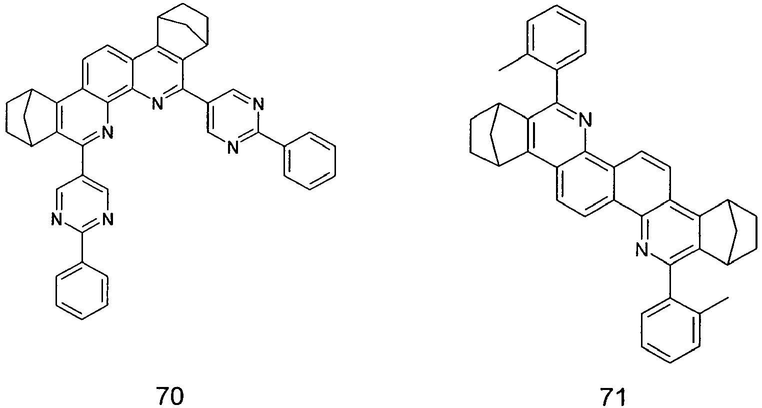

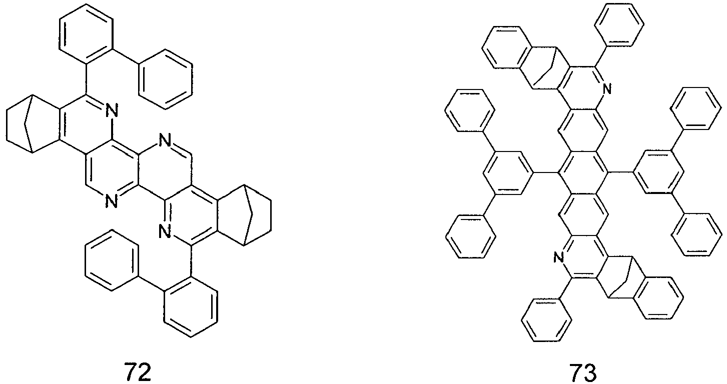

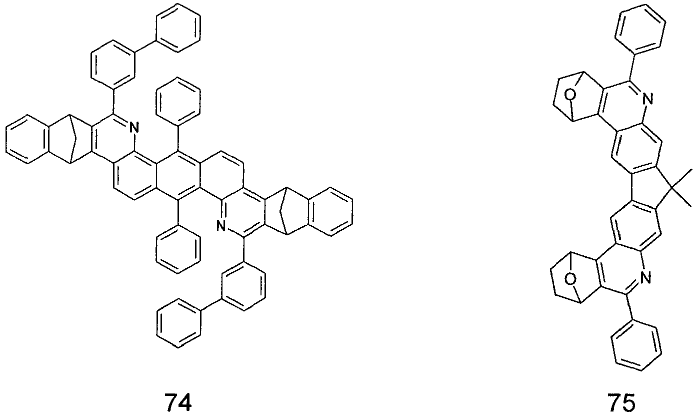

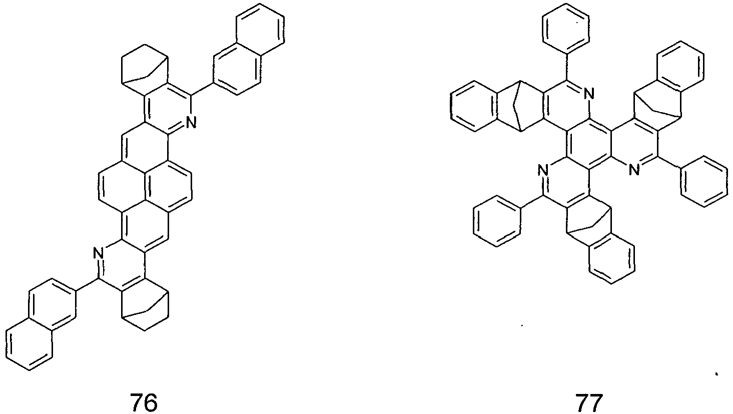

Die Verbindungen mit mindestens einer Struktur der Formeln 68 bis 77 können bevorzugt in Form eines Diastereomerengemisches vorliegen und/oder eingesetzt werden.

Weiterhin umfassen besonders bevorzugte Verbindungen Strukuren gemäß den folgenden Formeln 78 bis 85:

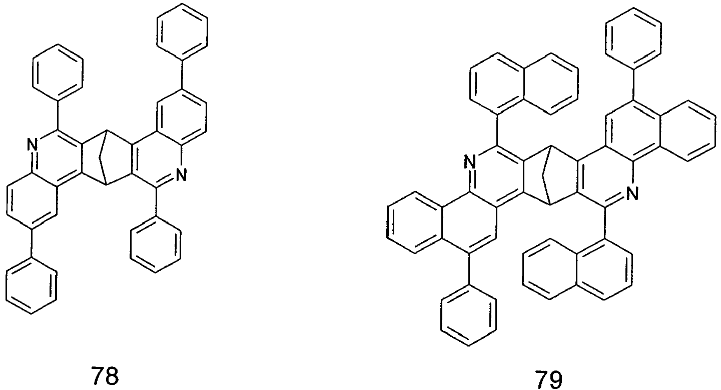

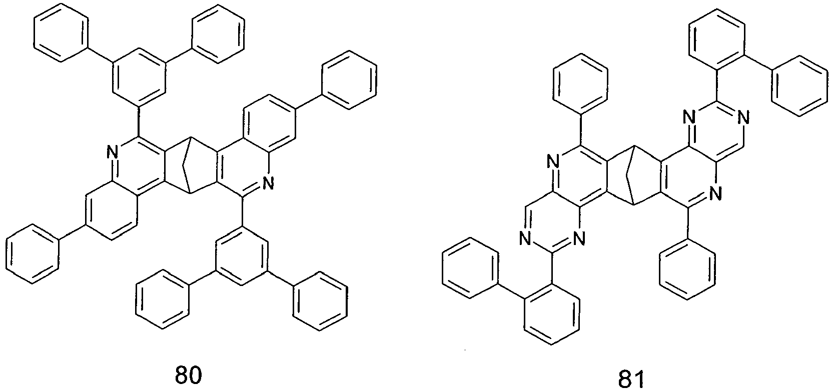

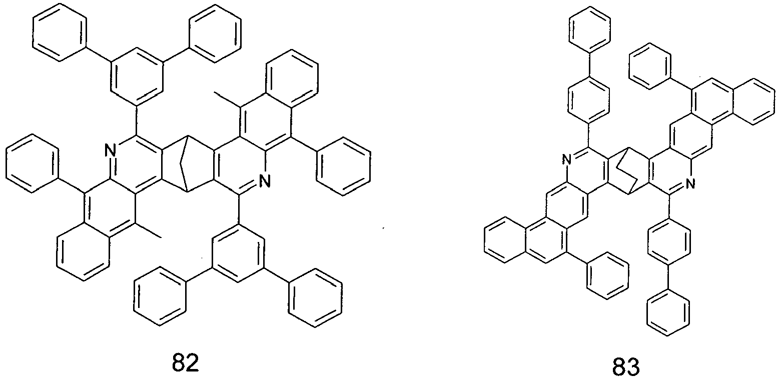

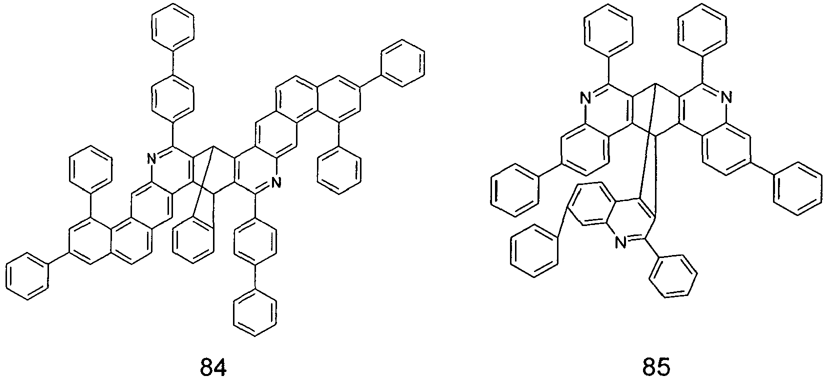

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I) und/oder (II) durch Umsetzung mindestens eines Aldehyds mit mindestens einem aromatischen oder heteroaromatischen Amin und mindestens einem bicyclischen Olefin. Zur Umsetzung können auch Mischungen von Aldehyden, aromatischen Aminen und/oder bicyclischen Olefinen eingesetzt werden. Bevorzugt werden jedoch reine Stoffe umgesetzt, um ein definiertes Produkt zu erhalten.

Eine Umsetzung ergibt sich beispielhaft gemäß folgendem Schema:

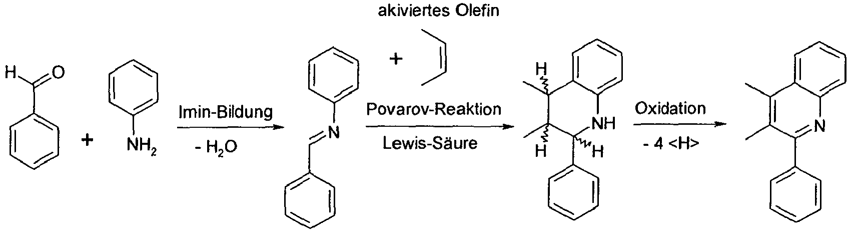

Die erfindungsgemäßen Verbindungen sind vorzugsweise durch Lewissäure-katalysiert Umsetzung von Arylaminen mit Aldehyden zu Iminen und nachgelagerte Povarov-Reaktion mit aktivierten Olefinen zu Tetrahydro-iso-chinolinen und deren anschließende Dehydrierung zugänglich. Die Reaktionssequenz Iminbildung und Povarov-Reaktion kann dabei bevorzugt als Eintopfverfahren durchgeführt werden. Als aktivierte Olefine finden bi- und tri-cyclische Olefine, zum Beispiel vom Bicyclo[2.2.1]- (Norbornen) und Bicyclo[2.2.2]-Typ (Barrelen) Verwendung.

Als Lewissäuren kommen Hautgruppenelementverbindungen wie Bor-, Aluminium-, Gallium- und Indiumhalogenide sowie deren Addukte, besonders bevorzugt Bortrifluorid, Bortrifluorid-Etherate, Aluminiumtrichlorid, Galliumtrichlorid, Indiumtrichlorid, Phosphor-, Bismut- und Antimonhalogenide wie Bismuttrichlorid oder aber auch Übergangmetall- und Lanthanoidverbindungen, bevorzugt Zr, Cu, Ag, Au, Zn, Y, La, Yb-Salze, wie Halogenide, Acetate und Triflate, zum Einsatz.

Die Dehydrierung des substituierten Tetrahydro-iso-chinolins kann nach literaturbekannten Verfahren, z.B. durch Übergangsmetall-katalysierte Dehydrieung bzw. Transferdehydrierung, oder durch Oxidation mit Metalloxiden (z.B. Mangandioxid) oder Chinonen (z.B. DDQ) erfolgen.

Ferner können Aldehyde mit einer, bevorzugt zwei oder mehr Aldehydgruppen verwendet werden, wobei vorzugsweise aromatische oder heteroaromatische Aldehyde eingesetzt werden. Zu den bevorzugten Aldehyden gehören unter anderem die in den Synthesebeispielen dargelegten Verbindungen.

Zu den bevorzugten Monoaldehyden zählen beispielsweise Benzaldehyd (CAS 100-52-7), mit Alkylgruppen substituierte Benzaldehyde, 2-Naphthaldehyd (CAS 66-99-9), 6-tert-Butyl-3-formylpyridin (CAS 391900-69-9), 2-tert-Butylpyrimidin-5-carbaldehyd (CAS 104461-06-5), 3-Phenylbenzaldehyd (CAS 1204-60-0), 2-Benzofurancarboxaldehyd (CAS 4265-16-1), 3-Dibenzofurancarboxaldehyd (CAS 51818-91-8), 9,9'-Spirobi[9H-fluorene]-2-carboxaldehyd (CAS 124575-66-2) und [1,1':3',1"-Terphenyl]-5'-carboxaldehyd (CAS 220955-80-6).

Zu den bevorzugten Dialdehyden zählen unter anderem 1,3-Phthalaldehyd (CAS 626-19-7), 1,4-Phthalaldehyd (CAS 623-27-8), 1,5-Naphthalenedicarboxaldehyd (CAS 7141-15-3), 1,6-Pyrenedicarboxaldehyde (CAS 252338-01-5) und 9,9'-Spirobi[9H-fluorene]-2,7-dicarboxaldehyd, (CAS 856014-12-5).

Ferner können aromatische oder heteroaromatische Amine mit einer, bevorzugt zwei oder mehr Amingruppen verwendet werden. Zu den bevorzugten aromatische oder heteroaromatische Aminen gehören unter anderem die in den Synthesebeispielen dargelegten Verbindungen.

Zu den bevorzugten Monoaminen zählen beispielsweise Anilin (CAS 62-63-3), 4-Methylanilin (CAS 106-49-0), 3,5-Di-Methylanilin, 4-t-Butylanilin (CAS 769-92-6), 4-Phenylanilin (CAS 92-67-1), 2-tert-Butyl-5-aminopyrimidin (CAS 59950-55-9), 2-Benzofuranamin (CAS 139266-08-3), 2-Aminobenzo[b]thiophen (CAS 4521-30-6), 1-Aminonaphthalen (CAS 134-32-7), 8-Aminochinolin (CAS 578-66-5), 1-Anthracenamin (CAS 610-49-1), 2-Anthracenamin (CAS 613-13-8) und 1-Phenazinamin (9Cl) (CAS 2876-22-4).

Zu den bevorzugten Diaminen zählen unter anderem p-Phenylendiamin (CAS 106-50-3), m-Phenylendiamin (CAS 108-45-2), o-Phenylendiamin (CAS 95-54-5), 1,5-Naphthalendiamin (CAS 2243-62-1) und 1,5-Naphthyridin-4,8-diamin (CAS 64761-26-8).

Ferner können bicyclischen Olefinen mit einer, zwei oder mehr Doppelbindungen verwendet werden. Zu den bevorzugten bicyclischen

Olefinen gehören unter anderem die in den Synthesebeispielen dargelegten Verbindungen.

Zu den bevorzugten bicyclischen Olefinen zählen unter anderem Norbornen (CAS 498-66-8), Norbornadien (CAS 121-46-0), 7,7-Dimethyl norbornen (CAS 6541-60-2), Benzonorbornadien (CAS 4453-90-1), 7-Oxanorbornen (CAS 6705-50-6), 7-Oxabenzonorbornadien (CAS 573-57-9), Bicyclo(2,2,2)-2-octen (CAS 931-64-6), Bicyclo(2,2,2)octa-2,5-dien (CAS 500-23-2), Bicyclo(2,2,2)octa-2,5,7-trien (CAS 500-24-3) und 1,4-Dihydro-1,4-Ethenonaphthalin (CAS 7322-47-6).

Vorzugsweise kann bei einer Umsetzung eine Lewis-Säure als Katalysator eingesetzt werden, wobei mit Vorteil Bortrifluoridetherat (CAS 60-29-7) verwendet werden kann.

Zur Erhalt von Verbindungen mit den erfindungsgemäßen Strukturen, wird im Allgemeinen eine Oxidation durchgeführt. Hierzu sind übliche Oxidationsmittel, beispielsweise Mangandioxid oder Chinone geeignet.

Die zuvor dargelegten Reaktionen sind dem Fachmann im Prinzip unter dem Namen "Povarov-Reaktion" bekannt und in der Literatur, beispielsweise in J. Org. Chem. 75 (2010) 702-715 und in Tetrahedron 65 (2009) 2721-2750 ausführlich mit weiteren Hinweisen beschrieben.

Eine nach der Umsetzung von mindesten einem Aldehyd mit mindestens einem aromatischen Amin und mindestens einem bicyclischen Olefin erhaltene Zwischenverbindung kann in einer Kupplungsreaktion umgesetzt werden.

Geeignete Reaktionen zur Bildung von C-C-Verknüpfungen und/oder C-N-Verknüpfungen sind dem Fachmann bekannt und in der Literatur beschrieben. Besonders geeignete und bevorzugte Kupplungsreaktionen, die alle zu C-C-Verknüpfungen führen, sind solche gemäß BUCHWALD, SUZUKI, YAMAMOTO, STILLE, HECK, NEGISHI, SONOGASHIRA und HIYAMA.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) und/oder Formel (II) in hoher Reinheit, bevorzugt mehr als 99 % (bestimmt mittels ¹H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formeln (I) und/oder (II) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere nach Anspruch 14 enthaltend eine oder mehrere der oben aufgeführten

Strukturen der Formeln (I) und/oder (II) oder erfindungsgemäße Verbindungen, wobei ein oder mehrere Bindungen erfindungsgemäßen Verbindungen oder der Strukturen der Formeln (I) und/oder (II) zum Polymer, Oligomer oder Dendrimer vorhanden sind. Je nach Verknüpfung der Strukturen der Formeln (I) und/oder (II) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Ferner können die vorliegenden Verbindungen ein relativ geringes Molekulargewicht aufweisen. Sie können ein Molekulargewicht von

vorzugsweise höchstens 10000 g/mol, besonders bevorzugt höchstens 5000 g/mol und speziell bevorzugt höchstens 3000 g/mol aufweisen.

Weiterhin zeichnen sich bevorzugte Verbindungen dadurch aus, dass diese sublimierbar sind. Diese Verbindungen weisen im Allgemeinen eine Molmasse von weniger als ca. 1200 g/mol auf.

Von besonderem Interesse sind des Weiteren erfindungsgemäße Verbindungen, die sich durch eine hohe Glasübergangstemperatur auszeichnen. In diesem Zusammenhang sind insbesondere Verbindungen umfassend Strukturen der allgemeinen Formeln (I) oder (II) bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005.

Die Verbindungen können mindestens ein Metallatom ausgewählt aus Iridium, Ruthenium, Palladium, Platin, Osmium oder Rhenium, vorzugsweise Iridium oder Platin aufweisen. Diese Verbindungen sind insbesondere als phosphoreszierende Emitter geeignet. Beispielsweise können die zuvor dargelegten Verbindungen 1 bis 27 sowie die in Beispielen dargelegten Verbindungen A1 bis A27 mit Platin und/oder Iridium zu emittierenden Metallkomplexen umgesetzt werden.

Bevorzugt sind insbesondere Metallkomplexe der Formel (M-1) M(L)ₙ(L')ₘ Formel (M-1), wobei für die verwendeten Symbole und Indizes gilt:
- M: ist Iridium oder Platin;
- L: ist gleich oder verschieden bei jedem Auftreten ein Ligand, umfassend umfassend mindestens eine Struktur der Formeln (I) und/oder (II);
- L': ist gleich oder verschieden bei jedem Auftreten ein beliebiger Coligand;
- n: ist 1, 2 oder 3;
- m: ist 0, 1, 2, 3 oder 4.

Beispielsweise können die entsprechenden freien Liganden L und
gegebenenfalls L' Metallalkoholate der Formel (M-2),
Metallketoketonate der Formel (M-3), Metallhalogenide der Formel (M-4), dimere Metallkomplexe der Formel (M-5) oder Metallkomplexe der Formel (M-6) sein, 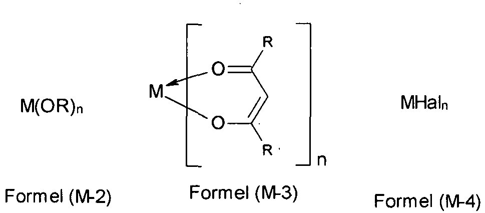 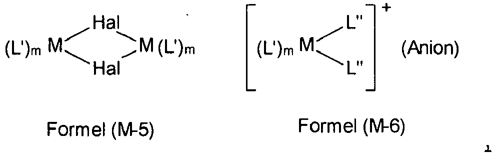
wobei die Symbole M, m, n und R die oben angegebenen Bedeutungen haben, Hal = F, Cl, Br oder I ist, L" für einen Alkohol, insbesondere für einen Alkohol mit 1 bis 4 C-Atomen oder ein Nitril, insbesondere Acetonitril oder Benzonitril, steht und (Anion) ein nicht-koordinierendes Anion ist, wie beispielsweise Triflat.

Es können ebenfalls Metallverbindungen, insbesondere Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [IrCl₂(acac)₂]⁻, beispielsweise Na[IrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl₃·xH₂O, wobei x üblicherweise für eine Zahl zwischen 2 und 4 steht.

Geeignete Platin-Edukte sind beispielsweise PtCl₂, K₂[PtCl₄], PtCl₂(DMSO)₂, Pt(Me)₂(DMSO)₂ oder PtCl₂(Benzonitril)₂.

Die Synthese der Komplexe wird bevorzugt durchgeführt wie in WO 2002/060910, WO 2004/085449 und WO 2007/065523 beschrieben. Heteroleptische Komplexe können beispielsweise auch gemäß WO 2005/042548 synthetisiert werden. Dabei kann die Synthese beispielsweise auch thermisch, photochemisch und/oder durch Mikrowellenstrahlung aktiviert werden. In einer bevorzugten Ausführungsform der Erfindung wird die Reaktion ohne die Verwendung eines zusätzlichen Lösemittels in der Schmelze durchgeführt. Dabei bedeutet "Schmelze", dass der Ligand geschmolzen vorliegt und die Metall-Vorstufe in dieser Schmelze gelöst oder suspendiert ist. Zur Aktivierung der Reaktion ist es weiterhin auch möglich, eine Lewis-Säure, beispielsweise ein Silbersalz oder AtCl₃, zuzugeben.

Nochmals ein weiterer Gegenstand der vorliegenden Erfindung ist eine Formulierung, enthaltend eine erfindungsgemäße Verbindung bzw. ein erfindungsgemäßes Oligomer, Polymer oder Dendrimer und mindestens eine weitere Verbindung. Die weitere Verbindung kann beispielsweise ein Lösemittel sein. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Nochmals ein weiterer Gegenstand der vorliegenden Erfindung ist eine Zusammensetzung enthaltend eine erfindungsgemäße Verbindung und wenigstens ein weiteres organisch funktionelles Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

Die oben beschriebenen Verbindungen, umfassend Strukturen der Formeln (I) und/oder (II), bzw. die oben aufgeführten bevorzugten Ausführungsformen können in einer elektronischen Vorrichtung bevorzugt als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung, umfassend Strukturen der Formeln (I) und/oder (II), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen FeldEffekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine Verbindung, umfassend Strukturen der Formeln (I) und/oder (II).
Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrix-materialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen. Weiterhin können die erfindungsgemäßen Verbindungen zur Erzeugung von Singulett-Sauerstoff oder in der Photokatalyse eingesetzt werden.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) und/oder (II) bzw. die oben aufgeführten bevorzugten Ausführungsformen als emittierende Verbindung und/oder als Matrixmaterial in einer oder mehreren emittierenden Schichten, bevorzugt in Kombination mit einem weiteren Matrixmaterial und/oder einer weiteren emittierenden Verbindung.

Hierbei kann mindestens eine erfindungsgemäße Verbindung, vorzugsweise ein erfindungsgemäßer Metallkomplex umfassend Strukturen gemäß Formel (I) und/oder (II) als emittierende Verbindung in einer emittierenden Schicht eingesetzt werden, bevorzugt in Kombination mit einem Matrixmaterial, welches Verbindungen mit Strukturen gemäß Formel (I) oder (II) umfasst. Ferner kann ein erfindungsgemäßer Metallkomplex umfassend Strukturen gemäß Formel (I) und/oder (II) mit einem Matrixmaterial ohne Strukturen gemäß Formel (I) und/oder (II) kombiniert werden. Weiterhin kann eine erfindungsgemäße Verbindung umfassend Strukturen gemäß Formel (I) und/oder (II) als Matrixmaterial eingesetzt werden, wobei dieses Matrixmaterial mit einem Metallkomplex ohne Strukturen gemäß Formel (I) und/oder (II) kombiniert werden kann.

Wenn die efindungsgemäße Verbindung umfassend Strukturen gemäß Formel (I) und/oder (II) als emittierende Verbindung in einer emittierenden Schicht eingesetzt wird, wird sie bevorzugt in Kombination mit einem oder mehreren Matrixmaterialien eingesetzt. Die Mischung aus der efindungsgemäßen Verbindung umfassend Strukturen gemäß Formel (I) und/oder (II) und dem Matrixmaterial enthält zwischen 0.1 und 99 Vol.-%, vorzugsweise zwischen 1 und 90 Vol.-%, besonders bevorzugt zwischen 3 und 40 Vol.-%, insbesondere zwischen 5 und 15 Vol.-% der efindungsgemäßen Verbindung umfassend Strukturen gemäß Formel (I) und/oder (II) bezogen auf die Gesamtmischung aus Emitter und Matrixmaterial. Entsprechend enthält die Mischung zwischen 99.9 und 1 Vol.-%, vorzugsweise zwischen 99 und 10 Vol.-%, besonders bevorzugt zwischen 97 und 60 Vol.-%, insbesondere zwischen 95 und 85 Vol.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß
WO 2004/013080, WO 2004/093207, WO 2006/005627 oder
WO 2010/006680, Triarylamine, Carbazolderivate, z. B. CBP (N,N-Bis-carbazolylbiphenyl), m-CBP oder die in WO 2005/039246,
US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß
WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder
WO 2008/056746, Zinkkomplexe, z. B. gemäß EP 652273 oder
WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Weiterhin können die erfindungsgemäßen Verbindungen als Matrixmaterialien eingesetzt werden. Hierbei können die erfindungsgemäßen Matrixmaterialien sowohl zusammen mit emittierenden Verbindungen eingesetzt werden, die mindestens eine Struktur gemäß Formeln (I) und/oder (II) umfassen, als auch mit emittierenden Verbindungen die keine Struktur gemäß Formeln (I) und/oder (II) aufweisen. In diesem Zusammenhang ist festzuhalten, dass die zuvor genannten Verhältnisse von Matrixmaterial zu emittierenden Verbindungen entsprechend gelten.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons, eines Triazin-Derivats oder eines Phosphinoxid-Derivats mit einem Triarylamin-Derivat oder einem Carbazol-Derivat als gemischte Matrix für den erfindungsgemäßen Metallkomplex. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise die erfindungsgemäßen Verbindungen umfassend Strukturen gemäß Formel (I) und/oder (II) als Co-Matrix für längerwellig emittierende Triplettemitter, beispielsweise für grün oder rot emittierende Triplettemitter, eingesetzt werden.

Die erfindungsgemäßen Verbindungen lassen sich auch in anderen Funktionen in der elektronischen Vorrichtung einsetzen, beispielsweise als Lochtransportmaterial in einer Lochinjektions- oder -transportschicht, als Ladungserzeugungsmaterial oder als Elektronenblockiermaterial. Ebenso lassen sich die erfindungsgemäßen Komplexe als Matrixmaterial für andere phosphoreszierende Metallkomplexe in einer emittierenden Schicht einsetzen.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Weiterhin ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren lochleitende Schichten, besonders bevorzugt als lochleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine elektronischen Vorrichtung, insbesondere eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß Formel (I) und/oder (II) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronische Vorrichtungen, insbesondere organische Elektrolumineszenzvorrichtungen enthaltend erfindungsgemäße Verbindungen umfassend Strukturen gemäß Formel (I) und/oder (II) bzw. die oben aufgeführten bevorzugten Ausführungsformen, angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronischen Vorrichtungen, insbesondere organische Elektrolumineszenzvorrichtungen enthaltend Verbindungen, Oligomere, Polymere oder Dendrimere mit Strukturen gemäß Formel (I) und/oder (II) als emittierende Materialien, als elektronenleitende Materialien oder als lochleitende Materialien weisen eine sehr gute Lebensdauer auf.
2. Elektronischen Vorrichtungen, insbesondere organische Elektrolumineszenzvorrichtungen enthaltend Verbindungen, Oligomere, Polymere oder Dendrimere mit Strukturen gemäß Formel (I) und/oder (II) als emittierende Materialien, als elektronenleitende Materialien oder als lochleitende Materialien weisen eine hervorragende Effizienz auf. Insbesondere ist die Effizienz deutlich höher gegenüber analogen Verbindungen, die keine Struktureinheit gemäß Formel (I) oder Formel (II) enthalten.
3. Die erfindungsgemäßen Verbindungen, umfassend mindestens ein Metallatom, vorzugsweise ausgewählt aus Ir oder Pt, weisen teilweise ein sehr schmales Emissionsspektrum auf, was zu einer hohen Farbreinheit der Emission führt, wie sie insbesondere für Displayanwendungen wünschenswert ist.
4. Die erfindungsgemäßen Verbindungen, umfassend mindestens ein Metallatom, vorzugsweise ausgewählt aus Ir oder Pt, weisen im Vergleich zu analogen Verbindungen, welche keine Struktureinheit gemäß Formel (I) oder Formel (II) enthalten, eine reduzierte Aggregation auf. Dies äußert sich in einer niedrigeren Sublimationstemperatur sowie einer höheren Löslichkeit sowie in der Elektrolumineszenzvorrichtung in einer verringerten Triplett-Triplett-Löschung.
5. Die erfindungsgemäßen Verbindungen, Oligomere, Polymere oder Dendrimere mit Strukturen gemäß Formel (I) und/oder (II) zeigen eine sehr hohe Stabilität und führen zu Verbindungen mit einer sehr hohen Lebensdauer.
6. Mit Verbindungen, Oligomere, Polymere oder Dendrimere mit Strukturen gemäß Formel (I) und/oder (II) kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
7. Die Verwendung von Verbindungen, Oligomere, Polymere oder Dendrimere mit Strukturen gemäß Formel (I) und/oder (II) in Schichten elektronischer Vorrichtungen, insbesondere organischer Elektrolumineszenzvorrichtungen führt zu einer hohen Mobilität der Elektron-Leiterstrukturen.
8. Verbindungen, Oligomere, Polymere oder Dendrimere mit Strukturen gemäß Formel (I) und/oder (II) zeichnen sich durch eine ausgezeichnete thermische Stabilität aus, wobei Verbindungen mit einer Molmasse von weniger als ca. 1200 g/mol gut sublimierbar sind
9. Verbindungen, Oligomere, Polymere oder Dendrimere mit Strukturen gemäß Formel (I) und/oder (II) weisen eine ausgezeichnete Glasfilmbildung auf.
10. Verbindungen, Oligomere, Polymere oder Dendrimere mit Strukturen gemäß Formel (I) und/oder (II) bilden aus Lösungen sehr gute Filme.

Diese oben genannten Vorteile gehen nicht mit einer Verschlechterung der weiteren elektronischen Eigenschaften einher.

Ein weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Lochinjektionsmaterial, Lochtransportmaterial (HTM), Lochblockiermaterial (HBM), Elektronentransportmaterial (ETM) Elektroneninjektionsmaterial, Elektronenblockiermaterial und/oder Emittermaterial, beispielsweise als Triplettemittermaterial (TTM) oder blauen Singulettemitter (SEB).

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Allgemeines Darstellungsverfahren: Umsetzung von Monoaldehyd mit Monoamin und Monoolefin

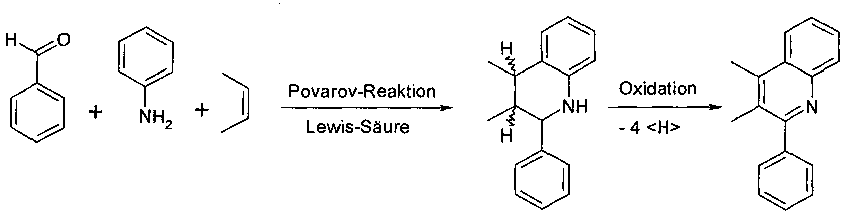

Ein gut gerührtes Gemisch aus 500 mmol des Arylamins, 550 mmol des Arylaldehyds, 1 mol des aktivierten Olefins und 1300 ml Dichlormethan wird mit 100 mmol der Lewis-Säure versetzt und dann 40 h unter Rückfluss erhitzt. Nach Erkalten wäscht man die Reaktionsmischung zweimal mit je 400 ml Wasser, trocknet die organische Phase über Magnesiumsulfat und entfernt dann das Dichlormethan im Vakuum. Der Rückstand wird in 1000 ml o-Dichlorbenzol aufgenommen, mit 5 mol Mangandioxid versetzt und 16 h am Wasserabscheider unter Rückfluss erhitzt. Nach Erkalten fügt man 1000 ml Ethylacetat zu, saugt vom Mangandioxid über eine Celite-Schicht ab, wäscht das Mangandioxid mit 1000 ml Ethylacetat nach und befreit die vereinigten Filtrate im Vakuum von den Lösungsmitteln. Der Rückstand wird umkristallisiert und abschließend durch fraktionierte Sublimation (p ca. 10⁻⁴ - 10⁻⁶ mbar, T ca. 150 - 400°C) von Leichtsiedern und nicht flüchtigen Nebenkomponenten befreit. Verbindungen mit einer Molmasse größer als ca. 1200 g/mol werden bevorzugt durch Tempern im Hochvakuum von Lösungsmittelresten befreit.

Werden polyfunktionelle Bausteine verwendet, wird die Stöchiometrie entsprechend angepasst.

### Beispiel A1

### 7,8,9, 10-Tetrahydro-7,10-methano-6-phenylphenanthridin

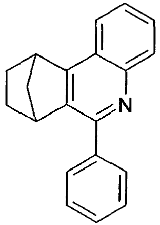

Ein gut gerührtes Gemisch aus 46.6 g (500 mmol) Anilin [62-63-3], 58.4 g (550 mmol) Benzaldehyd [100-52-7], 94.2 g (1 mol) Norbornen [498-66-8] und 1300 ml Dichlormethan wird tropfenweise mit 14.2 g (100 mmol) Bortrifluoridetherat [60-29-7] versetzt und dann 40 h unter Rückfluss erhitzt. Nach Erkalten wäscht man die Reaktionsmischung zweimal mit je 400 ml Wasser, trocknet die organische Phase über Magnesiumsulfat und entfernt dann das Dichlormethan im Vakuum. Der Rückstand wird in 1000 ml o-Dichlorbenzol aufgenommen, mit 435 g (5 mol) Mangandioxid versetzt und 16 h am Wasserabscheider unter Rückfluss erhitzt. Nach Erkalten fügt man 1000 ml Ethylacetat zu, saugt vom Mangandioxid über eine Celite-Schicht ab, wäscht das Mangandioxid mit 1000 ml Ethylacetat nach und befreit die vereinigten Filtrate im Vakuum von den Lösungsmitteln. Der Rückstand wird zweimal aus Cyclohexan umkristallisiert und abschließend durch fraktionierte Sublimation (p ca. 10⁻⁴ - 10⁻⁵ mbar, T ca. 230°C) von Leichtsiedern und nicht flüchtigen Nebenkomponenten befreit. Ausbeute: 76.0 g (280 mmol), 56%; Reinheit: ca. 99.5%-ig nach ¹H NMR.

### Analog können folgende Verbindungen dargestellt werden:

| **Bsp.** | **Edukte** | **Produkt** | **Ausbeute** |
|---|---|---|---|
| **Typ A: Monoaldehyd + Monoamin + Monoolefin** | | | |
| A2 | 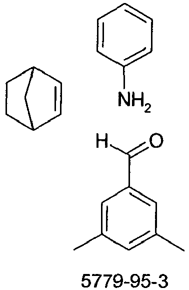 | 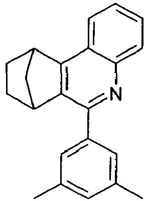 | 66% |
| A3 | 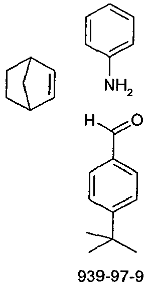 | 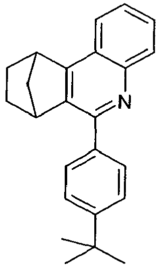 | 64% |
| A4 | 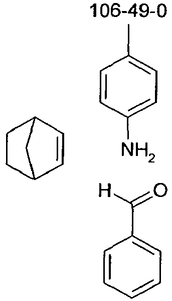 | 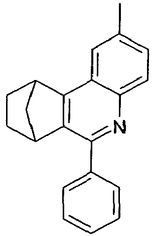 | 56% |
| A5 | 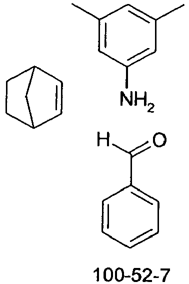 | 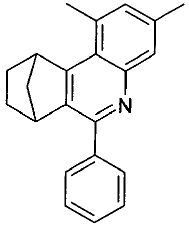 | 58% |
| A6 | 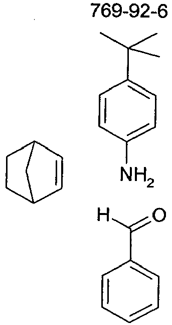 | 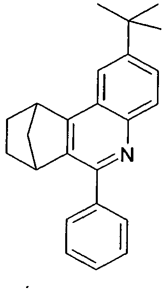 | 61% |
| A7 | 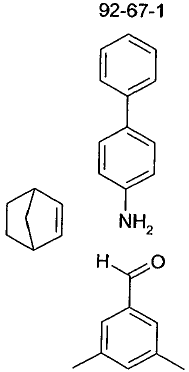 | 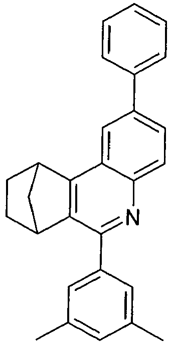 | 63% |
| A8 | 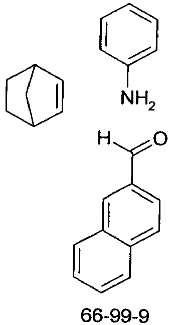 | 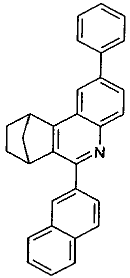 | 58% |
| A9 | 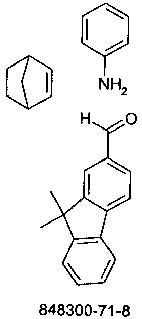 | 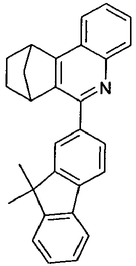 | 55% |
| A10 | 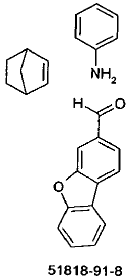 | 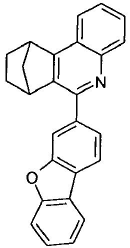 | 60% |
| A11 | 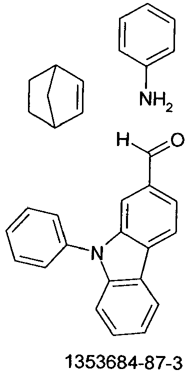 | 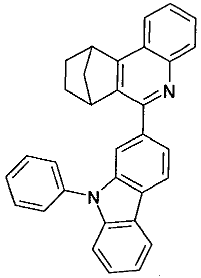 | 34% |
| A12 | 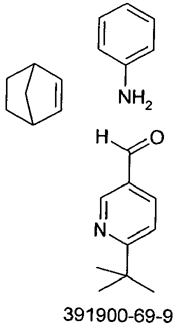 | 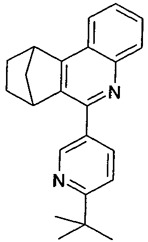 | 69% |
| A13 | 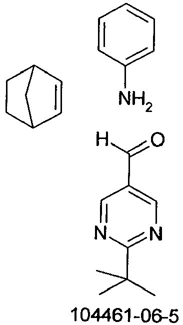 | 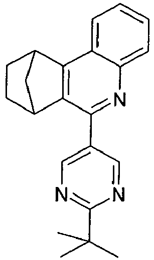 | 67% |
| A14 | 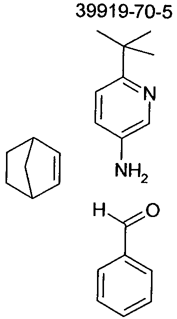 | 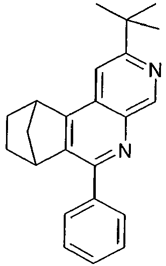 | 23% |
| | | 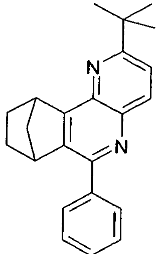 | 17% |
| | | Chromatographische Trennung der Regioisomeren | |
| A15 | 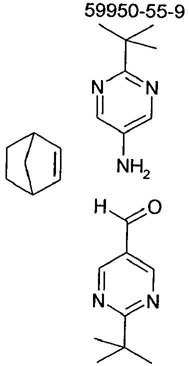 | 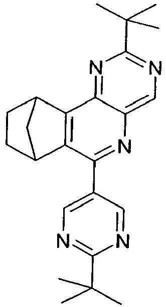 | 50% |
| A16 | 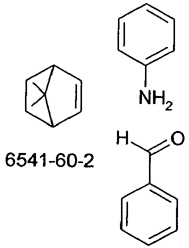 | 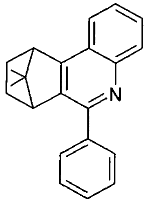 | 48% |
| A17 | 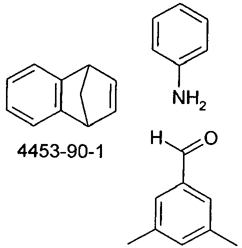 | 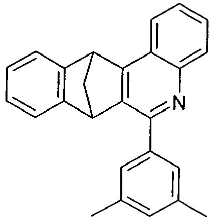 | 68% |
| A18 | 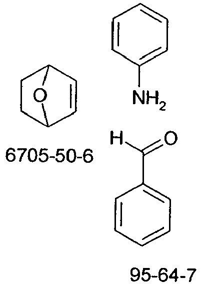 | 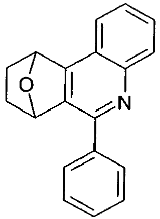 | 45% |
| A19 | 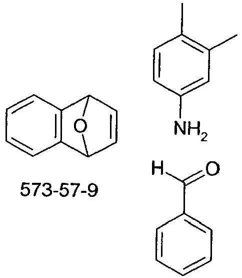 | 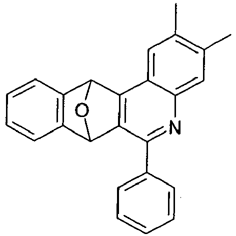 | 65% |
| A20 | 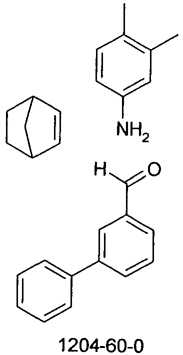 | 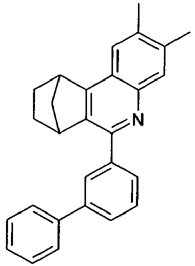 | 54% |
| A21 | 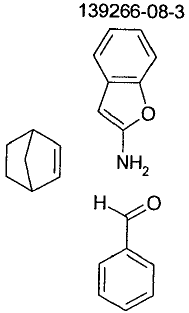 | 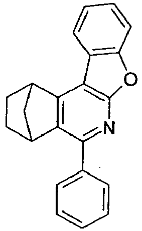 | 38% |
| A22 | 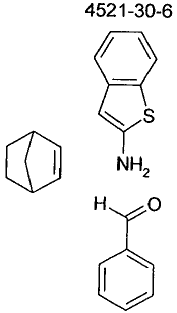 | 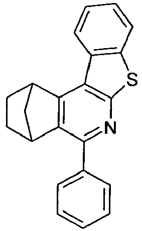 | 34% |
| A23 | 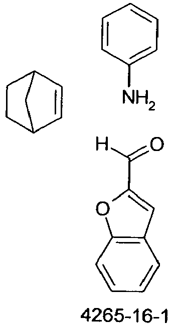 | 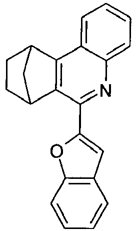 | 36% |
| A24 | 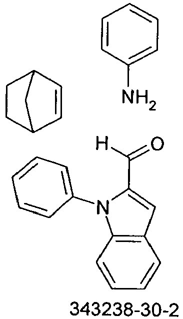 | 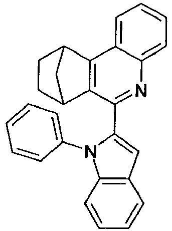 | 28% |
| A25 | 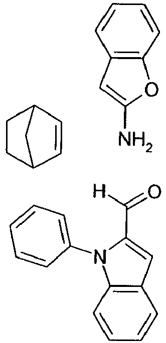 | 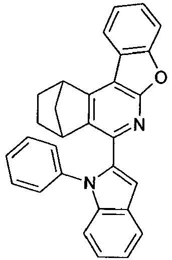 | 32% |
| A26 | 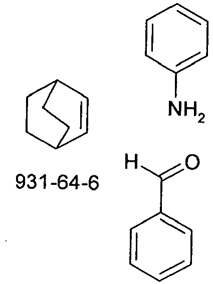 | 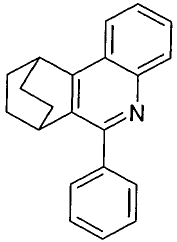 | 25% |
| A27 | 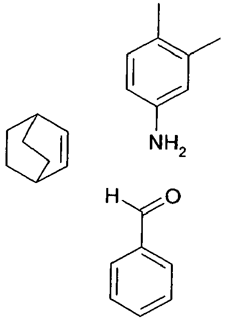 | 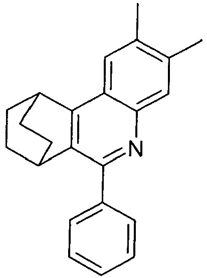 | 23% |
| A28 | 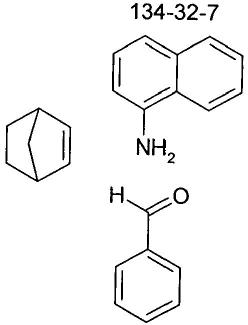 | 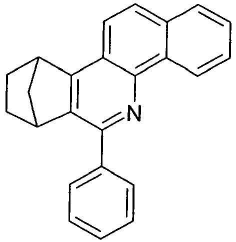 | 34% |
| A29 | 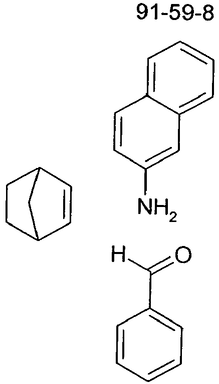 | 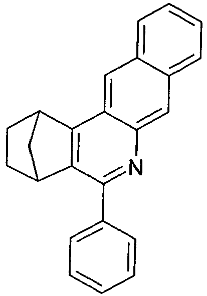 | 45% |
| A30 | 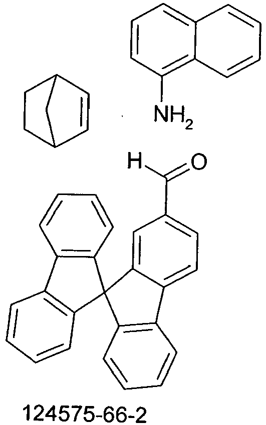 | 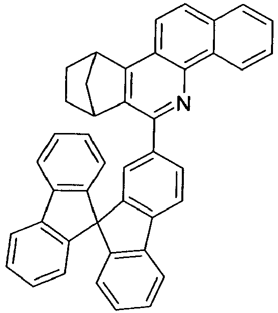 | 46% |
| A31 | 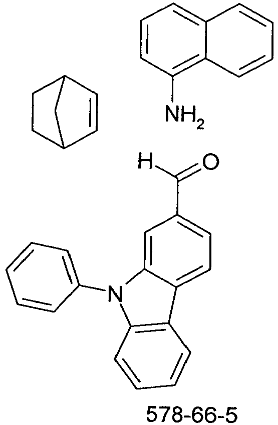 | 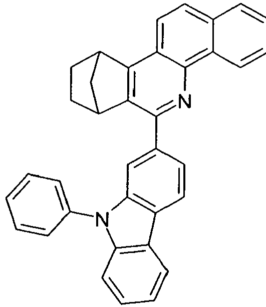 | 50% |
| A32 | 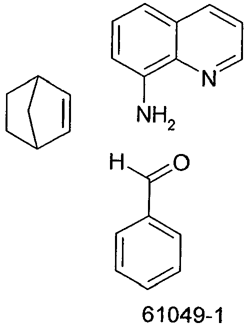 | 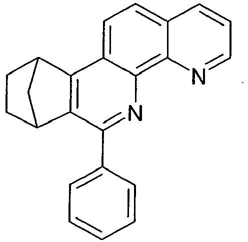 | 53% |
| A33 | 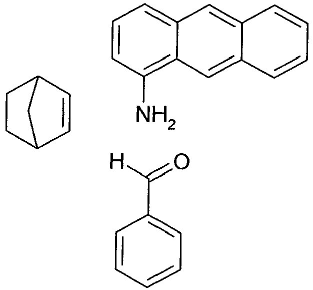 | 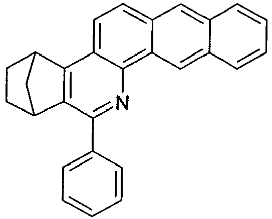 | 33% |
| A34 | 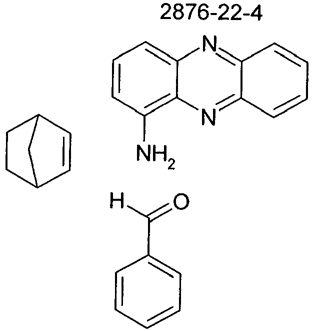 | 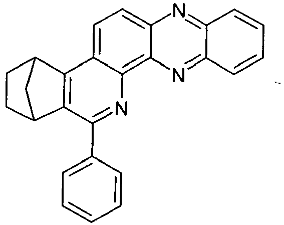 | 28% |
| A35 | 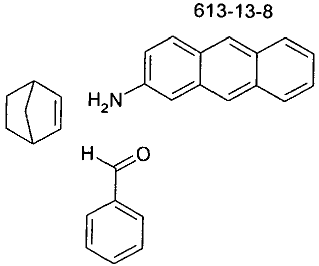 | 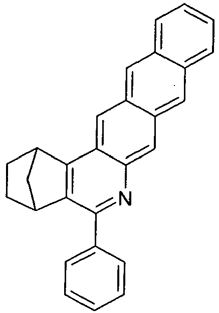 | 23% |
| A36 | 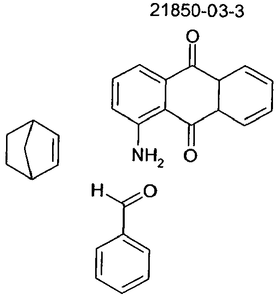 | 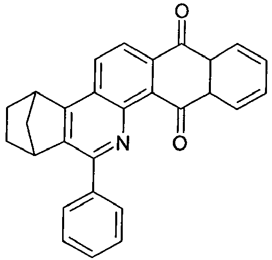 | 30% |
| A37 | 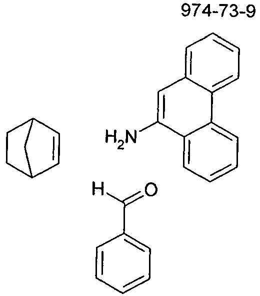 | 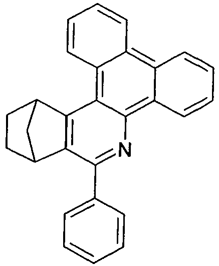 | 46% |
| A38 | 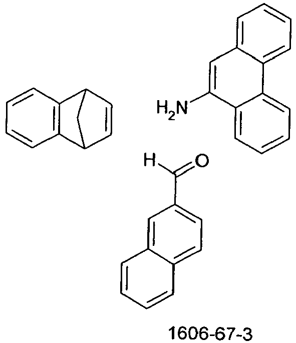 | 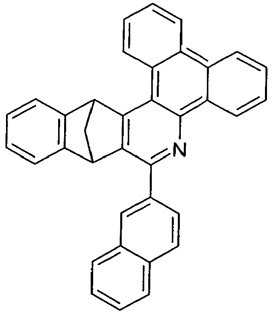 | 47% |
| A39 | 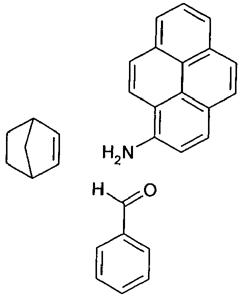 | 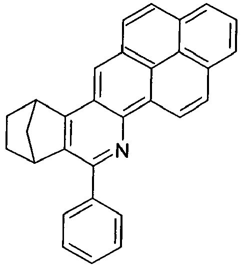 | 51% |
| A40 | 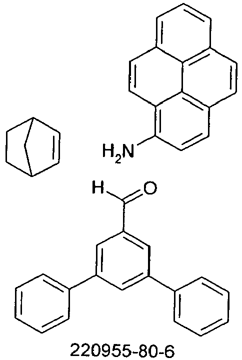 | 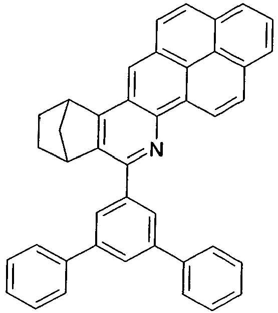 | 49% |
| A41 | 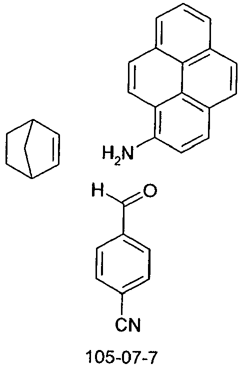 | 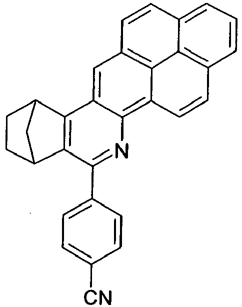 | 47% |
| A42 | 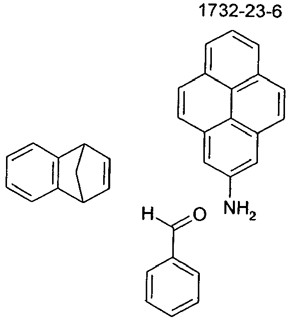 | 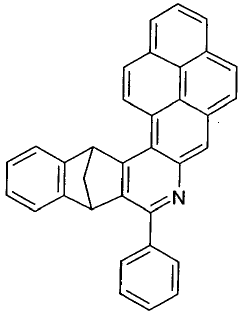 | 44% |
| A43 | 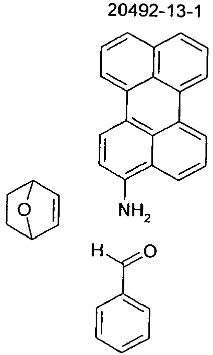 | 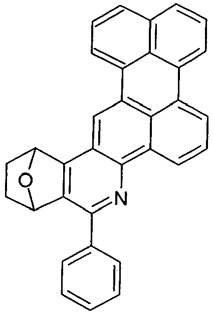 | 43% |
| A44 | 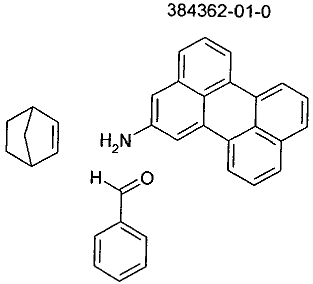 | 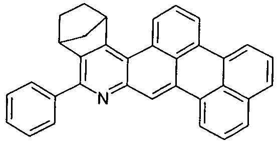 | 38% |
| A45 | 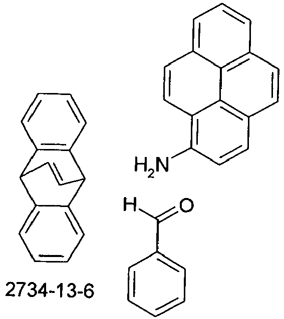 | 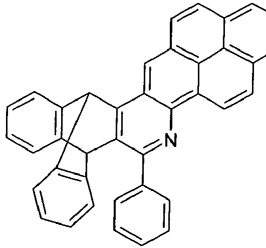 | 45% |
| A46 | 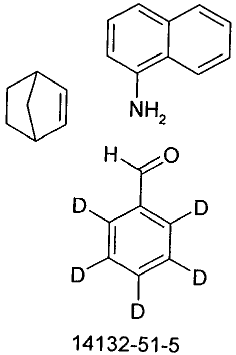 | 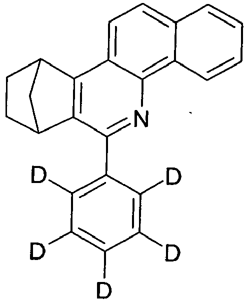 | 47% |
| A47 | 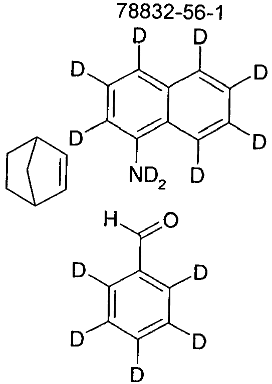 | 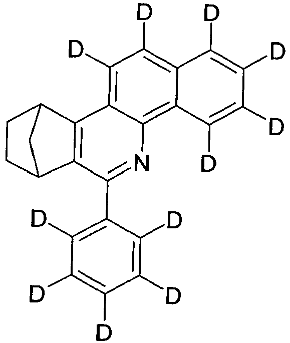 | 48% |
| A48 | 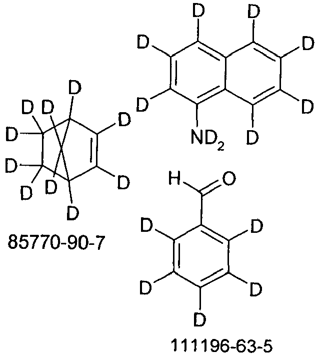 | 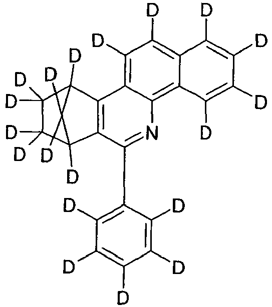 | 48% |
| A49 A50* | 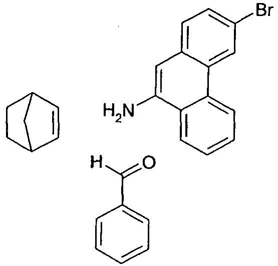 | 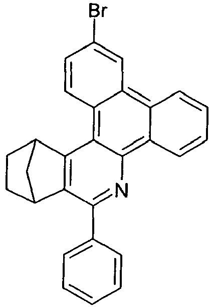 | 50% |
| | 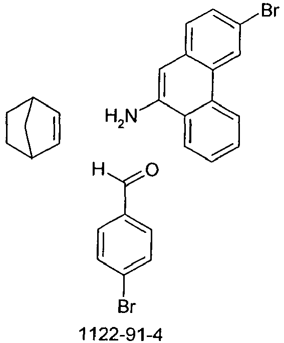 | 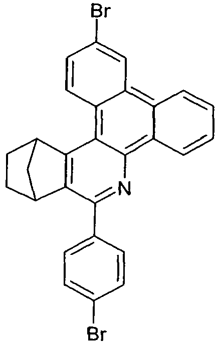 | 49% |
| A51* | 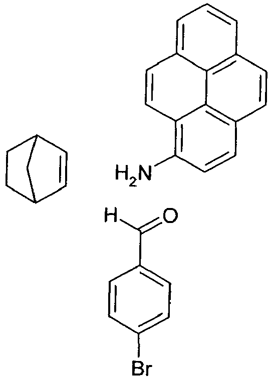 | 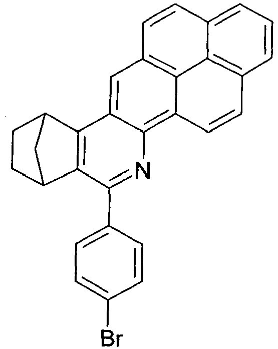 | 45% |
| * Referenzverbindung | | | |
| A52 | 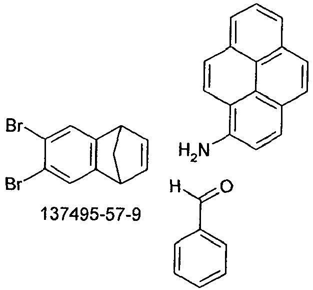 | 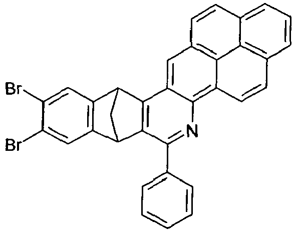 | 32% |

| **Typ B: Dialdehyd + Monoamin + Monoolefin** | | | |
|---|---|---|---|
| B1 | 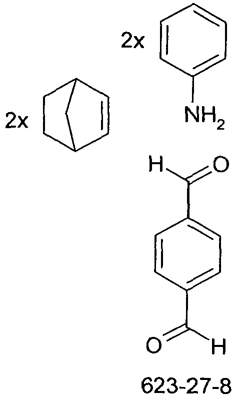 | 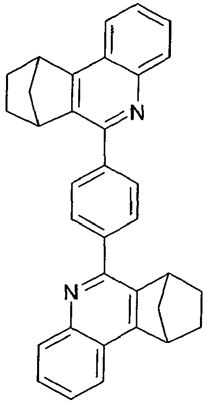 | 30% |
| | | Diastereomerengemisch | |
| B2 | 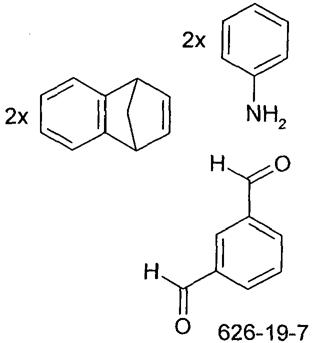 | 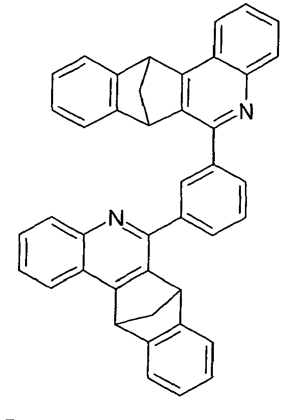 | 33% |
| | | Diastereomerengemisch | |
| B3 | 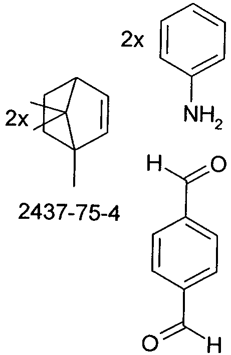 | 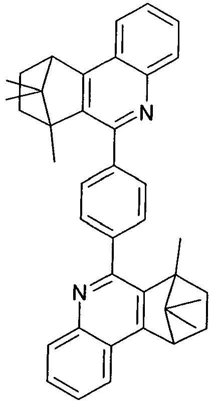 | 30% |
| B4 | 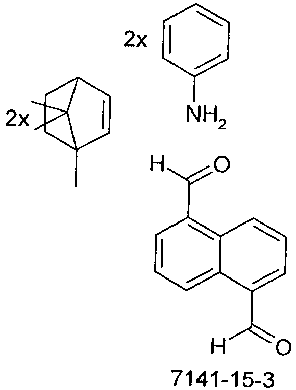 | 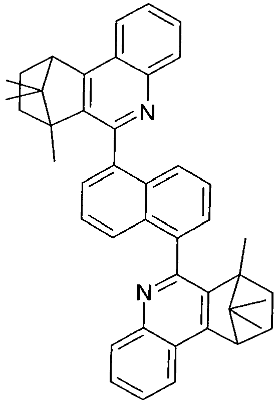 | 28% |
| B5 | 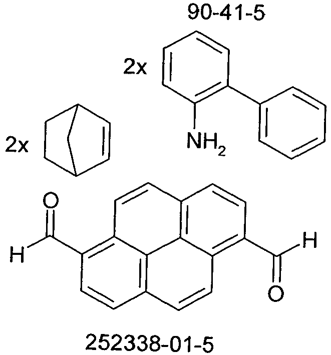 | 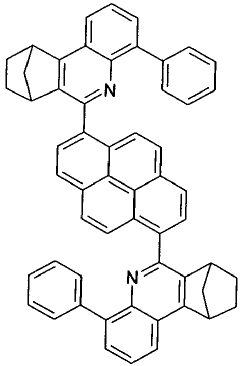 | 35% |
| | | Diastereomerengemisch | |
| B6 | 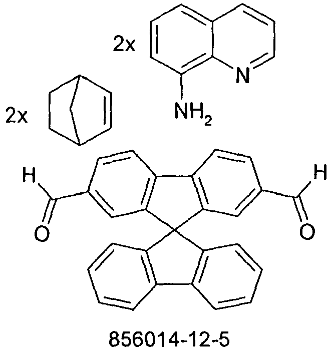 | 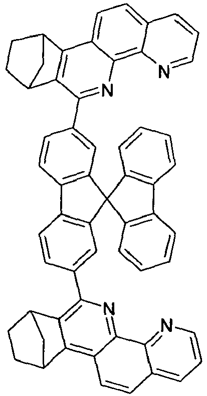 | 23% |
| | | Diastereomerengemisch | |
| B7 | 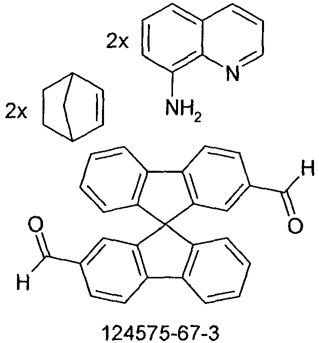 | 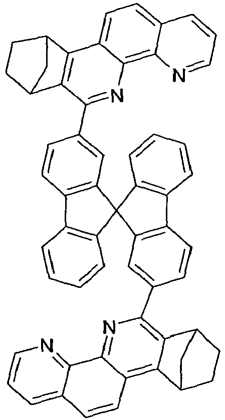 | 25% |
| | | Diastereomerengemisch | |

| **Typ C: Trialdehyd + Monoamin + Monoolefin** | | | |
|---|---|---|---|
| C1 | 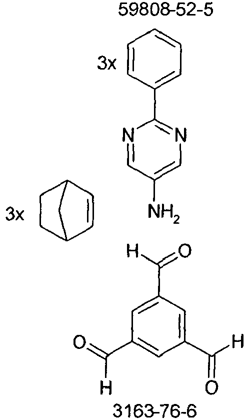 | 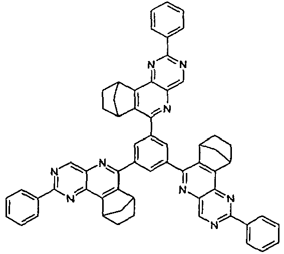 | 19% |
| | | Diastereomerengemisch | |

| **Typ D: Monoaldehyd + Diamin + Monoolefin** | | | |
|---|---|---|---|
| D1 | 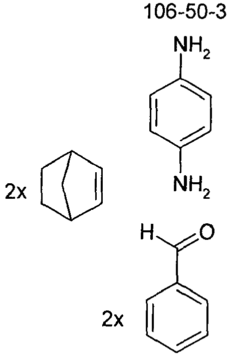 | 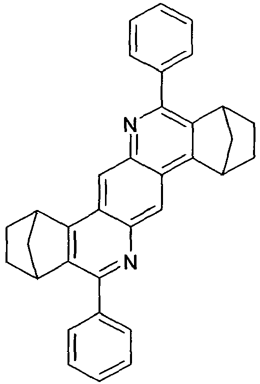 | 27% |
| | | Diastereomerengemisch | |
| D2 | 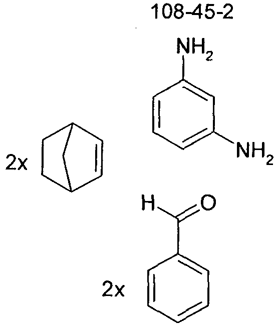 | 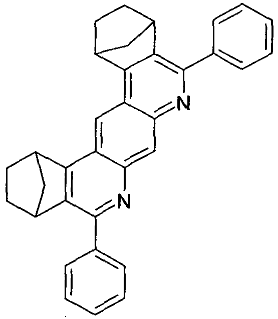 | 24% |
| | | Diastereomerengemisch | |
| D3 | 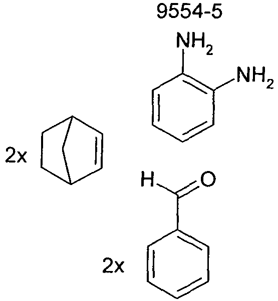 | 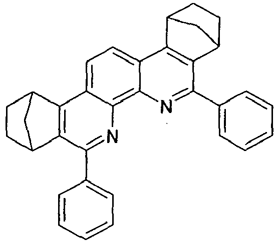 | 30% |
| | | Diastereomerengemisch | |
| D4 | 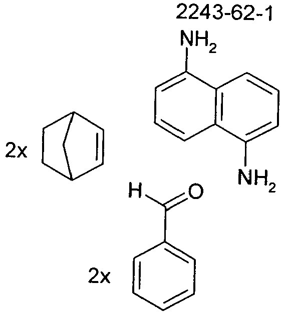 | 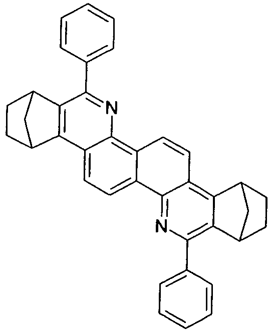 | 29% |
| | | Diastereomerengemisch | |
| D5 | 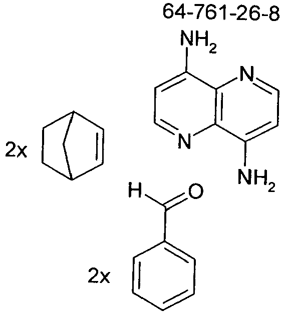 | 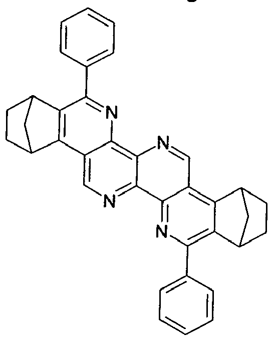 | 24% |
| | | Diastereomerengemisch | |
| D6 | 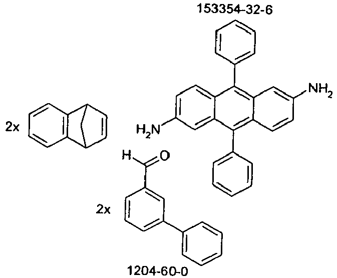 | 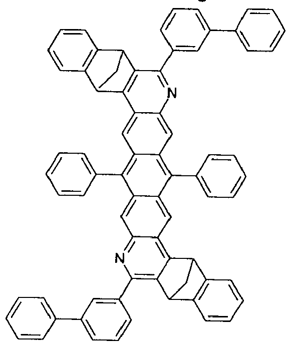 | 20% |
| | | Diastereomerengemisch | |
| D7 | 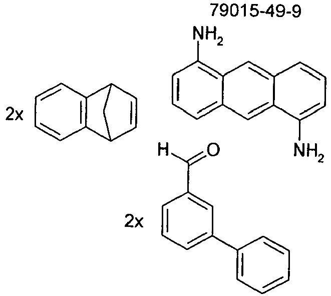 | 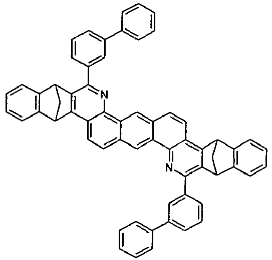 | 26% |
| | | Diastereomerengemisch | |
| D8 | 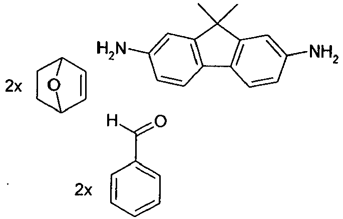 | 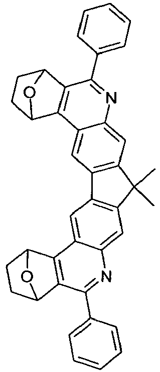 | 28% |
| | | Diastereomerengemisch | |
| D9 | 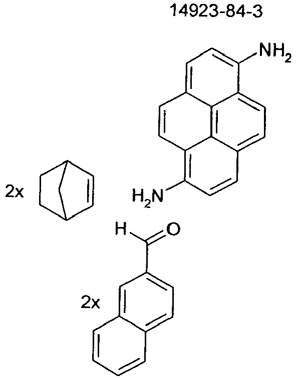 | 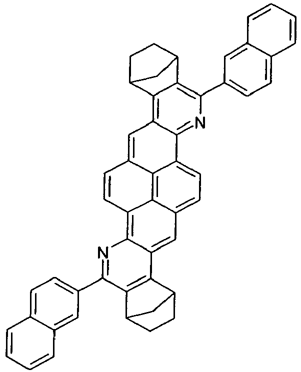 | 25% |
| | | Diastereomerengemisch | |

| **Typ E: Monoaldehyd + Triamin + Monoolefin** | | | |
|---|---|---|---|
| E1 | 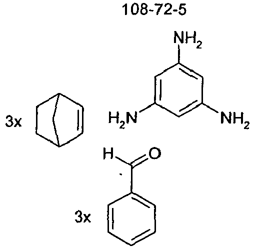 | 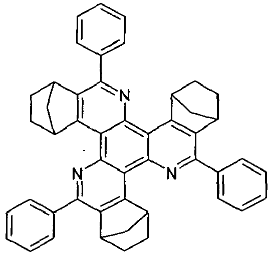 | 19% |
| | | Diastereomerengemisch | |

| **Typ F: Monoaldehyd + Monoamin + Diolefin** | | | |
|---|---|---|---|
| F1 | 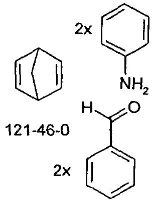 | 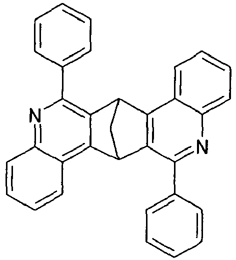 | 26% |
| F2 | 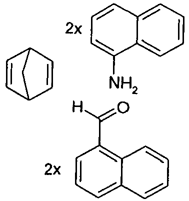 | 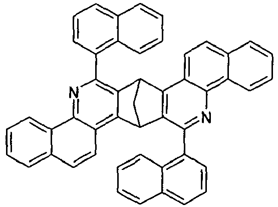 | 25% |
| F3 | 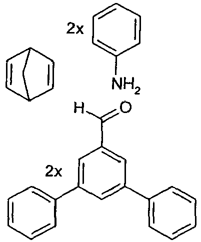 | 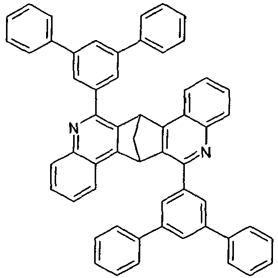 | 28% |
| F4 | 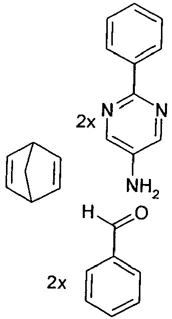 | 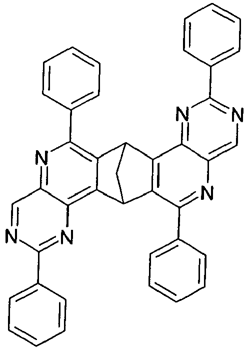 | 31% |
| F5 | 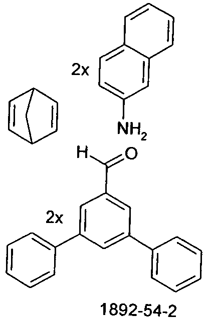 | 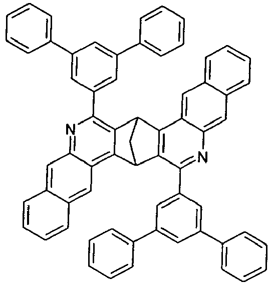 | 20% |
| F6 | 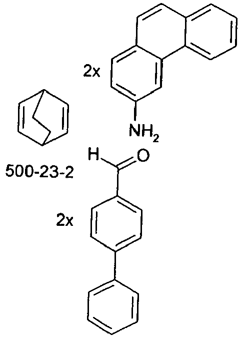 | 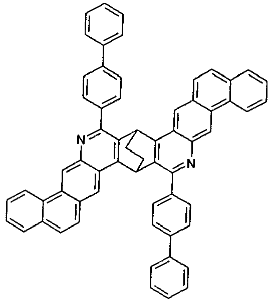 | 25% |
| F7 | 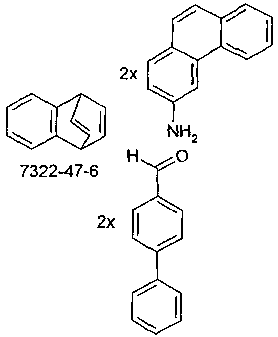 | 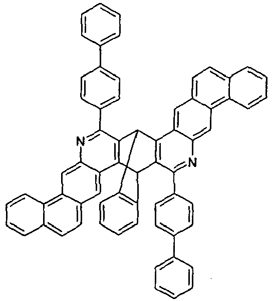 | 24% |

| **Typ G: Monoaldehyd + Monoamin + Triolefin** | | | |
|---|---|---|---|
| G1 | 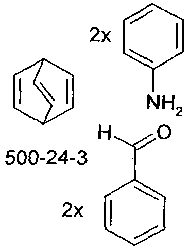 | 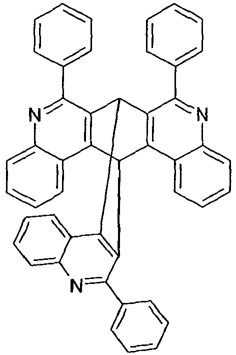 | 13% |

Die Verb. vom Typ A33 ff, B, D, D, E, F und G können bevorzung als e-TMM, HBM, ETM, SMB und SEB Verwendung finden.

### Beispiel A53

### Funktionalisierung der Materialen durch Suzuki-Kupplung

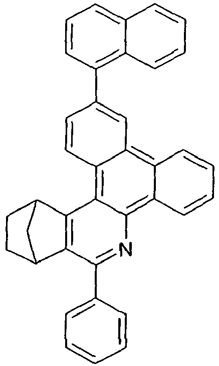

Ein Gemisch aus 22.5 g (50 mmol) A49, 12.9 g (75 mmol) 1-Napthylboronsäure, 31.8 g (150 mmol) Trikaliumphosphat, 224.5 mg (1 mmol) Palladium(II)-acetat, 1.8 g (6 mmol) Tri-o-tolylphosphin, 200 ml Toluol, 50 ml Dioxan und 250 ml Wasser wird unter gutem Rühren 20 h unter Rückfluss erhitzt. Nach Erkalten trennt man die wässrige Phase ab, wäscht diese zweimal mit je 100 ml Wasser und einmal mit 100 ml ges. Kochsalzlösung und filtriert dann über ein Celite-Bett ab, um Palladium zu entfernen. Nach Einengen wird der Rückstand fünfmal aus DMF umkristallisiert und dann zweimal im Hochvakuum (p ca. 10⁻⁵ mbar, T: 280 - 300°C) fraktioniert sublimiert. Ausbeute: 11.2 g (22.5 mmol), 45% d. Th.. Reinheit: 99.9% n. HPLC.

Analog können folgende Verbindungen dargestellt werden:

| **Bsp.** | **Bromid Boronsäure** | **Produkt** | **Ausbeute** |
|---|---|---|---|
| A54 | 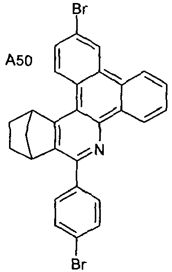 | 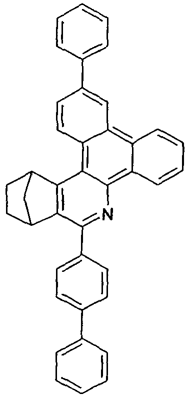 | 64% |
| | 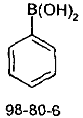 | | |
| A55 | 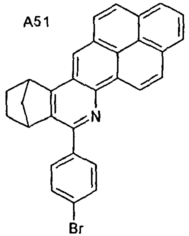 | 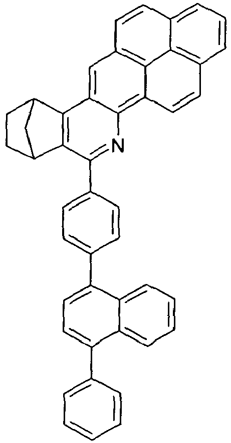 | 74% |
| | 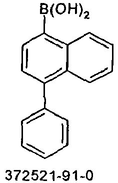 | | |
| A56 | 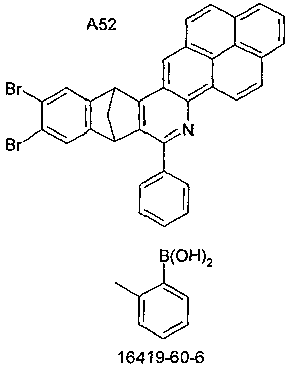 | 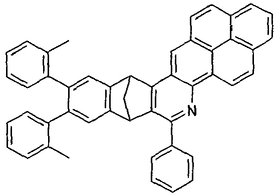 | 70% |

### Beispiel A57

### Funktionalisierung der Materialen durch Buchwald-Kupplung

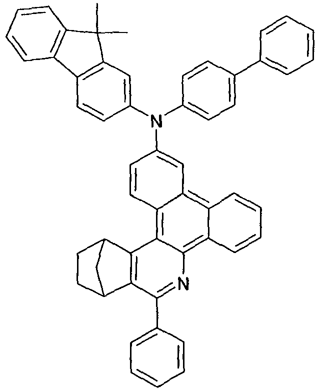

Ein Gemisch aus 22.5 g (50 mmol) A49, 22.6 g (60 mmol) N-[1,1'-biphenyl]-4-yl-9,9-dimethyl-9H-fluoren [897671-69-1] , 7.2 g (75 mmol) Natrium-tert-butylat, 224.5 mg (1 mmol) Palladium(II)acetat, 263 mg (1.3 mmol) Tri-tert-butylphopsphin und 300 ml Toluol wird unter gutem Rühren 20 h unter Rückfluss erhitzt. Nach Erkalten wäscht man die Reaktionsmischung zweimal mit je 100 ml Wasser und einmal mit 100 ml ges. Kochsalzlösung und filtriert dann über ein Celite-Bett ab, um Palladium zu entfernen. Nach Einengen wird der Rückstand fünfmal aus DMF umkristallisiert und dann zweimal im Hochvakuum (p ca. 10-5 mbar, T: 300 - 310 °C) fraktioniert sublimiert. Ausbeute: 14.3 g (19.5 mmol), 39 % d. Th.. Reinheit: 99.9 % n. HPLC.

### Analog können folgende Verbindungen dargestellt werden:

| **Bsp.** | **Bromid Amin** / **Carbazol** | **Produkt** | **Ausbeute** |
|---|---|---|---|
| A58 | 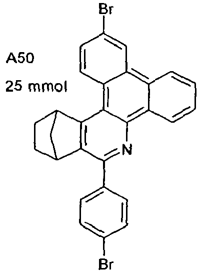 | 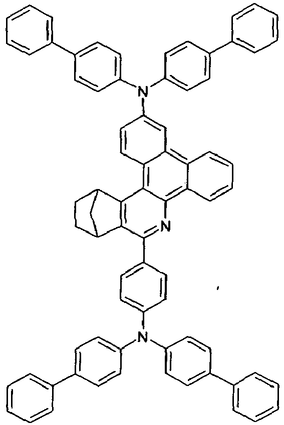 66% | |
| | 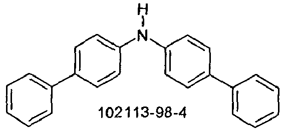 | | |
| A59 | 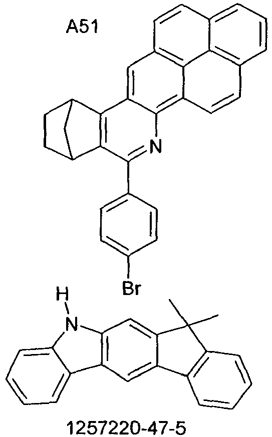 | 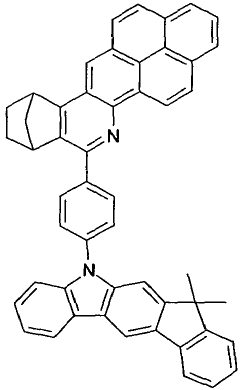 | 54% |

Verbindung A1-A27 können bevorzugt als zweizähnige Chelatliganden für Übergangsmetalle, wie z.B. Iridium und Platin, und als elektronenleitendes Triplexmatrixmaterial (eTMM)- bzw. Elektronentransportmaterial (ETM) Verwendung finden.

Die Verb. vom Typ A28 ff, B, D, D, E, F und G können bevorzung als elektronenleitendes Triplexmatrixmaterial (e-TMM), Lochblockiermaterial (HBM), Elektronentransportmaterial (ETM), blaues Singulettmaterial (SMB) und blauen Singulettemitter (SEB) Verwendung finden

### Beispiel

### Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (50 nm, Indium-Zinn-Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 3 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M3:M2:Ir(L1)₃ (55%:35%:10%) bedeutet hierbei, dass das Material M3 in einem Volumenanteil von 55%, M2 in einem Anteil von 35% und Ir(L1)₃ in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 4 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m² in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m² auf 500 cd/m². Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

### Verwendung von erfindungsgemäßen Verbindungen in OLEDs

Die erfindungsgemäßen Verbindungen lassen sich unter anderem als HTM, TMM, ETM, HBM, SMB und SEB in OLEDs einsetzen.

**Tabelle 1: Aufbau der OLED**

| **Bsp.** | **HTL2 Dicke** | **HTL-003 Dicke** | **EML Dicke** | **HBL Dicke** | **ETL Dicke** |
|---|---|---|---|---|---|
| **Verwendung als HTM** | | | | | |
| D-Vac 1 | HTM 220 nm | A57 10 nm | M1 :M2:Ir-G (65%:30%:5%) 25 nm | --- | ETM1:ETM2 (50%:50%) 20 nm |
| D-Vac 2 | HTM 220 nm | A58 10 nm | M1:M2:Ir-G (65%:30%:5%) 25 nm | --- | ETM1:ETM2 (50%:50%) 20 nm |
| D-Vac 3 | HTM 220 nm | A57 10 nm | M1:M2:Ir-G (65%:30%:5%) 25 nm | A30 5 nm | ETM1:ETM2 (50%:50%) 20 nm |
| D-Vac 4 | HTM 220 nm | A57 10 nm | M1 :M2:Ir-G (65%:30%:5%) 25 nm | HBM-Ref 5 nm | ETM1:ETM2 (50%:50%) 20 nm |

| **Verwendung als TMM** | | | | | |
|---|---|---|---|---|---|
| D-Vac 5 | HTM 220 nm | --- | A32:M2:Ir-G (65%:30%:5%) 25 nm | M1 10 nm | ETM1:ETM2 (50%:50%) 20 nm |
| D-Vac 5-Ref | HTM 220 nm | --- | TMM-Ref:M2:Ir-G (65%:30%:5%) 25 nm | M1 10 nm | ETM1:ETM2 (50%:50%) 20 nm |
| D-Vac 6 | HTM 220 nm | --- | A37:M2:Ir-R (65%:30%:5%) 30 nm | M1 10 nm | ETM1:ETM2 (50%:50%) 20 nm |
| D-Vac 7 | HTM 220 nm | --- | B3:M2:Ir-R (60%:30%:10%) 30 nm | M1 10 nm | ETM1:ETM2 (50%:50%) 20 nm |
| D-Vac 8 | HTM 220 nm | --- | B3:M2:Ir-R (60%:35%:5%) 30 nm | D3 10 nm | ETM1:ETM2 (50%:50%) 20 nm |
| D-Vac 9 | HTM 220 nm | --- | M1:A59:Ir-R (60%:35%:5%) 30 nm | D3 10 nm | ETM1:ETM2 (50%:50%) 20 nm |
| D-Vac 10 | HTM 220 nm | --- | F4:A59:Ir-R (60%:35%:5%) 30 nm | D3 10 nm | ETM1:ETM2 (50%:50%) 20 nm |

| **Verwendung als ETM** | | | | | |
|---|---|---|---|---|---|
| D-Vac 11 | HTM 220 nm | --- | M1:M2:Ir-G (65%:30%:5%) 25 nm | M1 10 nm | E1:ETM2 (50%:50%) 20 nm |
| D-Vac 12 | HTM 220 nm | --- | D5:M2:Ir-R (45%:50%:5%) 25 nm | M1 10 nm | E1:ETM2 (50%:50%) 20 nm |

| **Verwendung als SEB / SMB** | | | | | |
|---|---|---|---|---|---|
| D-Vac 13 | HTM 190 nm | --- | D4:SEB (95%:5%) 20 nm | --- | ETM1:ETM2 (50%:50%) 30 nm |
| D-Vac 13-Ref | HTM 190 nm | --- | SMB-Ref:SEB (95%:5%) 20 nm | --- | ETM1:ETM2 (50%:50%) 30 nm |
| D-Vac 14 | HTM 190 nm | --- | A39:SEB (95%:5%) 20 nm | --- | ETM1:ETM2 (50%:50%) 30 nm |
| D-Vac 15 | HTM 190 nm | --- | A42:SEB (95%:5%) 20 nm | --- | ETM1:ETM2 (50%:50%) 30 nm |
| D-Vac 16 | HTM 190 nm | --- | SMB:A44 (95%:5%) 20 nm | --- | ETM1:ETM2 (50%:50%) 30 nm |

**Tabelle 2: Ergebnisse der Vakuum-prozessierten OLEDs**

| **Bsp.** | **EQE (%) 1000 cd/m²** | **Spannung (V) 1000 cd/m²** | **CIE x/y 1000 cd/m²** | **LD50 (h) 1000 cd/m²** |
|---|---|---|---|---|
| **Verwendung als HTM** | | | | |
| D-Vac 1 | 18.3 | 3.5 | 0.34/0.63 | 55000 |
| D-Vac 2 | 19.4 | 3.5 | 0.34/0.64 | --- |
| D-Vac 3 | 17.6 | 3.6 | 0.35/0.64 | 70000 |
| D-Vac 4 | 18.1 | 3.7 | 0.35/0.64 | 50000 |

| **Verwendung als TMM** | | | | |
|---|---|---|---|---|
| D-Vac 5 | 18.8 | 3.6 | 0.35/0.64 | 70000 |
| D-Vac 5-Ref | 18.5 | 3.6 | 0.35/0.65 | 30000 |
| D-Vac 6 | 16.9 | 3.3 | 0.67/0.33 | 85000 |
| D-Vac 7 | 16.5 | 3.2 | 0.67/0.33 | --- |
| D-Vac 8 | 16.7 | 3.1 | 0.67/0.33 | --- |
| D-Vac 9 | 16.7 | 3.2 | 0.66/0.34 | --- |
| D-Vac 10 | 16.3 | 3.3 | 0.67/0.33 | --- |

| **Verwendung als ETM** | | | | |
|---|---|---|---|---|
| D-Vac 11 | 17.0 | 3.3 | 0.35/0.64 | --- |
| D-Vac 12 | 16.6 | 3.2 | 0.67/0.33 | --- |

| **Verendung als SEB / SMB** | | | | |
|---|---|---|---|---|
| D-Vac 13 | 7.2 | 3.9 | 0.15/0.17 | 8000 |
| D-Vac 13-Ref | 7.0 | 4.2 | 0.15/0.17 | 6000 |
| D-Vac 14 | 7.0 | 4.0 | 0.15/0.17 | --- |
| D-Vac 15 | 7.4 | 4.2 | 0.15/0.17 | --- |
| D-Vac 16 | 5.5 | 3.9 | 0.16/0.24 | --- |

### 2) Lösungs-prozessierte Devices:

### A: Aus löslichen Funktionsmaterialien

Die erfindungsgemäßen Iridium-Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die lösungsprozessierten Devices enthalten eine Emissionsschicht aus (Polystyrol):Matrix1:Matrix2:Ir_G-Sol (25%:25%:40%:10%). Die Emissionsschicht wird in einer Inertgasatmosphäre, im vorliegenden Fall Argon, aufgeschleudert und 30 min bei 130 °C ausgeheizt. Zuletzt wird eine Kathode aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z.B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Matrix1** | **EQE (%) 1000 cd/m²** | **Spannung (V) 1000 cd/m²** | **CIE x/y 1000 cd/m²** |
|---|---|---|---|---|
| | **Matrix2** | | | |
| **Grüne OLEDs** | | | | |
| D-Sol1 | A57 | 18.3 | 5.4 | 0.35/0.63 |
| | M1 | | | |
| D-Sol2 | M2 | 18.6 | 5.7 | 0.34/0.64 |
| | C1 | | | |

**Tabelle 4: Strukturformeln der verwendeten Materialien**

| | |
|---|---|
| 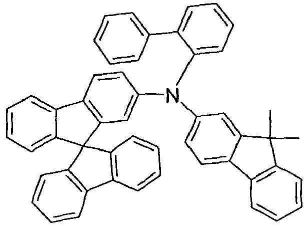 | 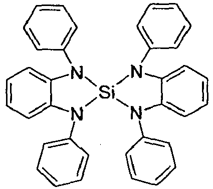 |
| HTM | EBM = M10 |
| 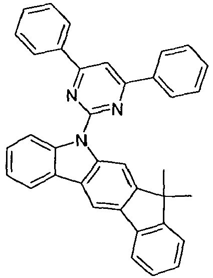 | 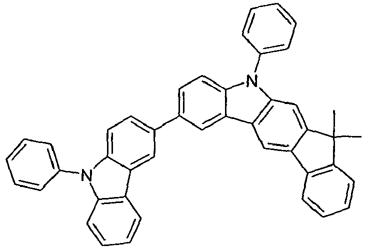 |
| M1 = HBM | M2 |
| 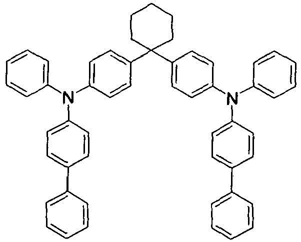 | 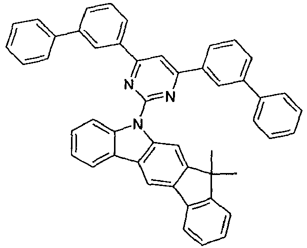 |
| M3 | M4 |
| 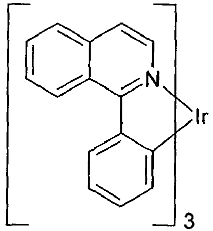 | 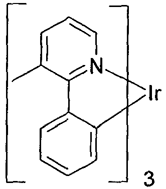 |
| Ir-R | Ir-G |
| 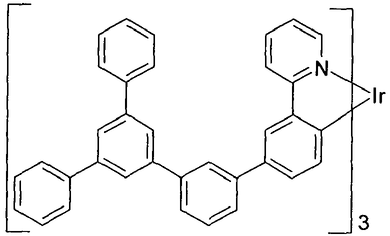 | |
| Ir-G-Sol | |
| 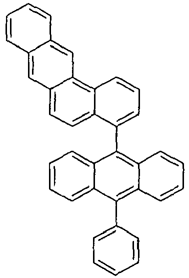 | 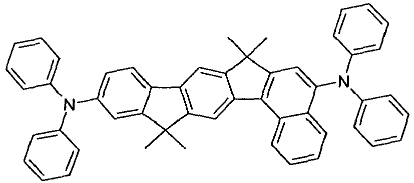 |
| SBM | SEB |
| 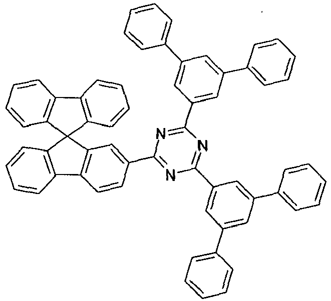 | 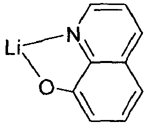 |
| ETM1 | ETM2 |
| 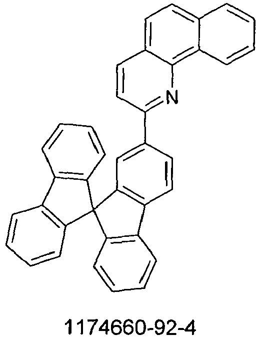 | 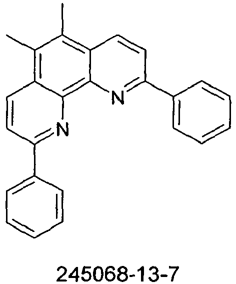 |
| HBM-Ref | TMM-Ref |
| | |
| 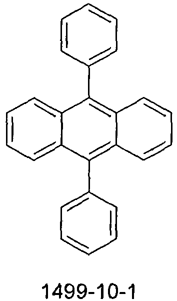 | |
| SMB-Ref | |

## Patentansprüche

1. Verbindung, umfassend mindestens eine Struktur der Formeln (I) und/oder (II) 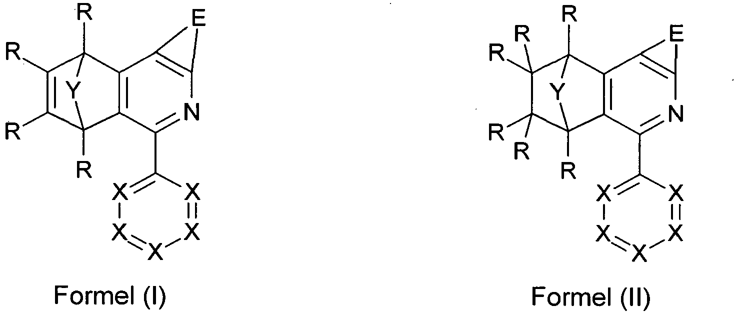 wobei für die verwendeten Symbole gilt:
X ist bei jedem Auftreten gleich oder verschieden CR oder N;
E ist eine bivalente Brücke, wobei die Gruppe E zusammen mit den hiermit verbundenen C-Atomen einen fünf- oder sechsgliedrigen Ring bildet;
Y ist eine bivalente Brücke, ausgewählt aus O, S, C(R)₂, C(R)=C(R), N(R), B(R), Si(R)₂, C=O, C=NR, C=C(R)₂, S=O, SO₂, C(R)₂-C(R)₂, P(R) und P(=O)R;
R ist bei jedem Auftreten gleich oder verschieden H, D, F, Cl, I, N(R¹)₂, CN, OH, COOH, C(=O)N(R¹)₂, Si(R¹)₃, B(OR¹)₂, C(=O)R¹, P(=O)(R¹)₂, S(=O)R₁, S(=O)₂R¹, OSO₂R¹, eine gerad-kettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ring-atomen, das jeweils durch einen oder mehrere Reste R¹ substituiert sein kann, oder eine Aryloxy- oder Heteroaryloxy-gruppe mit 5 bis 40 aromatischen Ringatomen, die durch einen oder mehrere Reste R¹ substituiert sein kann, oder eine Aralkyl-oder Heteroaralkylgruppe mit 5 bis 40 aromatischen Ringatomen, die durch einen oder mehrere Reste R¹ substituiert sein kann, oder eine Diarylaminogruppe, Diheteroaryl-aminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ring-system bilden;
R¹ ist bei jedem Auftreten gleich oder verschieden H, D, F, Cl, Br, I, N(R²)₂, CN, NO₂, Si(R²)₃, B(OR²)₂, C(=O)R₂, P(=O)(R²)₂, S(=O)R², S(=O)₂R², OSO₂R², eine geradkettige Alkyl-, Alkoxy-oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere benachbarte Reste R¹ miteinander oder R¹ mit R ein mono- oder polycyclisches, aliphatisches, aromatisches oder heteroaromatisches Ringsystem bilden;
R² ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R² auch miteinander ein mono- oder polycyclisches, aliphatisches Ringsystem bilden.

2. Verbindung gemäß Anspruch 1, **dadurch gekennzeichnet, dass** E ausgewählt ist aus Gruppen der Formel X=X-X=X, X-W-X, W-X=X, X=X-W, worin
W ausgewählt ist aus O, S, C(R)₂, N(R), B(R), Si(R)₂, C=O, S=O, SO₂, P(R) und P(=O)R, wobei R bei jedem Auftreten gleich oder verschieden ist und die in Anspruch 1 genannte Bedeutung hat; und
X bei jedem Auftreten gleich oder verschieden ist und die in Anspruch 1 genannte Bedeutung hat.

3. Verbindung gemäß Anspruch 1 oder 2, **dadurch gekennzeichnet, dass** die Verbindung Strukturen der Formeln (la) und/oder (IIa) 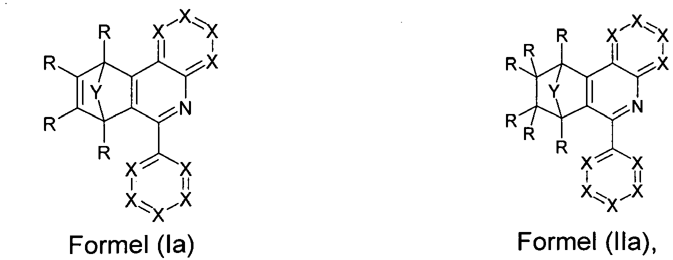 umfasst, wobei die verwendeten Symbole die in Anspruch 1 genannten Bedeutungen haben.

4. Verbindung gemäß einem oder mehreren der Ansprüche 1 bis 3, **dadurch gekennzeichnet, dass** die Verbindung Strukturen der Formeln (Ia1), (Ia2), (IIa1) und/oder (IIa2) 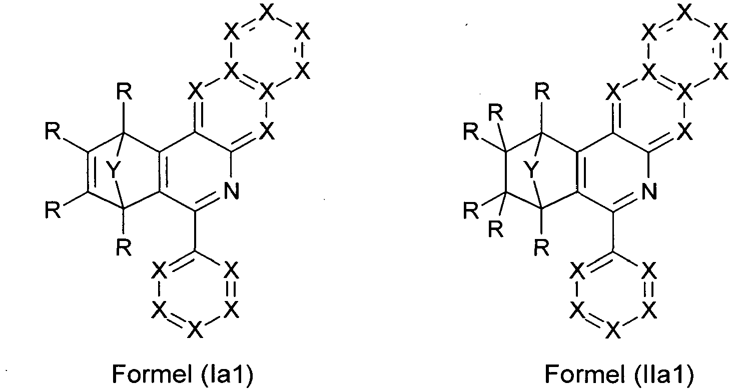 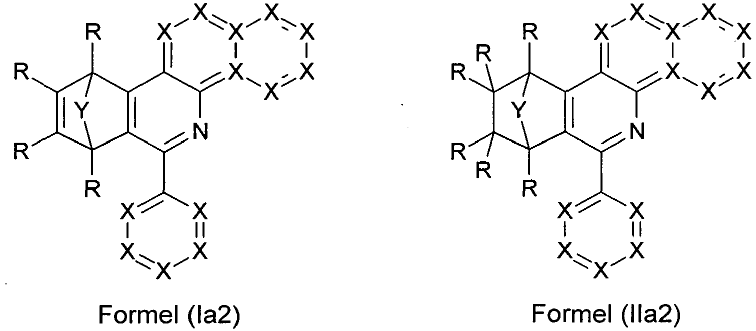 umfasst, wobei die verwendeten Symbole die in Anspruch 1 genannten Bedeutungen haben.

5. Verbindung gemäß einem oder mehreren der Ansprüche 1 bis 4, **dadurch gekennzeichnet, dass** die Verbindung Strukturen der Formeln (Ia3) und/oder (Ia4) 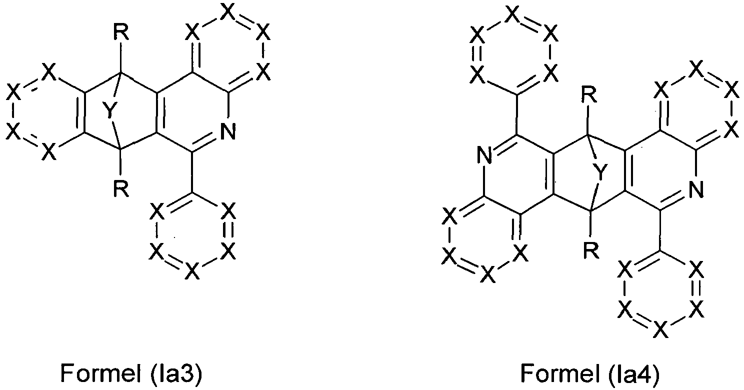 umfasst, wobei die verwendeten Symbole die in Anspruch 1 genannten Bedeutungen haben.

6. Verbindung gemäß Anspruch 1 oder 2, **dadurch gekennzeichnet, dass** die Verbindung Strukturen der Formeln (Ib) und/oder (IIb) 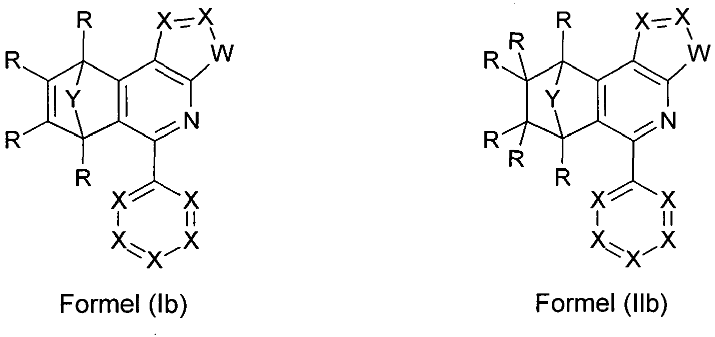 umfasst, wobei die verwendeten Symbole die in den Ansprüchen 1 und 2 genannten Bedeutungen haben.

7. Verbindung gemäß Anspruch 1 oder 2, die Verbindung Strukturen der Formel (Ic) 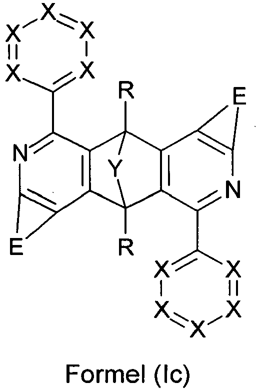 umfasst, wobei die verwendeten Symbole die in Anspruch 1 genannten Bedeutungen haben und E bei jedem Auftreten gleich oder verschieden sein kann.

8. Verbindungen gemäß einem oder mehreren der Ansprüche 1 bis 7, **dadurch gekennzeichnet, dass** die Verbindungen mindestens zwei bicyclische Gruppen aufweisen, die durch ein Strukturelement umfassend eine Gruppe Y gebildet werden, wobei Y bei jedem Auftreten gleich oder verschieden sein kann und die in Anspruch 1 genannte Bedeutung aufweist.

9. Verbindung gemäß einem oder mehreren der Ansprüche 1 bis 8, **dadurch gekennzeichnet, dass** die Verbindung Strukturen der Formel CyE-(CyF)ₙ aufweist, wobei für die Symbole und Indizes gilt: n ist 2 oder 3 CyE ist ein Strukturelement ausgewählt aus den Formeln 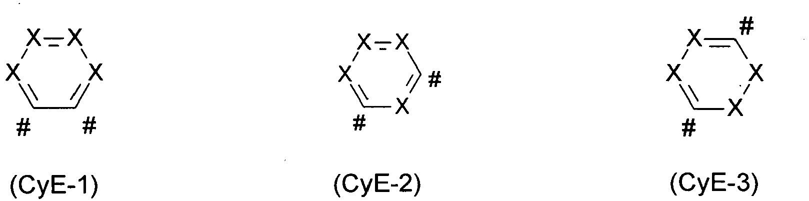 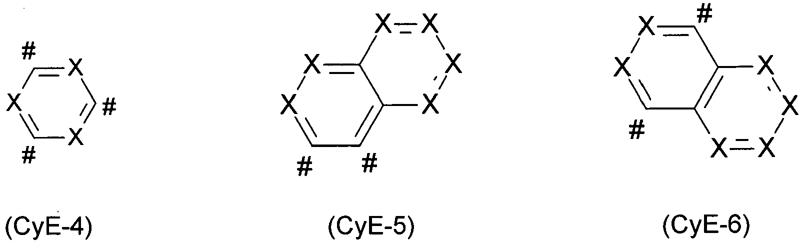 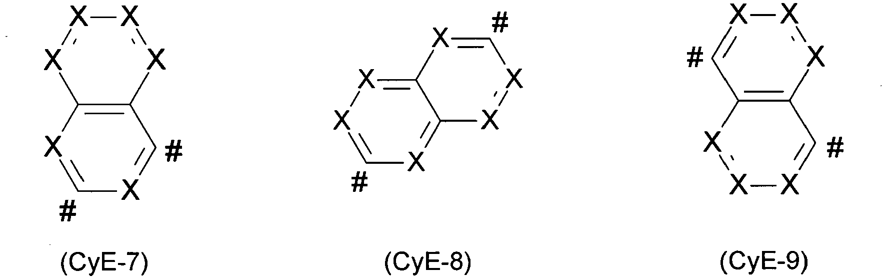 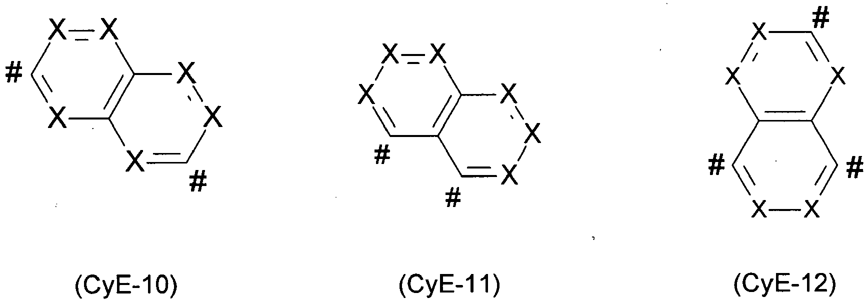 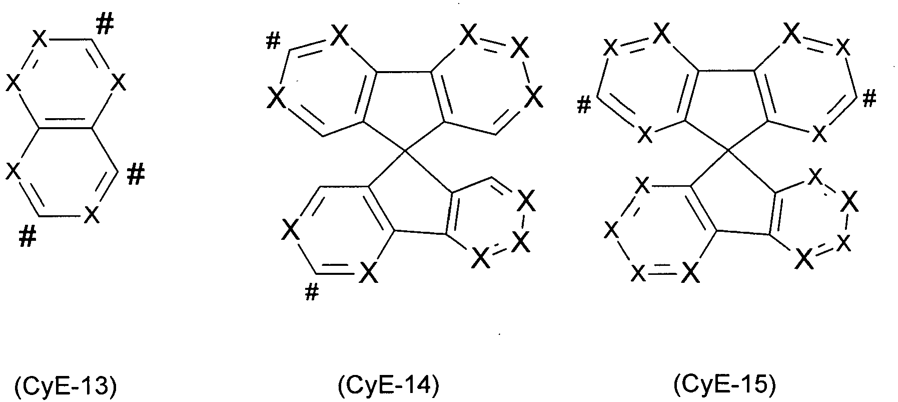 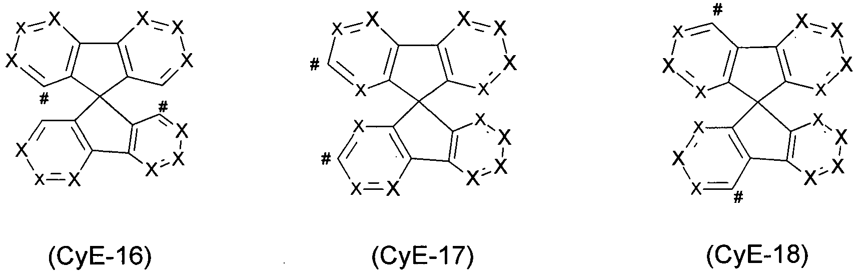 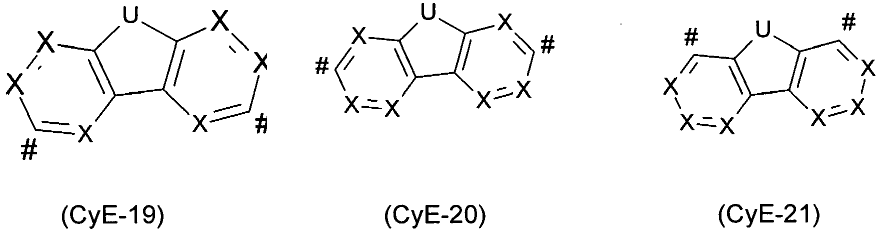 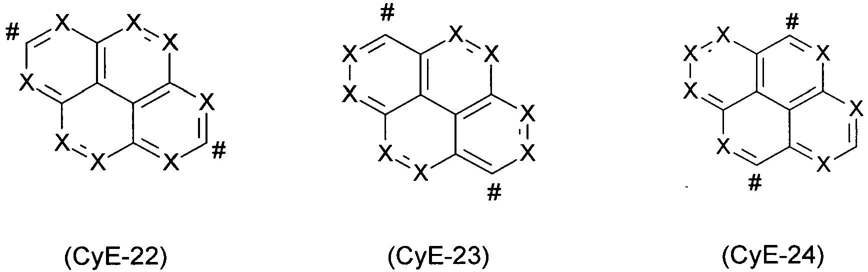 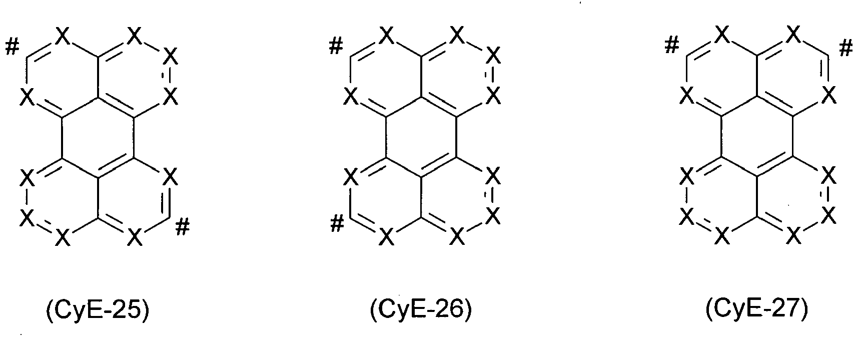 und CyF ist mindestens ein Strukturelement ausgewählt aus den Formeln 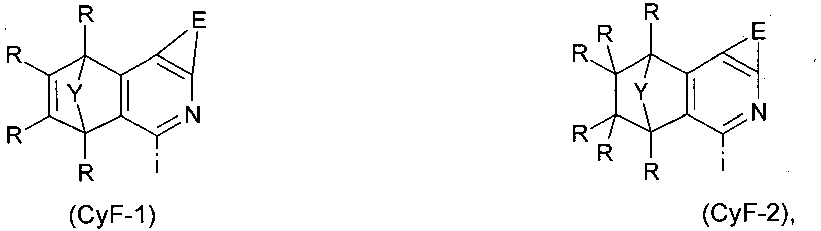 , wobei die verwendeten Symbole E, R, Y und X die in Anspruch 1 genannten Bedeutungen haben, U ausgewählt ist aus O, S, C(R)₂, N(R), B(R), Si(R)₂, C=O, S=O, SO₂, P(R) und P(=O)R, die gestrichelte Linie in den Formeln CyF-1 und CyF-2 die Bindung an die Gruppe CyE kennzeichnen und Gruppe CyF jeweils an der durch # gekennzeichneten Position an CyE bindet.

10. Verbindung gemäß Anspruch 9, **dadurch gekennzeichnet, dass** CyF mindestens ein Strukturelement ausgewählt aus den Formeln 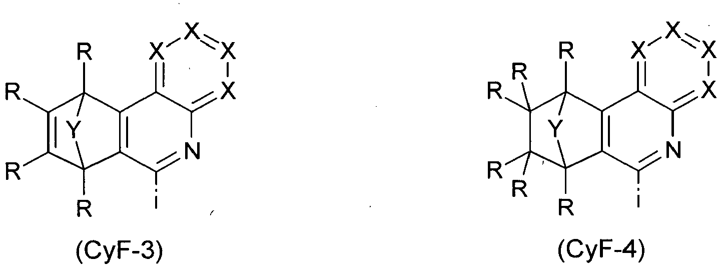 ist, wobei die verwendeten Symbole R, Y und X die in Anspruch 1 genannten Bedeutungen haben, die gestrichelte Linie in den Formeln CyF-3 und CyF-4 die Bindung an CyE kennzeichnen und CyF jeweils an der durch # gekennzeichneten Position an CyE gebunden ist.

11. Verbindung gemäß einem oder meheren der Ansprüche 1 bis 10, **dadurch gekennzeichnet, dass** die Verbindungen Strukturen der Formel CyG(CyH)ₙ aufweisen, wobei CyG und CyH zusammen jeweils einen Ring aufspannen und für die Symbole und Indizes gilt: n ist 2 oder 3
CyG ist ein Strukturelement ausgewählt aus den Formeln 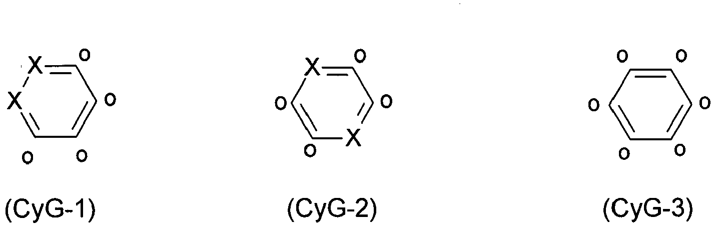 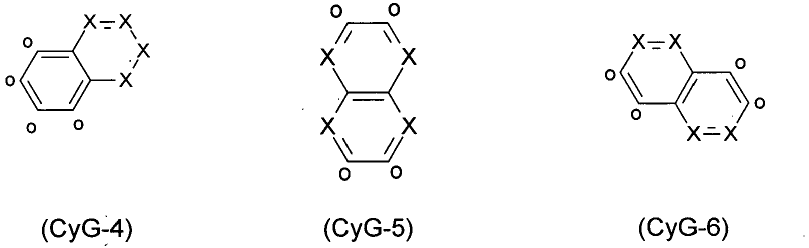 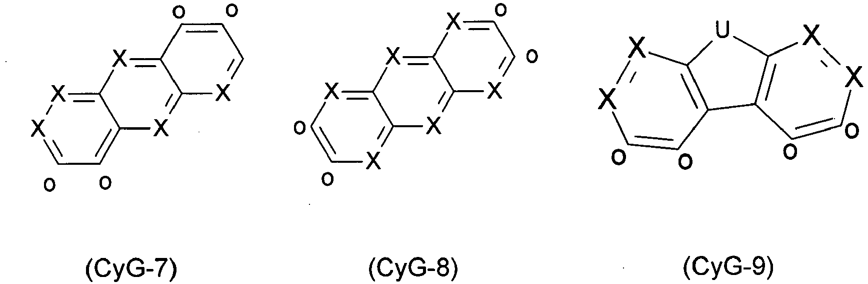 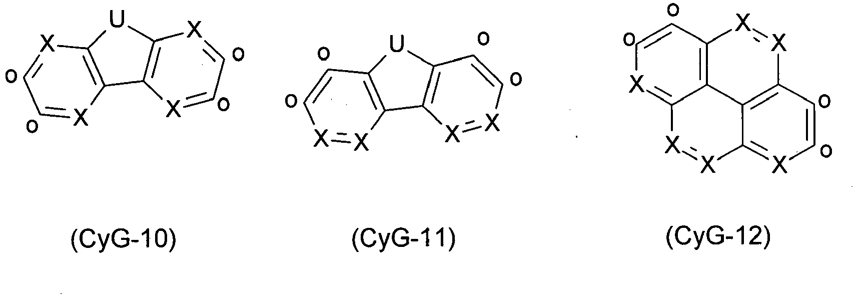 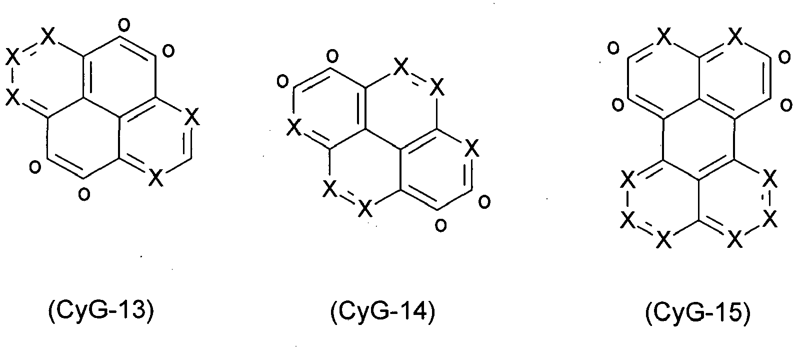 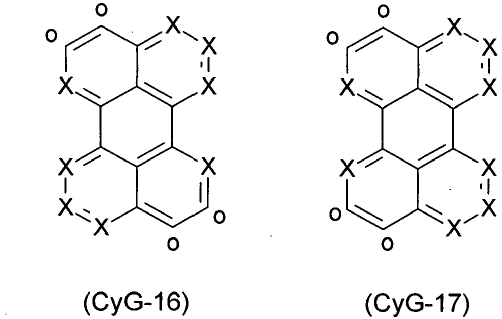 und CyH ist mindestens ein Strukturelement ausgewählt aus den Formeln 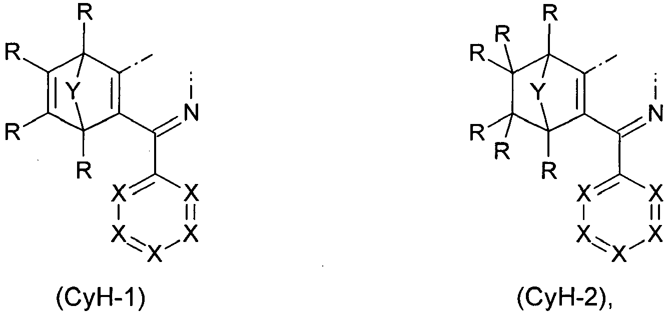 wobei die verwendeten Symbole R, Y und X die in Anspruch 1 genannten Bedeutungen haben, U ausgewählt ist aus O, S, C(R)₂, N(R), B(R), Si(R)₂, C=O, S=O, SO₂, P(R) und P(=O)R, die gestrichelte Linie in den Formeln CyH-1 und CyH-2 die Bindung an CyG kennzeichnen und CyH jeweils an den durch o gekennzeichneten Positionen an CyG unter Bildung eines Ringes binden.

12. Verbindung gemäß einem oder mehreren der Ansprüche 1 bis 11, **dadurch gekennzeichnet, dass** in den Strukturen gemäß Formeln (I), (II), (la), (IIa), (Ia1), (IIa1), (Ia2), (IIa2), (Ia3), (Ia4), (Ib), (IIb) und (Ic) sowie den Strukturen gemäß Formel CyE-(CyF)ₙ und CyG(CyH)ₙ die Symbole X so gewählt werden, dass das Verhältnis von CR zu N größer oder gleich 3 ist.

13. Verbindung gemäß einem oder mehreren der Ansprüche 1 bis 12, **dadurch gekennzeichnet, dass** die Verbindung mindestens ein Metallatom ausgewählt aus Iridium oder Platin aufweist.

14. Oligomer, Polymer oder Dendrimer enthaltend eine oder mehrere Verbindungen umfassend mindestens eine Struktur der Formeln (I) und/oder (II) 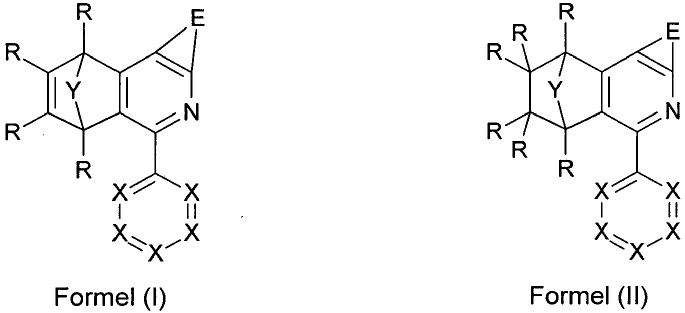 wobei für die verwendeten Symbole gilt:
R ist bei jedem Auftreten gleich oder verschieden H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ring-atomen, das jeweils durch einen oder mehrere Reste R¹ substituiert sein kann, oder eine Aryloxy- oder Heteroaryloxy-gruppe mit 5 bis 40 aromatischen Ringatomen, die durch einen oder mehrere Reste R¹ substituiert sein kann, oder eine Aralkyl-oder Heteroaralkylgruppe mit 5 bis 40 aromatischen Ringatomen, die durch einen oder mehrere Reste R¹ substituiert sein kann, oder eine Diarylaminogruppe, Diheteroaryl-aminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ring-system bilden;
und wobei für die anderen verwendeten Symbole die Definitionen des Anspruchs 1 gelten und wobei ein oder mehrere Bindungen der Verbindung zum Polymer, Oligomer oder Dendrimer vorhanden sind.

15. Zusammensetzung enthaltend mindestens eine Verbindung umfassend mindestens eine Struktur der Formeln (I) und/oder (II) 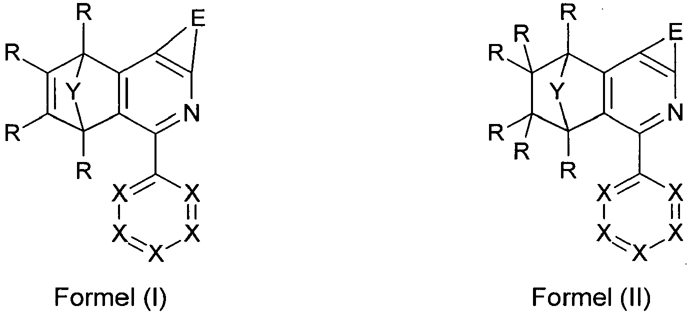 wobei für die verwendeten Symbole gilt:
R ist bei jedem Auftreten gleich oder verschieden H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyloder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden; und wobei für die anderen verwendeten Symbole die Definitionen des Anspruchs 1 gelten, und/oder ein Oligomer, Polymer oder Dendrimer gemäß Anspruch 14 und wenigstens ein weiteres organisch funktionelles Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

16. Formulierung enthaltend mindestens eine Verbindung umfassend mindestens eine Struktur der Formeln (I) und/oder (II) 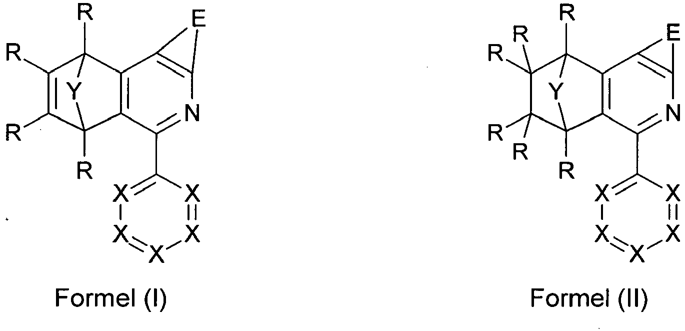 wobei für die verwendeten Symbole gilt:
R ist bei jedem Auftreten gleich oder verschieden H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyloder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
und wobei für die anderen verwendeten Symbole die Definitionen des Anspruchs 1 gelten, ein Oligomer, Polymer oder Dendrimer gemäß Anspruch 14 und/oder wenigstens eine Zusammensetzung gemäß Anspruch 15 und mindestens ein Lösungsmittel

17. Verfahren zur Herstellung einer Verbindung gemäß einem oder mehreren der Ansprüche 1 bis 13 oder eines Oligomers, Polymers oder Dendrimers gemäß Anspruch 14 durch Umsetzung mindestens eines Aldehyds mit mindestens einem aromatischen oder heteroaromatischen Amin und mindestens einem bicyclischen Olefin.

18. Verfahren gemäß Anspruch 17, **dadurch gekennzeichnet, dass** die nach der Umsetzung von mindesten einem Aldehyd mit mindestens einem aromatischen Amin und mindestens einem bicyclischen Olefin erhaltene Zwischenverbindung in einer Kupplungsreaktion umgesetzt wird.

19. Verwendung einer Verbindung umfassend mindestens eine Struktur der Formeln (I) und/oder (II) 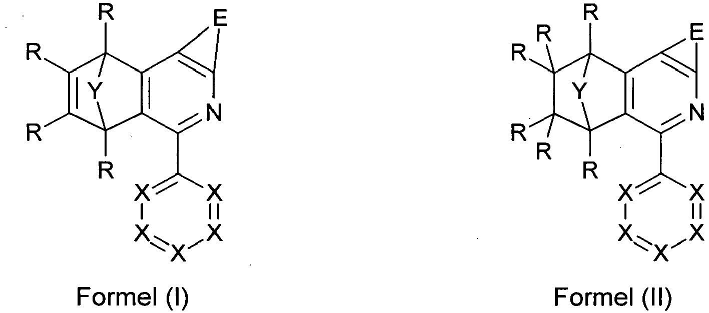 wobei für die verwendeten Symbole gilt:
R ist bei jedem Auftreten gleich oder verschieden H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 CAtomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyloder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
und wobei für die anderen verwendeten Symbole die Definitionen des Anspruchs 1 gelten oder eines Oligomers, Polymers oder Dendrimers gemäß Anspruch 14 oder wenigstens einer Zusammensetzung gemäß Anspruch 15 in einer elektronischen Vorrichtung als Lochinjektionsmaterial, Lochtransportmaterial, Lochblockiermaterial, Elektroneninjektionsmaterial, Elektronenblockiermaterial und/oder Emittermaterial.

20. Elektronische Vorrichtung, enthaltend mindestens eine Verbindung umfassend mindestens eine Struktur der Formeln (I) und/oder (II) 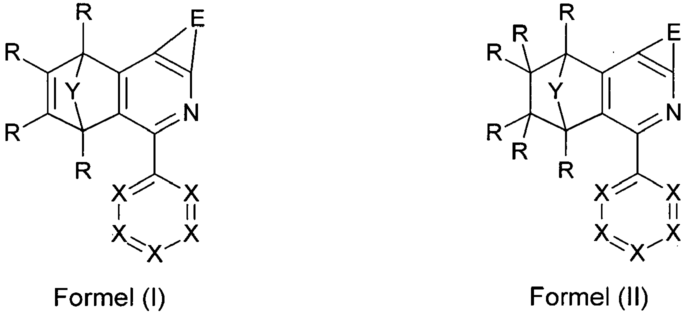 wobei für die verwendeten Symbole gilt:
R ist bei jedem Auftreten gleich oder verschieden H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, NR1, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann, oder eine Aralkyloder Heteroaralkylgruppe mit 5 bis 40 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei benachbarte Reste R auch miteinander ein mono- oder polycyclisches, aliphatisches, aromatisches oder heteroaromatisches Ringsystem bilden;
und wobei für die anderen verwendeten Symbole die Definitionen des Anspruchs 1 gelten, ein Oligomer, Polymer oder Dendrimer gemäß Anspruch 14 und/oder wenigstens eine Zusammensetzung gemäß Anspruch 15, wobei die elektronische Vorrichtung bevorzugt ausgewählt ist aus der Gruppe bestehend aus organischen Elektrolumineszenzvorrichtungen, wobei organische Laser-dioden, organischen lichtemittierenden Transistoren , organische lichtemittierende Dioden (OLED) und organische lichtemittierende elektrochemische Zellen (OLEC) bevorzugte organische Elektrolumineszenzvorrichtungen sind, OLEDs und OLECs ganz bevorzugte organische Elektrolumineszenzvorrichtungen sind und OLEDs ganz besonders bevorzugte organische Elektrolumineszenzvorrichtungen sind, organischen integrierten Schaltungen, organischen Feld-Effekt-Transistoren, organischen Dünnfilmtransistoren, organischen Solarzellen, organischen optischen Detektoren, organischen Photorezeptoren und organischen Feld-Quench-Devices.

## Claims

1. Compound including at least one structure of the formulae (I) and/or (II) 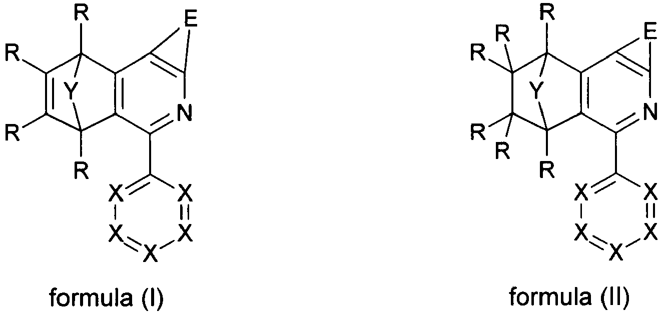 where the following applies to the symbols used:
X is on each occurrence, identically or differently, CR or N;
E is a divalent bridge, where the group E forms a five- or sixmembered ring together with the C atoms connected thereto;
Y is a divalent bridge selected from O, S, C(R)₂, C(R)=C(R), N(R), B(R), Si(R)₂, C=O, C=NR, C=C(R)₂, S=O, SO₂, C(R)₂-C(R)₂, P(R) and P(=O)R;
R is on each occurrence, identically or differently, H, D, F, Cl, I, N(R¹)₂, CN, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C=C, Si(R¹)₂, C=O, NR1, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
R¹ is on each occurrence, identically or differently, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R², where one or more non-adjacent CH₂ groups may be replaced by R²C=CR², C=C, Si(R²)₂, C=O, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 40 aromatic ring atoms, which may be substituted by one or more radicals R², or an aralkyl or heteroaralkyl group having 5 to 40 aromatic ring atoms, which may be substituted by one or more radicals R², or a diarylamino group, diheteroarylamino group or arylheteroarylamino group having 10 to 40 aromatic ring atoms, which may be substituted by one or more radicals R²; two or more adjacent radicals R¹ with one another or R¹ with R may form a mono- or polycyclic, aliphatic, aromatic or heteroaromatic ring system here;
R² is on each occurrence, identically or differently, H, D, F or an aliphatic, aromatic and/or heteroaromatic hydrocarbon radical having 1 to 20 C atoms, in which, in addition, one or more H atoms may be replaced by F; two or more substituents R² may also form a mono- or polycyclic, aliphatic ring system with one another here.

2. Compound according to Claim 1, **characterised in that** E is selected from the groups of the formulae X=X-X=X, X-W-X, W-X=X and X=X-W, in which
W is selected from O, S, C(R)₂, N(R), B(R), Si(R)₂, C=O, S=O, SO₂, P(R) and P(=O)R, where R is identical or different on each occurrence and has the meaning given in Claim 1; and
X is identical or different on each occurrence and has the meaning given in Claim 1.

3. Compound according to Claim 1 or 2, **characterised in that** the compound includes structures of the formulae (Ia) and/or (IIa) 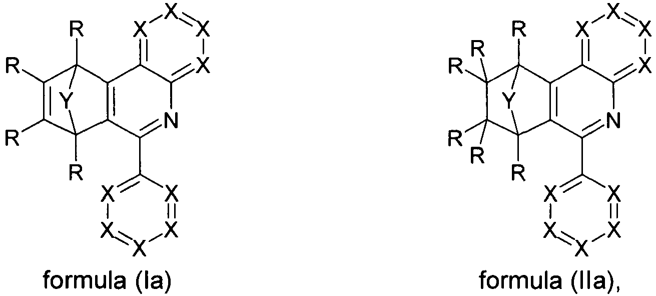 where the symbols used have the meanings given in Claim 1.

4. Compound according to one or more of Claims 1 bis 3, **characterised in that** the compound includes structures of the formulae (Ia1), (Ia2), (IIa1) and/or (IIa2) 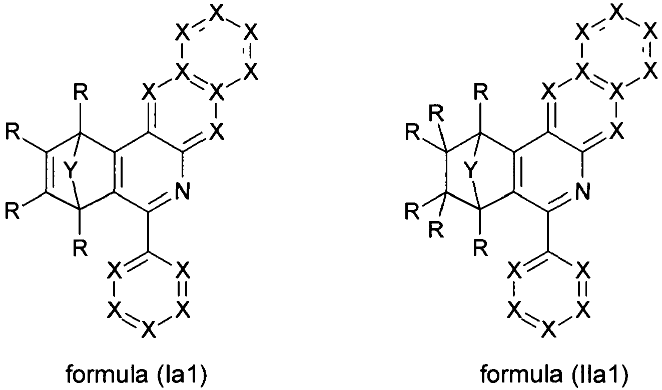 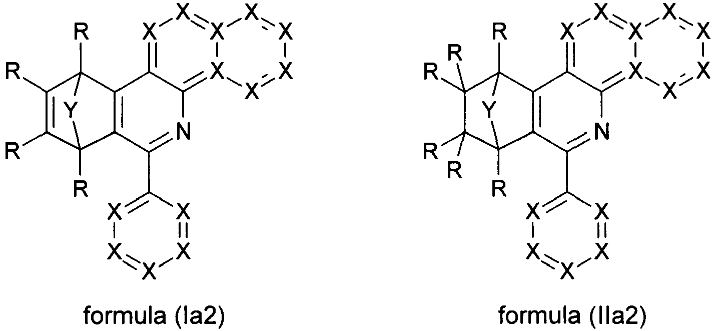 where the symbols used have the meanings given in Claim 1.

5. Compound according to one or more of Claims 1 to 4, **characterised in that** the compound includes structures of the formulae (Ia3) and/or (Ia4) 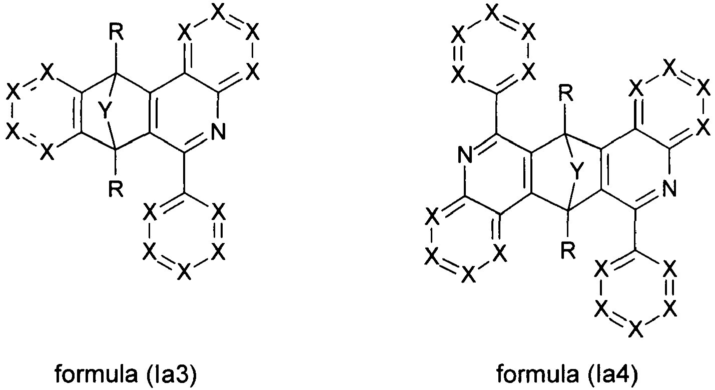 where the symbols used have the meanings given in Claim 1.

6. Compound according to Claim 1 or 2, **characterised in that** the compound includes structures of the formulae (Ib) and/or (IIb) 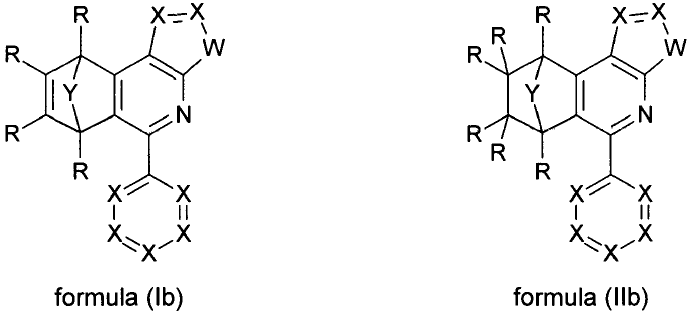 where the symbols used have the meanings given in Claims 1 and 2.

7. Compound according to Claim 1 or 2, **characterised in that** the compound includes structures of the formula (Ic) 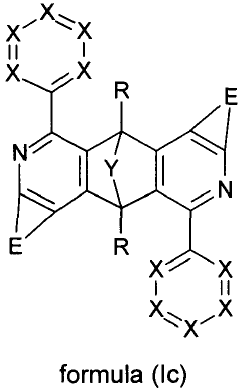 where the symbols used have the meanings given in Claim 1 and E may be identical or different on each occurrence.

8. Compounds according to one or more of Claims 1 to 7, **characterised in that** the compounds contain at least two bicyclic groups, which are formed by a structural element including a group Y, where Y may be identical or different on each occurrence and has the meaning given in Claim 1.

9. Compound according to one or more of Claims 1 to 8, **characterised in that** the compound contains structures of the formula CyE-(CyF)ₙ, where the following applies to the symbols and indices:
n is 2 or 3
CyE is a structural element selected from the formulae 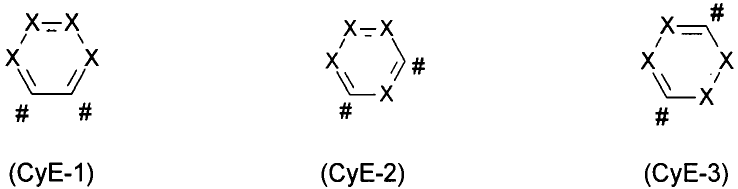 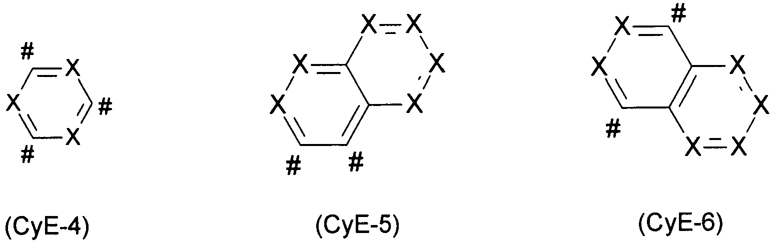 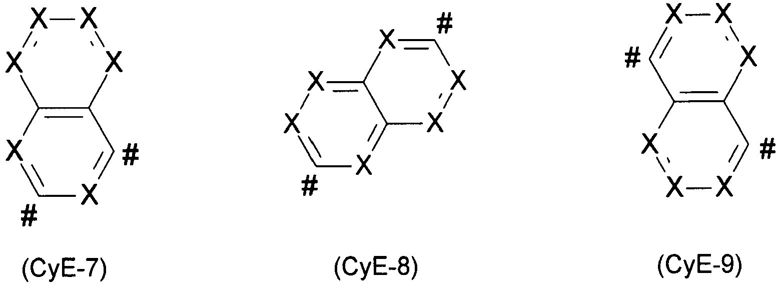 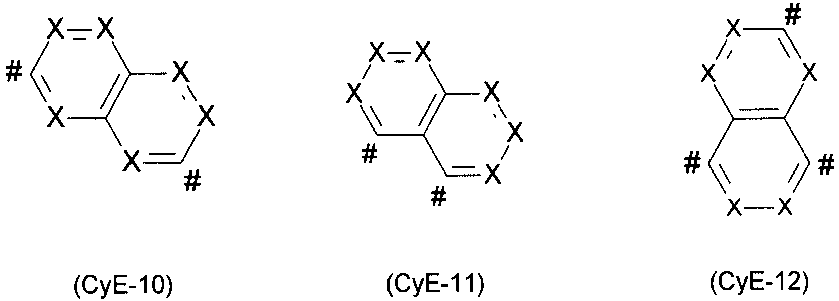 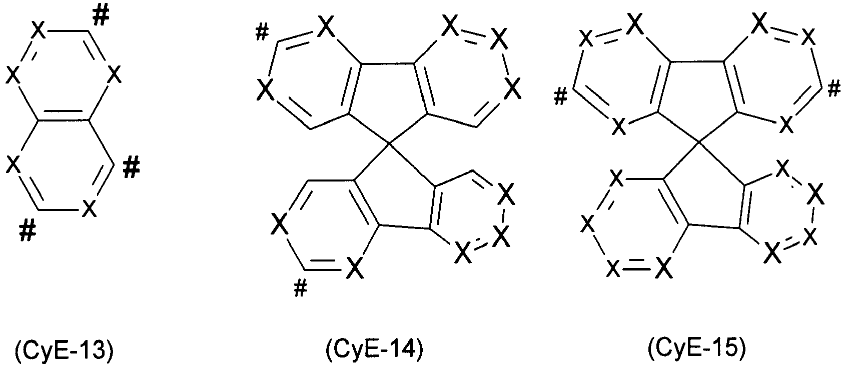 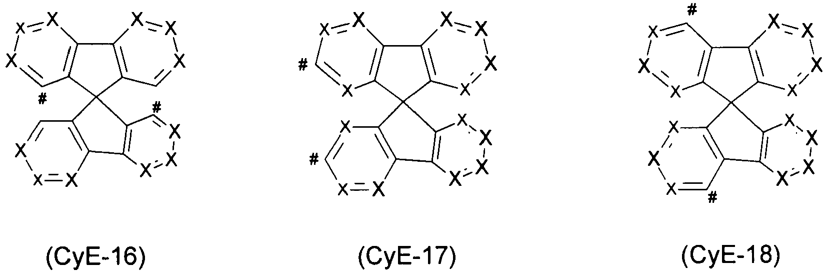 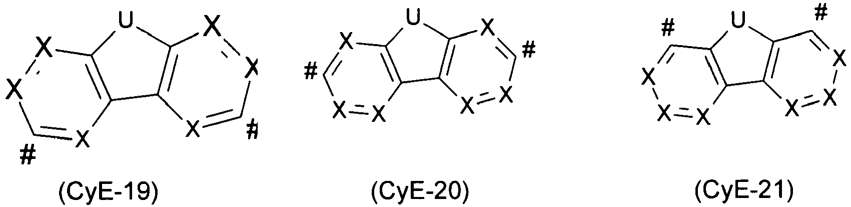 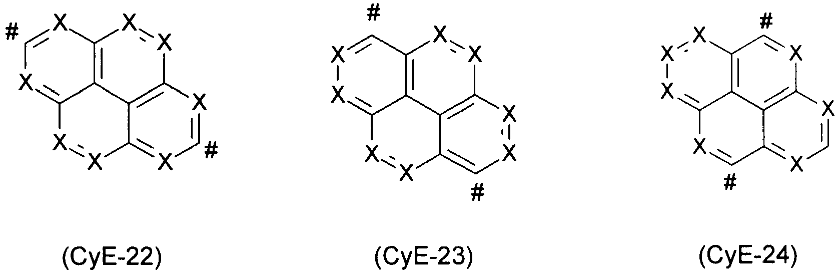 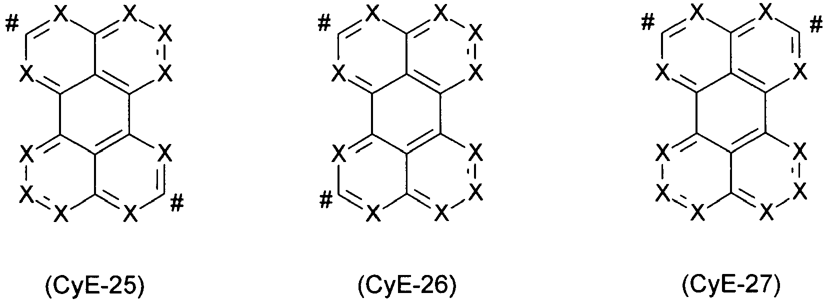 and CyF is at least one structural element selected from the formulae 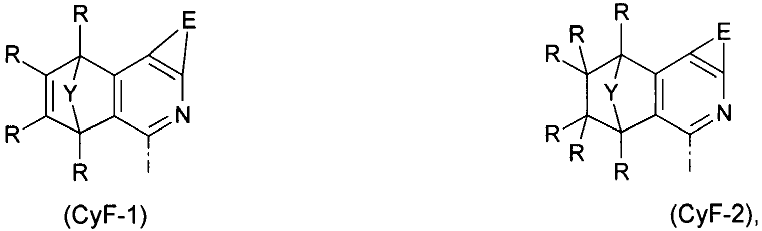 where the symbols E, R, Y and X used have the meanings given in Claim 1, U is selected from O, S, C(R)₂, N(R), B(R), Si(R)₂, C=O, S=O, SO₂, P(R) and P(=O)R, the dashed lines in the formulae CyF-1 and CyF-2 denote the bond to the group CyE and group CyF is in each case bonded to CyE at the position denoted by #.

10. Compound according to Claim 9, **characterised in that** CyF is at least one structural element selected from the formulae 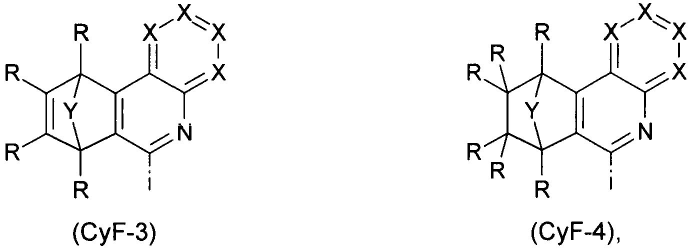 where the symbols R, Y and X used have the meanings given in Claim 1, the dashed lines in the formulae CyF-3 and CyF-4 denote the bond to CyE and CyF is in each case bonded to CyE at the position denoted by #.

11. Compound according to one or more of Claims 1 to 10, **characterised in that** the compound contains structures of the formula CyG(CyH)ₙ, where CyG and CyH together in each case form a ring and the following applies to the symbols and indices:
n is 2 or 3
CyG is a structural element selected from the formulae 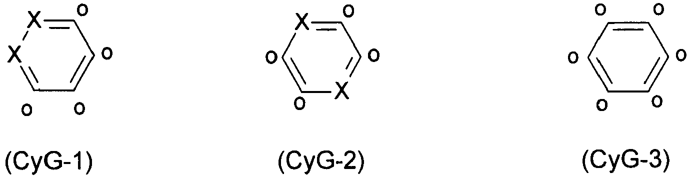 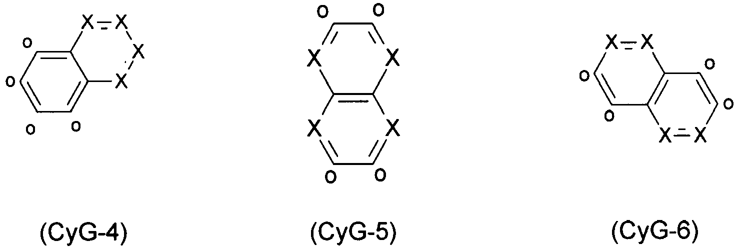 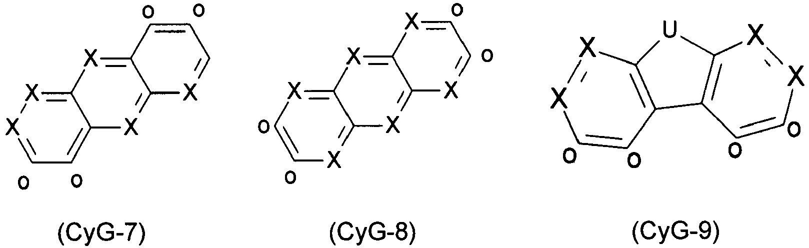 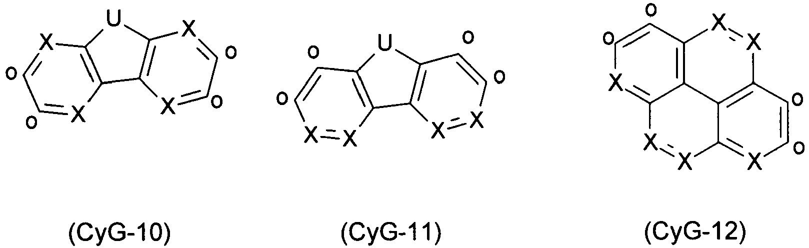 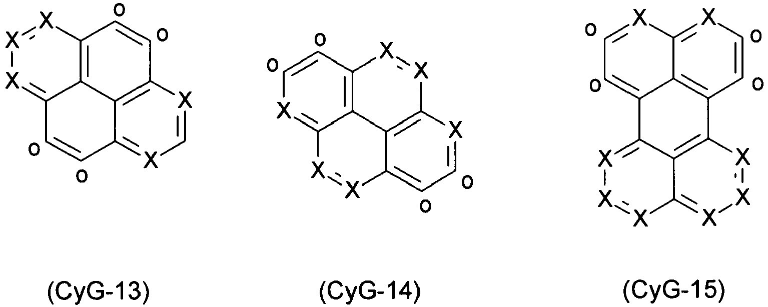 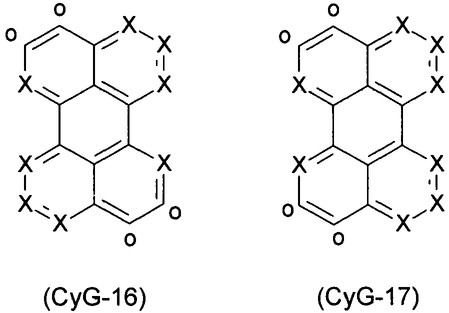 and CyH is at least one structural element selected from the formulae 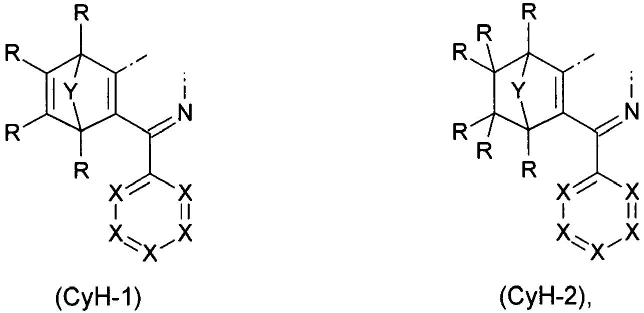
where the symbols R, Y and X used have the meanings given in Claim 1, U is selected from O, S, C(R)₂, N(R), B(R), Si(R)₂, C=O, S=O, SO₂, P(R) and P(=O)R, the dashed lines in the formulae CyH-1 and CyH-2 denote the bond to CyG, and CyH is in each case bonded to CyG at the position denoted by o.

12. Compound according to one or more of Claims 1 to 11, **characterised in that** the symbols X in the structures of the formulae (I), (II), (Ia), (IIa), (Ia1), (IIa1), (Ia2), (IIa2), (Ia3), (Ia4), (Ib), (IIb) and (Ic) and the structures of the formulae CyE-(CyF)ₙ and CyG(CyH)ₙ are selected so that the ratio of CR to N is greater than or equal to 3.

13. Compound according to one or more of Claims 1 to 12, **characterised in that** the compound contains at least one metal atom is selected from iridium and platinum.

14. Oligomer, polymer or dendrimer containing one or more compounds including at least one structure of the formulae (I) and/or (II) 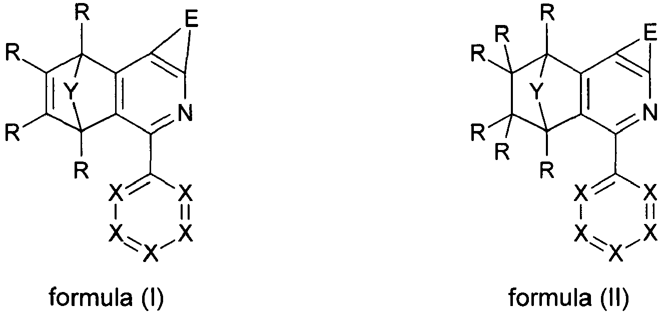 where the following applies to the symbols used:
R is on each occurrence, identically or differently, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C≡C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
and where the definitions of Claim 1 apply to the other symbols used and
where one or more bonds are present from the compound to the polymer, oligomer or dendrimer.

15. Composition comprising at least one compound including at least one structure of the formulae (I) and/or (II) 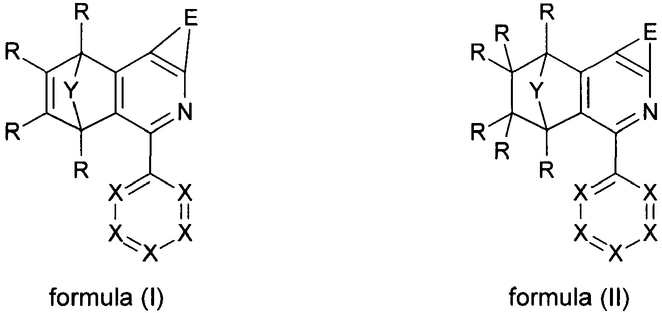 where the following applies to the symbols used:
R is on each occurrence, identically or differently, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C=C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
and where the definitions of Claim 1 apply to the other symbols used, and/or an oligomer, polymer or dendrimer according to Claim 14 and at least one further organofunctional material selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

16. Formulation comprising at least one compound including at least one structure of the formulae (I) and/or (II) 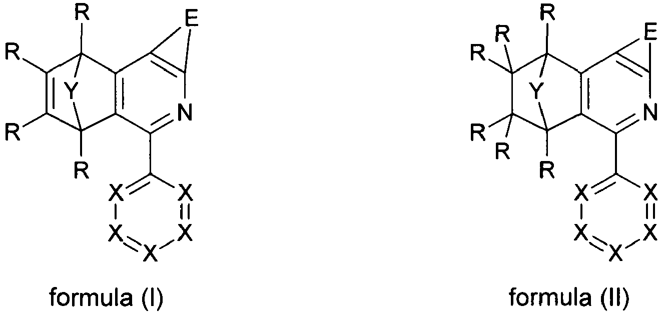 where the following applies to the symbols used:
R is on each occurrence, identically or differently, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C=C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
and where the definitions of Claim 1 apply to the other symbols used, an oligomer, polymer or dendrimer according to Claim 14 and at least one composition according to Claim 15 and at least one solvent.

17. Process for the preparation of a compound according to one or more of Claims 1 to 13 or an oligomer, polymer or dendrimer according to Claim 14 by reaction of at least one aldehyde with at least one aromatic or heteroaromatic amine and at least one bicyclic olefin.

18. Process according to Claim 17, **characterised in that** the intermediate compound obtained after the reaction of at least one aldehyde with at least one aromatic amine and at least one bicyclic olefin is reacted in a coupling reaction.

19. Use of a compound including at least one structure of the formulae (I) and/or (II) 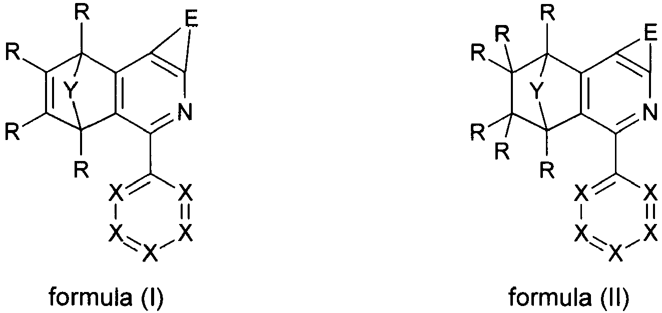 where the following applies to the symbols used:
R is on each occurrence, identically or differently, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C=C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
and where the definitions of Claim 1 apply to the other symbols used, or an oligomer, polymer or dendrimer according to Claim 14 or at least one composition according to Claim 15 in an electronic device as hole-injection material, hole-transport material, hole-blocking material, electron-injection material, electron-blocking material and/or emitter material.

20. Electronic device containing at least one compound including at least one structure of the formulae (I) and/or (II) 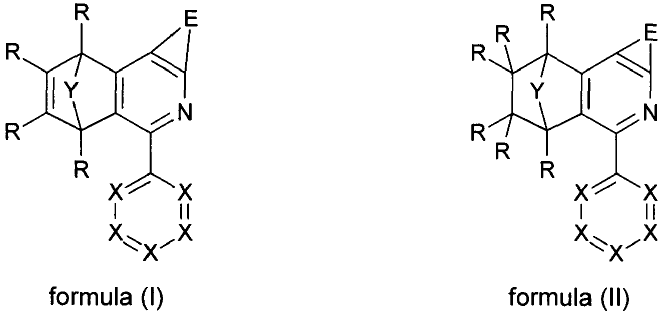 where the following applies to the symbols used:
R is on each occurrence, identically or differently, H, D, F, Br, CI, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, which may in each case be substituted by one or more radicals R¹, where one or more non-adjacent CH₂ groups may be replaced by R¹C=CR¹, C=C, Si(R¹)₂, C=O, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two adjacent radicals R may also form a mono- or polycyclic, aliphatic, aromatic or heteroaromatic ring system with one another here;
and where the definitions of Claim 1 apply to the other symbols used, an oligomer, polymer or dendrimer according to Claim 14 and/or at least one composition according to Claim 15, where the electronic device is preferably selected from the group consisting of organic electroluminescent devices, where organic laser diodes, organic light-emitting transistors, organic light-emitting diodes (OLEDs) and organic light-emitting electrochemical cells (OLECs) are preferred organic electroluminescent devices, OLEDs and OLECs are very preferred organic electroluminescent devices and OLEDs are very particularly preferred organic electroluminescent devices, organic integrated circuits, organic field-effect transistors, organic thin-film transistors, organic solar cells, organic optical detectors, organic photoreceptors and organic field-quench devices.

## Revendications

1. Composé incluant au moins une structure des formules (I) et/ou (II) : 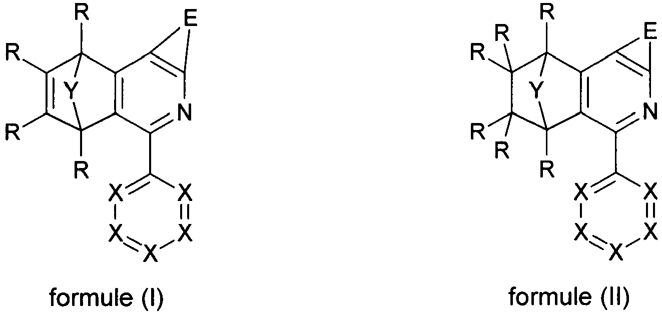 formules dans lesquelles ce qui suit s'applique aux symboles qui sont utilisés :
X est pour chaque occurrence, de manière identique ou différente, CR ou N ;
E est un pont divalent, dans lequel le groupe E forme un cycle à cinq éléments ou à six éléments en association avec les atomes de C qui lui sont connectés ;
Y est un pont divalent qui est sélectionné parmi O, S, C(R)₂, C(R)=C(R), N(R), B(R), Si(R)₂, C=O, C=NR, C=C(R)₂, S=O, SO₂, C(R)₂-C(R)₂, P(R) et P(=O)R ;
R est pour chaque occurrence, de manière identique ou différente, H, D, F, Cl, I, N(R¹)₂, CN, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, dans lequel un ou plusieurs groupe(s) CH₂ non adjacents peut/peuvent être remplacé(s) par R¹C=CR¹, C≡C, Si(R¹)₂, C=O, NR¹, O, S ou CONR¹ et dans lequel un ou plusieurs atome(s) de H peut/peuvent être remplacé(s) par D, F, Cl, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui comporte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
R¹ est pour chaque occurrence, de manière identique ou différente, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R², dans lequel un ou plusieurs groupe(s) CH₂ non adjacents peut/peuvent être remplacé(s) par R²C=CR², C=C, Si(R²)₂, C=O, NR², O, S ou CONR² et dans lequel un ou plusieurs atome(s) de H peut/peuvent être remplacé(s) par D, F, Cl, Br, I, CN ou NO₂, ou un système de cycle aromatique ou hétéroaromatique qui comporte 5 à 60 atomes de cycle aromatique, lequel système de cycle peut dans chaque cas être substitué par un radical ou par plusieurs radicaux R², ou un groupe aryloxy ou hétéroaryloxy qui comporte 5 à 40 atomes de cycle aromatique, lequel groupe peut être substitué par un radical ou par plusieurs radicaux R², ou un groupe aralkyle ou hétéroaralkyle qui comporte 5 à 40 atomes de cycle aromatique, lequel groupe peut être substitué par un radical ou par plusieurs radicaux R², ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R²; deux radicaux R¹ adjacents ou plus peuvent former, l'un avec l'autre ou les uns avec les autres, ou R¹ peut former avec R un système de cycle aliphatique, aromatique ou hétéroaromatique monocyclique ou polycyclique ici ;
R² est pour chaque occurrence, de manière identique ou différente, H, D, F ou un radical hydrocarbone aliphatique, aromatique et/ou hétéroaromatique qui comporte 1 à 20 atome(s) de C, dans lequel, en outre, un ou plusieurs atome(s) de H peut/peuvent être remplacé(s) par F ; deux substituants R² ou plus peuvent également former un système de cycle aliphatique monocyclique ou polycyclique l'un avec l'autre ou les uns avec les autres ici.

2. Composé selon la revendication 1, **caractérisé en ce que** E est sélectionné parmi les groupes des formules X=X-X=X, X-W-X, W-X=X et X=X-W, formules dans lesquelles :
W est sélectionné parmi O, S, C(R)₂, N(R), B(R), Si(R)₂, C=O, S=O, SO₂, P(R) et P(=O)R, dans lequel R est identique ou différent pour chaque occurrence et présente la signification qui a été donnée selon la revendication 1 ; et
X est identique ou différent pour chaque occurrence et présente la signification qui a été donnée selon la revendication 1.

3. Composé selon la revendication 1 ou 2, **caractérisé en ce que** le composé inclut des structures des formules (la) et/ou (IIa) : 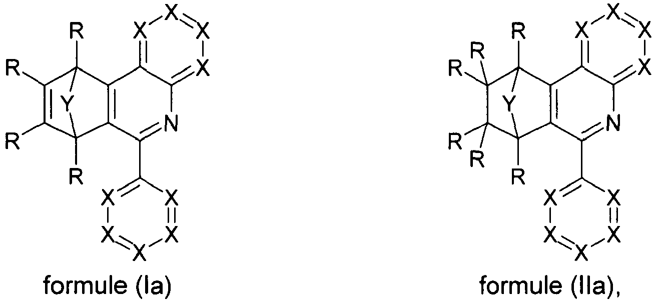 formules dans lesquelles les symboles qui sont utilisés présentent les significations qui ont été données selon la revendication 1.

4. Composé selon une ou plusieurs des revendications 1 à 3, **caractérisé en ce que** le composé inclut des structures des formules (Ia1), (Ia2), (IIa1) et/ou (IIa2) : 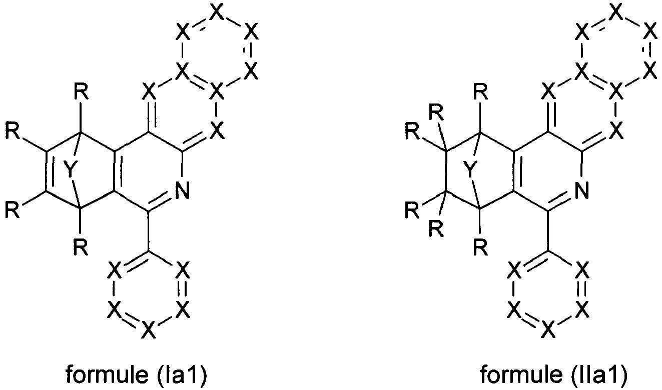 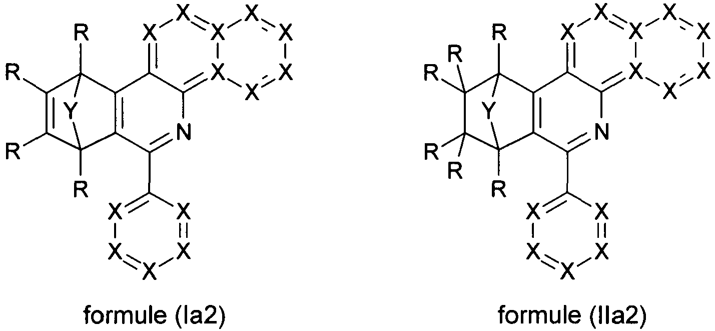 formules dans lesquelles les symboles qui sont utilisés présentent les significations qui ont été données selon la revendication 1.

5. Composé selon une ou plusieurs des revendications 1 à 4, **caractérisé en ce que** le composé inclut des structures des formules (Ia3) et/ou (Ia4) : 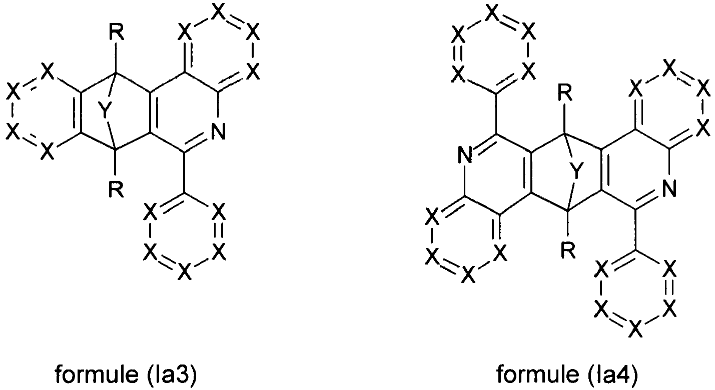 formules dans lesquelles les symboles qui sont utilisés présentent les significations qui ont été données selon la revendication 1.

6. Composé selon la revendication 1 ou 2, **caractérisé en ce que** le composé inclut des structures des formules (Ib) et/ou (IIb) : 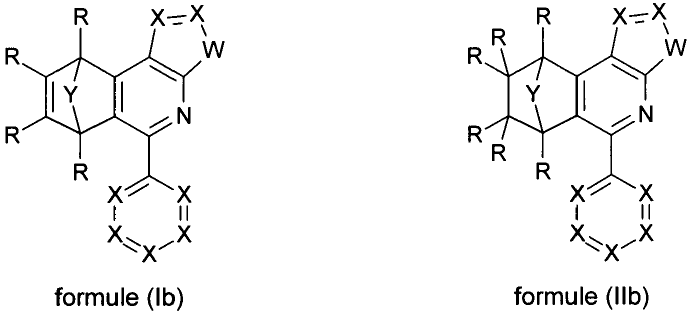 formules dans lesquelles les symboles qui sont utilisés présentent les significations qui ont été données selon les revendications 1 et 2.

7. Composé selon la revendication 1 ou 2, **caractérisé en ce que** le composé inclut des structures de la formule (Ic) : 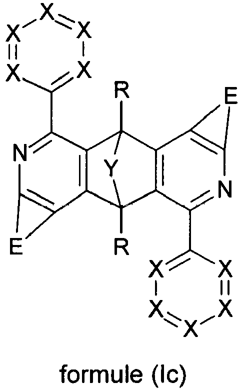 formule dans laquelle les symboles qui sont utilisés présentent les significations qui ont été données selon la revendication 1, et E peut être identique ou différent pour chaque occurrence.

8. Composés selon une ou plusieurs des revendications 1 à 7, **caractérisés en ce que** les composés contiennent au moins deux groupes bicycliques, lesquels groupes sont formés par un élément structurel qui inclut un groupe Y, dans lesquels Y peut être identique ou différent pour chaque occurrence et présente la signification qui a été donnée selon la revendication 1.

9. Composé selon une ou plusieurs des revendications 1 à 8, **caractérisé en ce que** le composé contient des structures de la formule CyE-(CyF)ₙ, formule dans laquelle ce qui suit s'applique aux symboles et indices :
n est 2 ou 3 ;
CyE est un élément structurel qui est sélectionné parmi les formules : 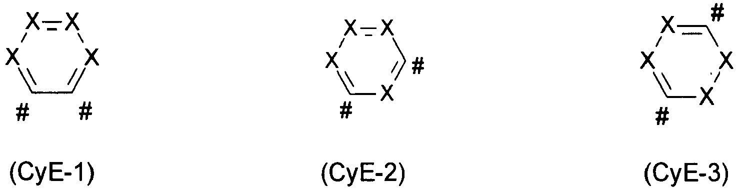 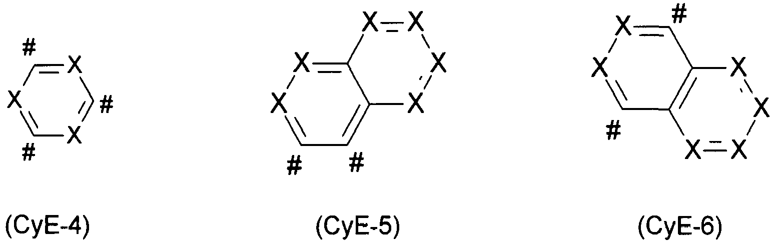 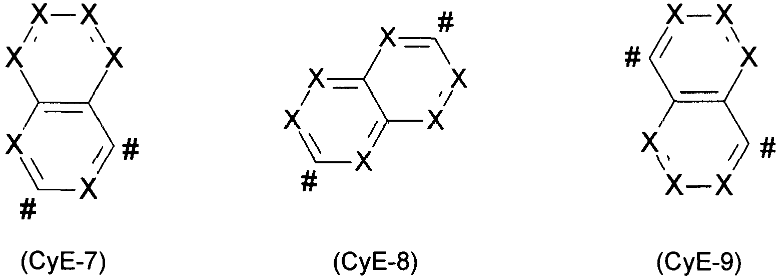 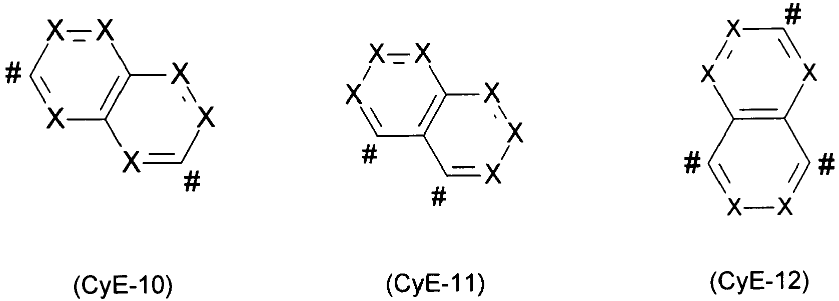 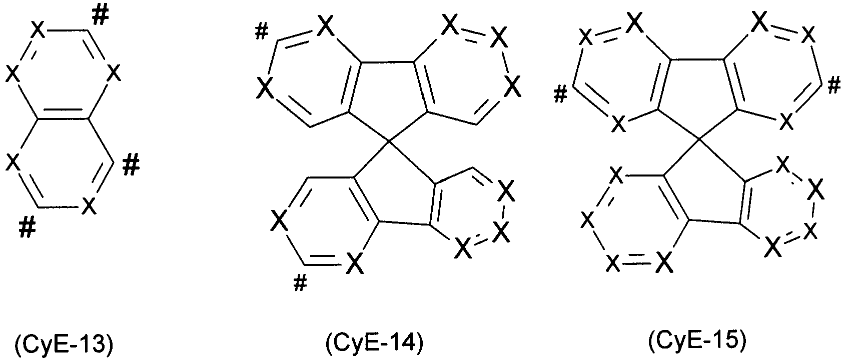 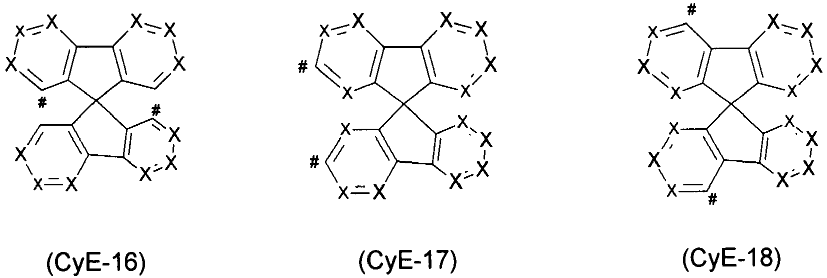 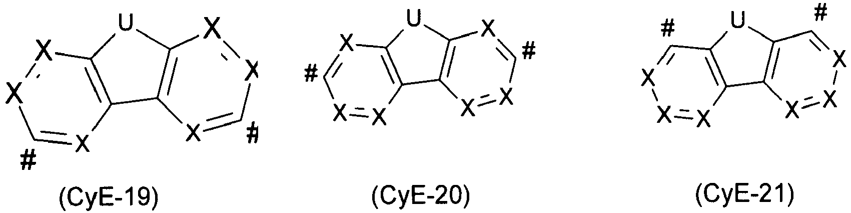 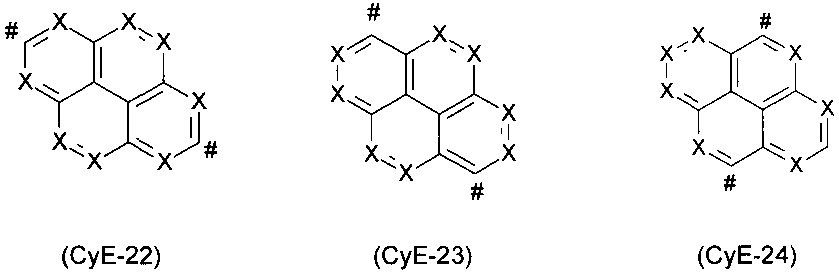 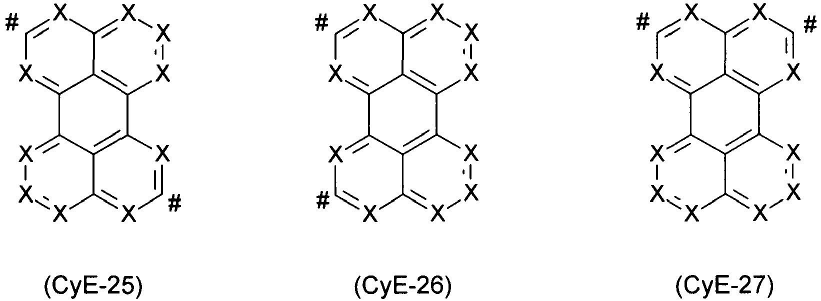 et CyF est au moins un élément structurel qui est sélectionné parmi les formules : 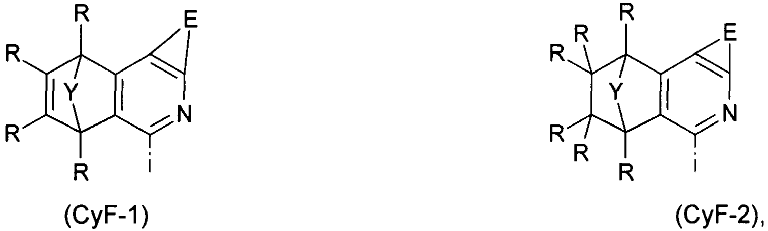 formules dans lesquelles E, R, Y et X qui sont utilisés présentent les significations qui ont été données selon la revendication 1, U est sélectionné parmi O, S, C(R)₂, N(R), B(R), Si(R)₂, C=O, S=O, SO₂, P(R) et P(=O)R, les lignes en pointillés dans les formules CyF-1 et CyF-2 représentent la liaison sur le groupe CyE, et le groupe CyF est dans chaque cas lié au groupe CyE au niveau de la position qui est représentée au moyen de #.

10. Composé selon la revendication 9, **caractérisé en ce que** CyF est au moins un élément structurel qui est sélectionné parmi les formules : 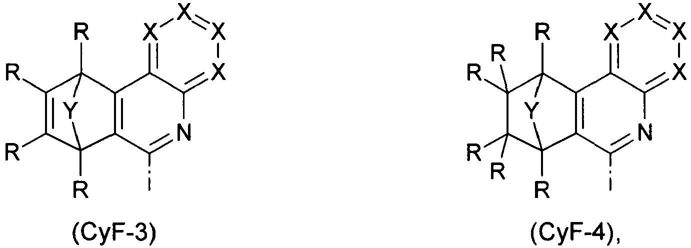 formules dans lesquelles les symboles R, Y et X qui sont utilisés présentent les significations qui ont été données selon la revendication 1, les lignes en pointillés dans les formules CyF-3 et CyF-4 représentent la liaison sur le groupe CyE, et le groupe CyF est dans chaque cas lié au groupe CyE au niveau de la position qui est indiquée au moyen de #.

11. Composé selon une ou plusieurs des revendications 1 à 10, **caractérisé en ce que** le composé contient des structures de la formule CyG(CyH)ₙ, formule dans laquelle CyG et CyH, en association dans chaque cas, forment un cycle, et ce qui suit s'applique aux symboles et indices :
n est 2 ou 3 ;
CyG est un élément structurel qui est sélectionné parmi les formules : 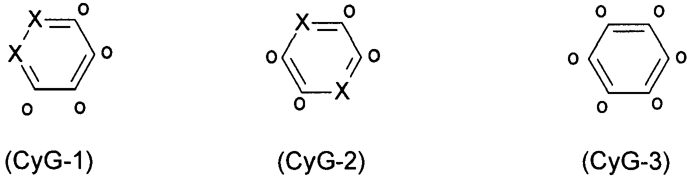 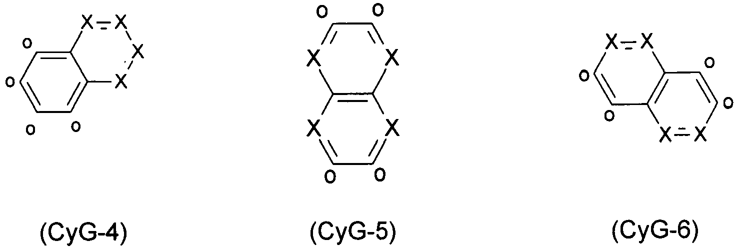 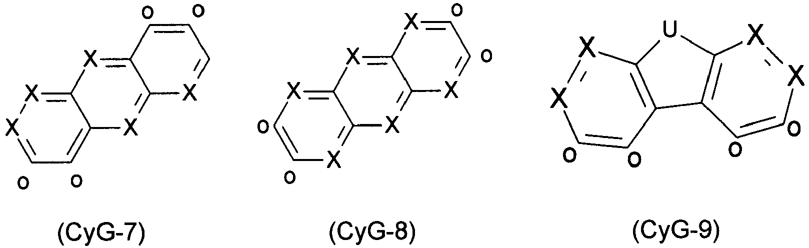 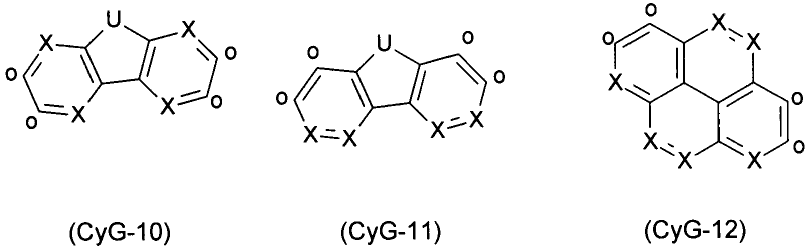 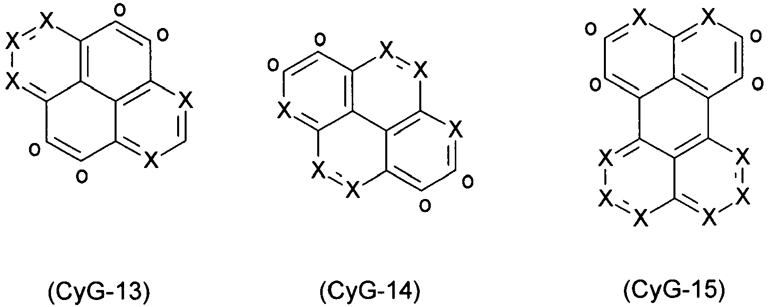 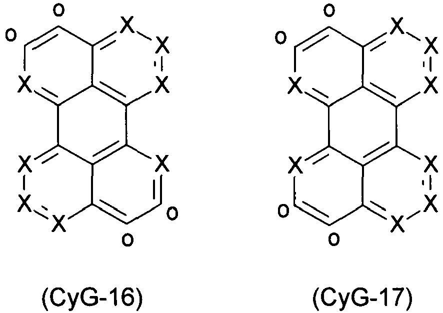 et CyH est au moins un élément structurel qui est sélectionné parmi les formules : 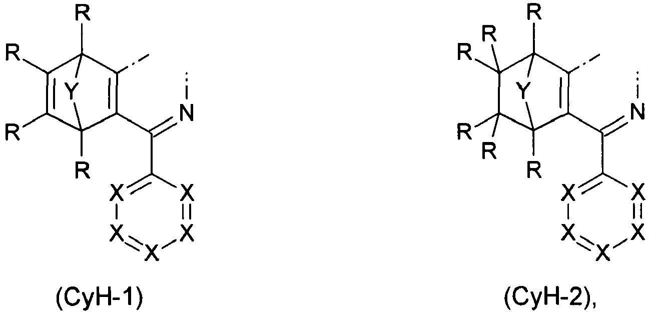 formules dans lesquelles les symboles R, Y et X qui sont utilisés présentent les significations qui ont été données selon la revendication 1, U est sélectionné parmi O, S, C(R)₂, N(R), B(R), Si(R)₂, C=O, S=O, SO₂, P(R) et P(=O)R, les lignes en pointillés dans les formules CyH-1 et CyH-2 représentent la liaison sur le groupe CyG, et le groupe CyH est dans chaque cas lié au groupe CyG au niveau de la position qui est indiquée au moyen de o.

12. Composé selon une ou plusieurs des revendications 1 à 11, **caractérisé en ce que** les symboles X dans les structures des formules (I), (II), (la), (IIa), (Ia1), (IIa1), (Ia2), (IIa2), (Ia3), (Ia4), (Ib), (IIb) et (Ic) et dans les structures des formules CyE-(CyF)ₙ et CyG(CyH)ₙ sont sélectionnés de telle sorte que le rapport de CR sur N soit supérieur ou égal à 3.

13. Composé selon une ou plusieurs des revendications 1 à 12, **caractérisé en ce que** le composé contient au moins un atome de métal qui est sélectionné parmi l'iridium et le platine.

14. Oligomère, polymère ou dendrimère contenant un ou plusieurs composé(s) qui inclue(nt) au moins une structure des formules (I) et/ou (II) : 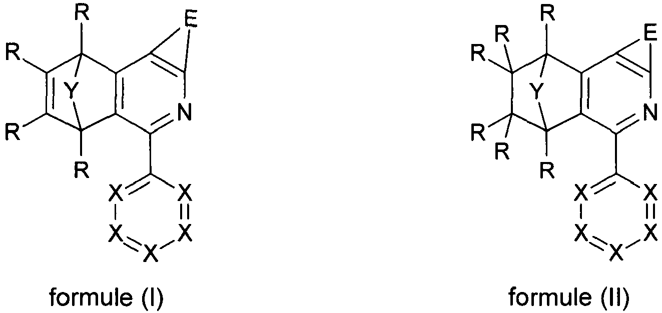 formules dans lesquelles ce qui suit s'applique aux symboles qui sont utilisés :
R est pour chaque occurrence, de manière identique ou différente, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, dans lequel un ou plusieurs groupe(s) CH₂ non adjacents peut/peuvent être remplacé(s) par R¹C=CR¹, C≡C, Si(R¹)₂, C=O, NR¹, O, S ou CONR¹ et dans lequel un ou plusieurs atome(s) de H peut/peuvent être remplacé(s) par D, F, Cl, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui comporte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
et formules dans lesquelles les définitions de la revendication 1 s'appliquent aux autres symboles qui sont utilisés et formules dans lesquelles une ou plusieurs liaison(s) est/sont présente(s) depuis le composé sur le polymère, l'oligomère ou le dendrimère.

15. Composition comprenant au moins un composé incluant au moins une structure des formules (I) et/ou (II) : 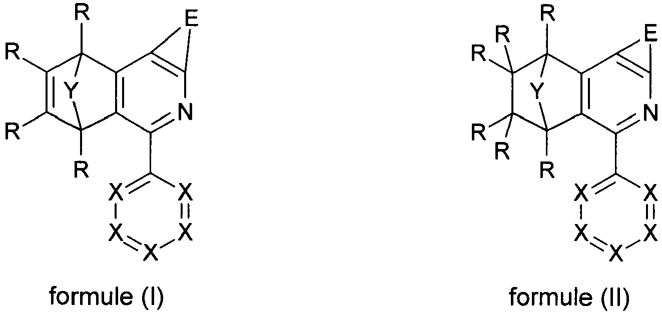 formules dans lesquelles ce qui suit s'applique aux symboles qui sont utilisés :
R est pour chaque occurrence, de manière identique ou différente, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, où un ou plusieurs groupe(s) CH₂ non adjacents peut/peuvent être remplacé(s) par R¹C=CR¹, C=C, Si(R¹)₂, C=O, NR¹, O, S ou CONR¹ et où un ou plusieurs atome(s) de H peut/peuvent être remplacé(s) par D, F, Cl, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui comporte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
et formules dans lesquelles les définitions de la revendication 1 s'appliquent aux autres symboles qui sont utilisés, et/ou un oligomère, un polymère ou un dendrimère selon la revendication 14 et au moins un autre matériau organofonctionnel qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

16. Formulation comprenant au moins un composé qui inclut au moins une structure des formules (I) et/ou (II) : 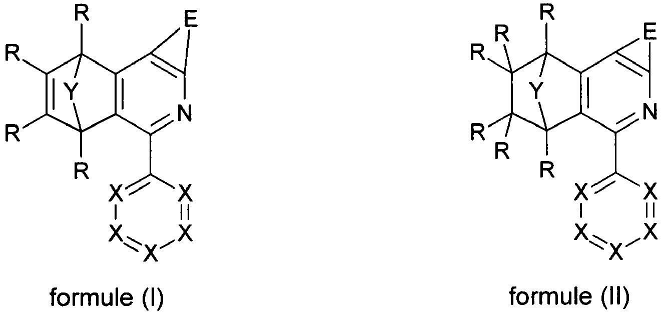 formules dans lesquelles ce qui suit s'applique aux symboles qui sont utilisés :
R est pour chaque occurrence, de manière identique ou différente, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, formulation dans laquelle un ou plusieurs groupe(s) CH₂ non adjacents peut/peuvent être remplacé(s) par R¹C=CR¹, C=C, Si(R¹)₂, C=O, NR¹, O, S ou CONR¹ et formulation dans laquelle un ou plusieurs atome(s) de H peut/peuvent être remplacé(s) par D, F, Cl, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui comporte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
et formulation dans laquelle les définitions de la revendication 1 s'appliquent aux autres symboles qui sont utilisés, un oligomère, un polymère ou un dendrimère selon la revendication 14 et au moins une composition selon la revendication 15 et au moins un solvant.

17. Procédé pour la préparation d'un composé selon une ou plusieurs des revendications 1 à 13 ou d'un oligomère, d'un polymère ou d'un dendrimère selon la revendication 14 au moyen de la réaction d'au moins un aldéhyde avec au moins un amine aromatique ou hétéroaromatique et au moins une oléfine bicyclique.

18. Procédé selon la revendication 17, **caractérisé en ce que** le composé intermédiaire qui est obtenu après la réaction d'au moins un aldéhyde avec au moins un amine aromatique et au moins une oléfine bicyclique est amené à réagir selon une réaction de couplage.

19. Utilisation d'un composé qui inclut au moins une structure des formules (I) et/ou (II) : 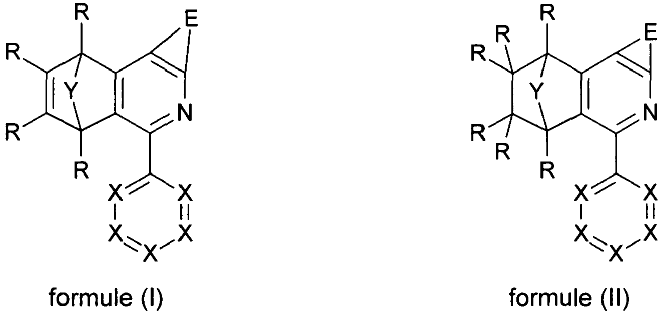 formules dans lesquelles ce qui suit s'applique aux symboles qui sont utilisés :
R est pour chaque occurrence, de manière identique ou différente, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, utilisation dans laquelle un ou plusieurs groupe(s) CH₂ non adjacents peut/ peuvent être remplacé(s) par R¹C=CR¹, C=C, Si(R¹)₂, C=O, NR¹, O, S ou CONR¹ et utilisation dans laquelle un ou plusieurs atome(s) de H peut/peuvent être remplacé(s) par D, F, Cl, Br, I ou CN, ou un système de cycle aromatique ou hétéroaromatique qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui comporte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aromatique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
et utilisation dans laquelle les définitions de la revendication 1 s'appliquent aux autres symboles qui sont utilisés, ou d'un oligomère, d'un polymère ou d'un dendrimère selon la revendication 14 ou d'au moins une composition selon la revendication 15 dans un dispositif électronique en tant que matériau d'injection de trous, matériau de transport de trous, matériau de blocage de trous, matériau d'injection d'électrons, matériau de blocage d'électrons et/ou matériau d'émetteur.

20. Dispositif électronique contenant au moins un composé qui inclut au moins une structure des formules (I) et/ou (II) : 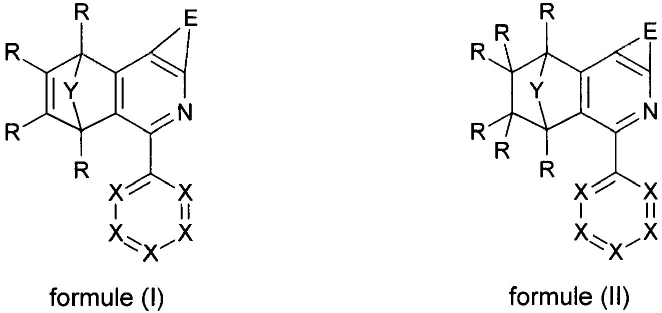 formules dans lesquelles ce qui suit s'applique aux symboles qui sont utilisés :
R est pour chaque occurrence, de manière identique ou différente, H, D, F, Br, Cl, I, N(R¹)₂, CN, NO₂, OH, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte 1 à 20 atome(s) de C ou un groupe alkényle ou alkynyle qui comporte 2 à 20 atomes de C ou un groupe alkyle, alcoxy ou thioalcoxy ramifié ou cyclique qui comporte 3 à 20 atomes de C, lequel groupe peut dans chaque cas être substitué par un radical ou par plusieurs radicaux R¹, groupe dans lequel un ou plusieurs groupe(s) CH₂ non adjacents peut/ peuvent être remplacé(s) par R¹C=CR¹, C=C, Si(R¹)₂, C=O, NR¹, O, S ou CONR¹ et groupe dans lequel un ou plusieurs atome(s) de H peut/peuvent être remplacé(s) par D, F, Cl, Br, I ou CN, ou un système de cycle aromatique ou hétéroaromati que qui comporte 5 à 60 atomes de cycle aromatique, lequel système de cycle peut dans chaque cas être substitué par un radical ou par plusieurs radicaux R¹, ou un groupe aryloxy ou hétéroaryloxy qui comporte 5 à 40 atomes de cycle aromatique, lequel groupe peut être substitué par un radical ou par plusieurs radicaux R¹, ou un groupe aralkyle ou hétéroaralkyle qui com porte 5 à 40 atomes de cycle aromatique, lequel groupe peut être substitué par un radical ou par plusieurs radicaux R¹, ou un groupe diarylamino, un groupe dihétéroarylamino ou un groupe arylhétéroarylamino qui comporte 10 à 40 atomes de cycle aro matique, lequel groupe peut être substitué par un radical ou par plusieurs radicaux R¹ ; deux radicaux R adjacents peuvent également former un système de cycle aliphatique, aromatique ou hétéroaromatique monocyclique ou polycyclique l'un avec l'autre ici ;
et dispositif électronique dans lequel les définitions de la revendication 1 s'appliquent aux autres symboles qui sont utilisés, un oligomère, un polymère ou un dendrimère selon la revendication 14 et/ou au moins une composition selon la revendication 15, où le dispositif électronique est de façon préférable sélectionné parmi le groupe qui est constitué par les dispositifs électroluminescents organiques, groupe au sein duquel les diodes laser organiques, les transistors à émission de lumière organiques, les diodes à émission de lumière organiques (OLED) et les cellules électrochimiques à émission de lumière organiques (OLEC) sont les dispositifs électroluminescents organiques qui ont la préférence, les OLED et les OLEC sont les dispositifs électroluminescents organiques très préférés et les OLED sont les dispositifs électroluminescents organiques qui sont très particulièrement préférés, les circuits intégrés organiques, les transistors à effet de champ organiques, les transistors à film mince organiques, les cellules solaires organiques, les détecteurs optiques organiques, les photorécepteurs organiques et les dispositifs à extinction de champ organiques.
